# EUROPEAN PATENT APPLICATION

(11) **EP 4 276 112 A1**
(43) Date of publication of application: **15.11.2023**
(21) Application number: 22736814.9
(22) Date of filing: 05.01.2022
(51) Int. Cl.: C07K 16/28, A61K 47/68, A61P 35/00, C12Q 1/6886, A61K 39/00

(54) **ANTI-FGFR3 ANTIBODY AND USE THEREOF**

(30) Priority: 05.01.2021 KR 20210000729
(71) Applicant: AIMED BIO INC., Seoul, 05835 (KR)
(72) Inventor: NAM, Do-Hyun, Seoul 06351 (KR); YOON, Yeup, Seoul 06351 (KR); MIN, Byeongkwi, Seoul 05698 (KR); SEO, Yun-Jee, Seoul 06288 (KR); OH, Jeong-Woo, Gwangmyeong-si Gyeonggi-do 14207 (KR)
(74) Representative: Mewburn Ellis LLP
(86) International application number: PCT/KR2022/000119
(87) International publication number: WO 2022/149837

(57) **Abstract**

The present invention relates to: an anti-fibroblast growth factor receptor 3 (FGFR3) antibody or antigen-binding fragment thereof; a nucleic acid encoding same; a recombinant expression vector comprising the nucleic acid; a host cell transfected with the recombinant expression vector; a method for producing the antibody or antigen-binding fragment thereof; a bispecific or multispecific antibody comprising the antibody or antigen-binding fragment thereof; an immune cell-engaging bispecific or multispecific antibody comprising an scFv comprising an scFv of the antibody and a second binding domain comprising at least one scFv of an antibody that binds to an immune cell-activating antigen; an antibody-drug conjugate (ADC) in which the antibody or the antigen-binding fragment thereof is bound to a drug; a chimeric antigen receptor (CAR) comprising the scFv of the anti-FGFR3 antibody as an antigen-binding site of an extracellular domain; an immune cell having the chimeric antigen receptor introduced thereinto; a composition for combination treatment comprising the immune cell; a composition for combination treatment comprising the antibody or the antigen-binding fragment thereof; a composition for treating cancer; a method for treating cancer; and a vesicle comprising the antibody or the antigen-binding fragment thereof.

## Description

### [Technical Field]

The present invention relates to an anti-fibroblast growth factor receptor 3 (FGFR3) antibody or antigen-binding fragment thereof, a nucleic acid encoding the same, a recombinant expression vector including the nucleic acid, a host cell transfected with the recombinant expression vector, a method for producing the antibody or antigen-binding fragment thereof, a bispecific or multispecific antibody including the antibody or antigen-binding fragment thereof, an immune cell-engaging bispecific or multispecific antibody including a scFv comprising a scFv of the antibody and a second binding domain including at least one scFv of an antibody that binds to an immune cell-activating antigen, an antibody-drug conjugate (ADC) in which the antibody or the antigen-binding fragment thereof is bound to a drug, a chimeric antigen receptor (CAR) including the scFv of the anti-FGFR3 antibody as an antigen-binding site of an extracellular domain, an immune cell into which the chimeric antigen receptor is introduced, a composition for combination therapy including the immune cell, a composition for combination therapy including the antibody or the antigen-binding fragment thereof, a composition for treating cancer, or an inflammatory or immune disease, a method of treating cancer or an inflammatory or immune disease, and a vesicle including the antibody or the antigen-binding fragment thereof.

### [Background Art]

Fibroblast growth factor (FGF) and tyrosine kinase receptor (FGFR) thereof play important roles in embryogenesis, maintenance of homeostasis of various tissue, wound healing processes and metabolic functions. In humans, there are 4 highly homologous FGFRs (FGFR1-4) and 22 FGFs (FGF1-14 and FGF16-23; refs. 1-4). The FGFRs include an extracellular region with three immunoglobulin domains (D1, D2, and D3), a single-pass transmembrane region, and a split cytoplasmic kinase moiety.

Dysregulated signaling by FGFR1-4 is associated with pathogenesis in several cancer types. Genomic FGFR alterations, which include gene amplification, chromosomal translocation, and activating mutations, can drive aberrant activation of the FGF pathway and promote neoplastic transformation of normal cells. In particular, FGFR3 amplification is associated with specific induction of bladder cancer. In particular, missense FGFR mutations are also found in multiple types of cancer, and S249C of FGFR3 may enhance FGF driving signals and tumor cell proliferation and cause a hotspot for somatic mutations.

Cancer drug development has targeted the FGF/FGFR pathways. Meanwhile, recently, fusion proteins caused by rearrangement of chromosomes due to cancer-causing mutations have been found in glioblastoma, lung cancer, bladder cancer, oral cancer, head and neck squamous cell cancer, gallbladder cancer, and cervical cancer. This fusion protein is produced by fusion of the FGFR3 gene and the TACC3 (transforming acidic coiled-coil containing protein 3) gene through tandem duplication of chromosome 4. FGFR can be continuously activated by the dimerizing domain of the partner protein that allows the FGFR monomers in the fusion protein to be sufficiently close to be activated. In FGFR3-TACC3, FGFR3 is autophosphorylated and activated by the coiled-coil domain of TACC3 without ligand binding.

TACC3 belongs to the TACC family and rearrangements on chromosome 4p16 corresponding to the TACC3- and FGFR3-including regions are often found in multiple myelopathy, and these rearrangements lead to increased expression of FGFR3 or TACC3 (KN Nelson et al., Oncotarget. 2018 Sep 28; 9(76): 34306-34319).

Based on the information identified as above, clinical trials for antibodies targeting FGFR3 or genes fused with the FGFR3 gene are currently underway.

B701 (Vofatamab) is a human immunoglobulin G1 monoclonal antibody against FGFR3, and clinical trials have been conducted to determine whether or not it exhibits antitumor activity and whether or not it can be used in combination with docetaxel. When the anti-FGFR3 monoclonal antibody B-701 is administered, it specifically binds to wild-type and mutant FGFR3, and inhibits FGFR3 activation and FGFR3-mediated signaling pathway. This inhibits cell proliferation and induces apoptosis in FGFR3-expressing tumors.

LY3076226 is an antibody-drug conjugate (ADC) in which an anti-FGFR3 antibody is conjugated with a microtubule inhibitor (DM4), and clinical trials have been conducted to determine whether or not it can be used for advanced or metastatic cancer. Phase 1 clinical trials are underway to determine whether or not LY3076226 is effective in advanced or metastatic cancer, including multiple myeloma and lymphoma with FGFR3 overexpression or transformation, locally advanced, unresectable or metastatic urinary tract carcinoma (T Ibrahim et al., Bladder Cancer, vol. 5, no. 2, pp. 87-102, 2019) .

Since these conventional anti-FGFR3 antibodies exhibit low affinity, target resolution, and efficacy, the present inventors made efforts to develop a novel anti-FGFR3 antibody with improved affinity, target resolution, and efficacy compared to conventional anti-FGFR3 antibodies. As a result, the present inventors found the characteristics such as affinity of the antibody, FGFR3-binding pattern analysis, FGFR3 resolution, cancer cell growth inhibition and efficacy test results, and in particular, selected antibodies with differentiated characteristics based on the strategy for selecting antibodies having more sensitive reactivity to FGFR3-TACC3 overexpressing brain tumor patient-derived cells than well-known anti-FGFR3 antibodies, and found that the selected antibodies can be used for cancer treatment. Based on this finding, the present invention has been completed.

### [Disclosure]

Therefore, it is one object of the present invention to provide a novel antibody FGFR3 (fibroblast growth factor receptor 3) or antigen-binding fragment thereof.

It is another object of the present invention to provide an antibody having higher sensitivity to FGFR3-TACC3 overexpressing brain tumor patient-derived cells than well-known anti-FGFR3 antibodies.

It is another object of the present invention to provide a nucleic acid encoding the antibody or antigen-binding fragment thereof.

It is another object of the present invention to provide a recombinant expression vector including the nucleic acid or a host cell transfected with the recombinant expression vector.

It is another object of the present invention to provide a method of producing an antibody or antigen-binding fragment thereof specifically binding to FGFR3.

It is another object of the present invention to provide a bispecific or multispecific antibody including the antibody or antigen-binding fragment thereof.

It is another object of the present invention to provide an immune cell-engaging bispecific or multispecific antibody including the antibody or antigen-binding fragment thereof.

It is another object of the present invention to provide an antibody-drug conjugate (ADC) in which the antibody or antigen-binding fragment thereof is conjugated with a drug.

It is another object of the present invention to provide a chimeric antigen receptor (CAR) including a scFv of the anti-FGFR3 antibody as an antigen-binding site of an extracellular domain, immune cells into which the chimeric antigen receptor is introduced, and a composition for combination therapy including the immune cells.

It is another object of the present invention to provide a composition for combination therapy containing the antibody or antigen-binding fragment thereof or the immune cell-engaging bispecific or multispecific antibody.

It is another object of the present invention to provide a composition or method for treating cancer containing the antibody or antigen-binding fragment thereof, the bispecific or multispecific antibody including the antibody or antigen-binding fragment thereof, the antibody-drug conjugate including the antibody or antigen-binding fragment thereof, the chimeric antigen receptor including the antibody or antigen-binding fragment thereof, the chimeric antigen receptor.

It is another object of the present invention to provide a vesicle including the antibody or antigen-binding fragment thereof.

In accordance with one aspect of the present invention, the above and other objects can be accomplished by the provision of an antibody or antigen-binding fragment thereof specifically binding to FGFR3 (fibroblast growth factor receptor 3), including:
a heavy chain CDR1 including at least one amino acid sequence selected from the group consisting of SEQ ID NOS: 1 to 18;
a heavy chain CDR2 including at least one amino acid sequence selected from the group consisting of SEQ ID NOS: 19 to 41;
a heavy chain CDR3 including at least one amino acid sequence selected from the group consisting of SEQ ID NOS: 42 to 66 and SEQ ID NOS: 186 to 198;
a light chain CDR1 including at least one amino acid sequence selected from the group consisting of SEQ ID NOS: 67 to 88;
a light chain CDR2 including at least one amino acid sequence selected from the group consisting of SEQ ID NOS: 89 to 109; and
a light chain CDR3 including at least one amino acid sequence selected from the group consisting of SEQ ID NOS: 110 to 132 and SEQ ID NOS: 199 to 202.

In accordance with another aspect of the present invention, provided is a nucleic acid encoding the antibody or antigen-binding fragment thereof.

In accordance with another aspect of the present invention, provided is a recombinant expression vector containing the nucleic acid.

In accordance with another aspect of the present invention, provided is a host cell transfected with the recombinant expression vector.

In accordance with another aspect of the present invention, provided is a method of producing an antibody or antigen-binding fragment thereof specifically binding to FGFR3 including culturing the host cell to produce an antibody, and isolating and purifying the antibody.

In accordance with another aspect of the present invention, provided is a bispecific or multispecific antibody including the antibody or antigen-binding fragment thereof.

In accordance with another aspect of the present invention, provided is an immune cell-engaging bispecific or multispecific antibody including a scFv comprising a scFv of the antibody and a second binding domain including at least one scFv of an antibody that binds to an immune cell-activating antigen.

In accordance with another aspect of the present invention, provided is an antibody-drug conjugate (ADC) in which the antibody or antigen-binding fragment thereof is conjugated with a drug.

In accordance with another aspect of the present invention, provided is a chimeric antigen receptor (CAR) including an extracellular domain including an antigen-binding site, a transmembrane domain, and an intracellular signaling domain, wherein the antigen-binding site of the extracellular domain is a scFv of the antibody.

In accordance with another aspect of the present invention, provided is an immune cell including the chimeric antigen receptor.

In accordance with another aspect of the present invention, provided is a composition for combination therapy containing the immune cell and a drug other than the anti-FGFR3 antibody.

In accordance with another aspect of the present invention, provided is a composition for combination therapy containing the antibody or antigen-binding fragment thereof and at least one selected from the group consisting of:
(i) an immune cell;
(ii) an immune cell containing a chimeric antigen receptor (CAR) including a scFv fragment of an antibody other than the anti-FGFR3 antibody as an extracellular domain; and
(iii) an immune checkpoint inhibitor.

In accordance with another aspect of the present invention, provided is a composition for combination therapy containing the bispecific or multispecific antibody and at least one selected from the group consisting of:
(i) an immune cell;
(ii) an immune cell containing a chimeric antigen receptor (CAR) including a scFv fragment of an antibody other than the anti-FGFR3 antibody as an extracellular domain; and
(iii) an immune checkpoint inhibitor.

In accordance with another aspect of the present invention, provided is a composition for treating cancer containing the antibody or antigen-binding fragment thereof, the bispecific or multispecific antibody including the antibody or antigen-binding fragment thereof, the antibody-drug conjugate including the antibody or antigen-binding fragment thereof, the chimeric antigen receptor including the antibody or antigen-binding fragment thereof, or immune cells into which the chimeric antigen receptor is introduced.

In accordance with another aspect of the present invention, provided is a composition for treating cancer in which a fusion gene of FGFR3 and TACC3 is amplified, the fusion gene has mutation, the fusion gene is overexpressed, or the fusion gene is constitutively activated, containing the antibody or antigen-binding fragment thereof, the bispecific or multispecific antibody including the antibody or antigen-binding fragment thereof, the antibody-drug conjugate including the antibody or antigen-binding fragment thereof, the chimeric antigen receptor including the antibody or antigen-binding fragment thereof, or immune cellsinto which the chimeric antigen receptor is introduced.

In accordance with another aspect of the present invention, provided is a method of treating cancer including determining, in a patient-derived sample, whether or not a fusion gene of FGFR3 and TACC3 is amplified, the fusion gene has mutation, the fusion gene is overexpressed, or the fusion gene is constitutively activated, and administering, to the patient, the antibody or antigen-binding fragment thereof, the bispecific or multispecific antibody including the antibody or antigen-binding fragment thereof, the antibody-drug conjugate including the antibody or antigen-binding fragment thereof, the chimeric antigen receptor including the antibody or antigen-binding fragment thereof, or immune cells into which the chimeric antigen receptor is introduced, when it is determined that the fusion gene of FGFR3 and TACC3 is amplified, the fusion gene has mutation, the fusion gene is overexpressed, or the fusion gene is constitutively activated.

In accordance with another aspect of the present invention, provided is a vesicle including the antibody or antigen-binding fragment thereof exposed to the outside or present inside.

### [Description of Drawings]

FIG. 1 shows a schematic diagram illustrating phage display-based bio-panning used in the present invention.
FIG. 1A is a schematic diagram illustrating cell panning using FGFR3 overexpressing patient-derived cells.
FIG. 1B is a schematic diagram illustrating stationary phase biopanning using FGFR3 recombinant protein.
FIG. 1C is a schematic diagram illustrating solution-phase bio-panning using FGFR3 recombinant protein.
FIG. 1D is a schematic diagram illustrating magnetism-based semi-automated panning using FGFR3 recombinant protein.
FIG. 2A shows the result of biopanning for antibody screening according to the present invention.
FIG. 2B shows the result of ELISA screening for antibody screening according to the present invention.
FIG. 2C shows the results of magnetic bead-based panning for antibody screening according to the present invention.
FIG. 3A is a schematic diagram illustrating conversion from scFv to IgG format.
FIG. 3B shows the results of Expi293-based antibody production and purity/physical property analysis.

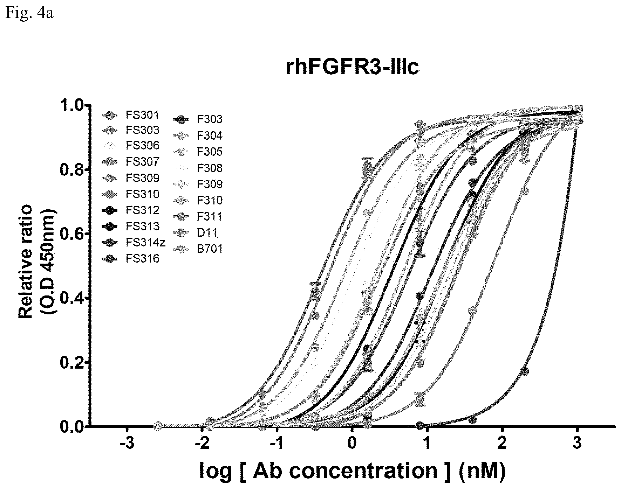

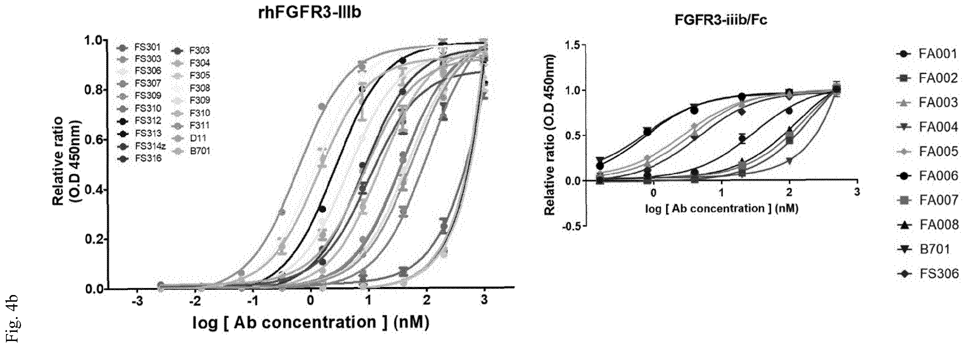

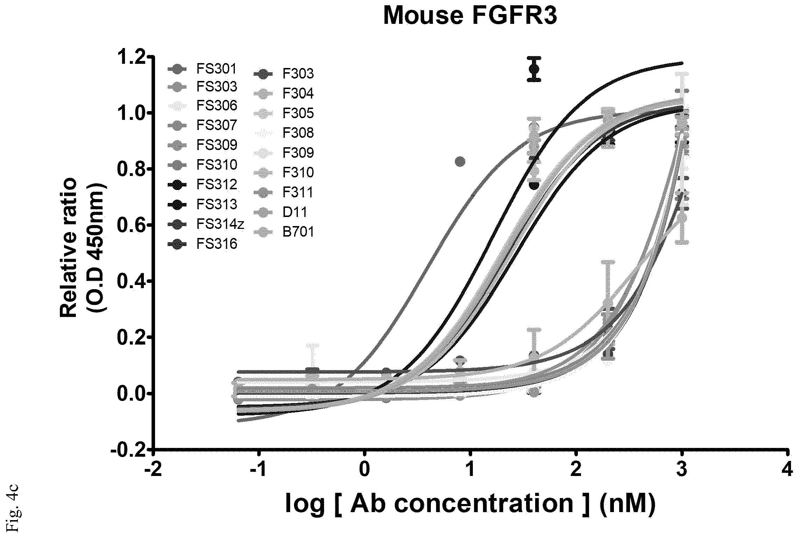

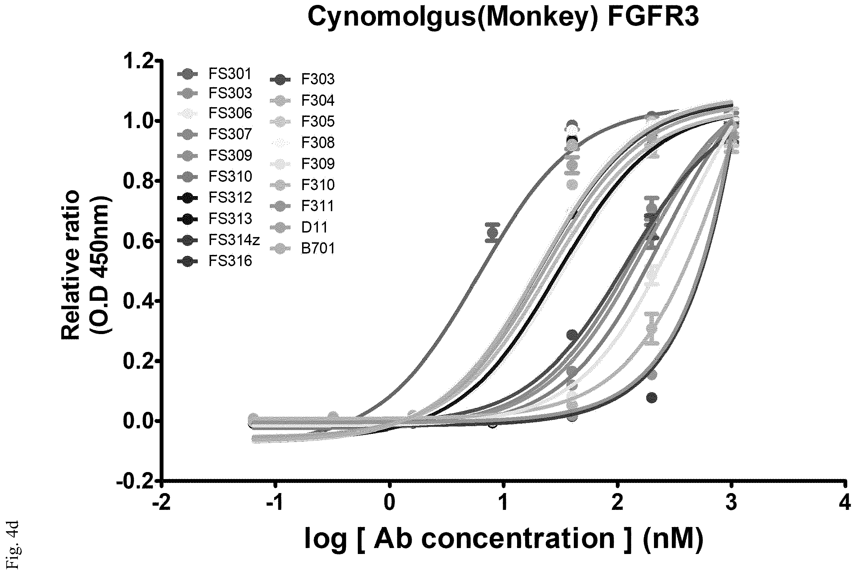

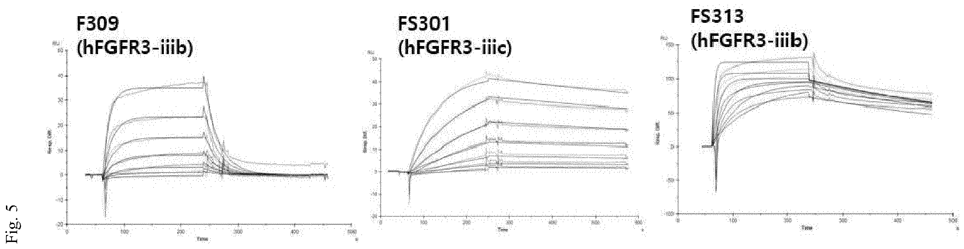

FIGS. 4A to 4D show the results of affinity ELISA of the FGFR3 antibody (IgG). FIG. 4A shows the specific-binding ability of the antibody to the recombinant protein human FGFR3-IIIc, FIG. 4B shows the specific-binding ability of the antibody to the recombinant protein human FGFR3-IIIb, FIG. 4C shows the specific-binding ability of the antibody to the recombinant protein mouse FGFR3, FIG. 4D shows the specific-binding ability of the antibody to the recombinant protein cynomolgus monkey FGFR3, and FIG. 5 shows the results of evaluation of KD value of the FGFR3 antibody (IgG) using a Biacore 3000.
FIG. 6 shows the results of FGFR3 expression confirmation and FGFR3-TACC3 fusion analysis of brain tumor patient-derived cells. FIG. 6A shows the results of mRNA expression level (RPKM) analysis, protein expression level analysis (western blotting), and cell surface expression analysis (FACS analysis) of patient-derived cells, and FIG. 6B shows the results of confirmation of FGFR3-TACC3 fusion through RT-PCR: fusion specific primer.
FIG. 7A shows the results of cell surface binding analysis of FGFR3 antibody clones to FGFR3(-TACC3) overexpressing brain tumor patient-derived cells. FIG. 7B shows the results of concentration-specific binding pattern analysis of FGFR3(-TACC3) overexpressing brain tumor patient-derived cells of the F309 antibody. FIG. 7C shows the results of the analysis of binding force of F309 and FS306 antibodies to the cell line not expression FGFR3.
FIG. 8 shows the results of analysis of the patient-derived cell surface FGFR3 (-TACC3) resolution of the FACS-based FGFR3 antibody and screening thereof.
FIG. 9A shows the results of FGFR3-specific binding ability analysis of F309 and FS306 antibodies, and confirmation of non-competitive ligand characteristics. FIG. 9B shows the results of identification of the unique epitope domains (Domain I) of F309 and FS306 antibodies. FIG. 9C shows the results of recombinant domain design for epitope domain mapping.

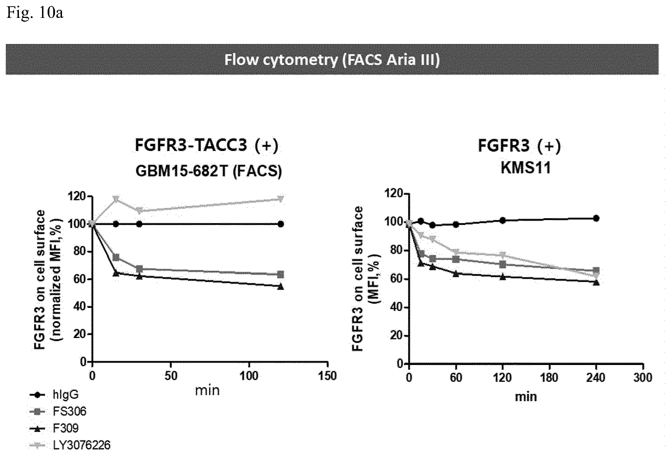

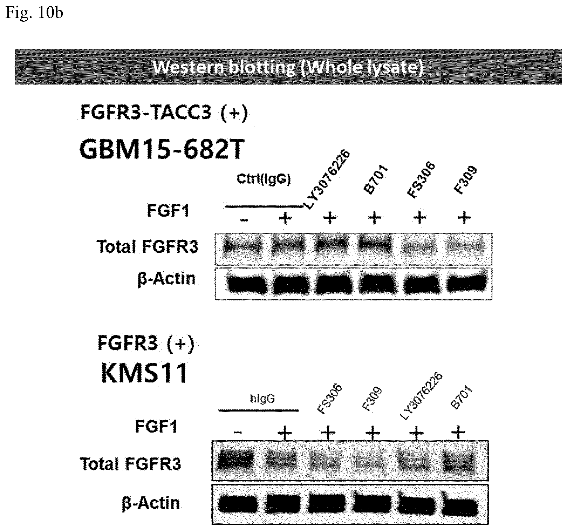

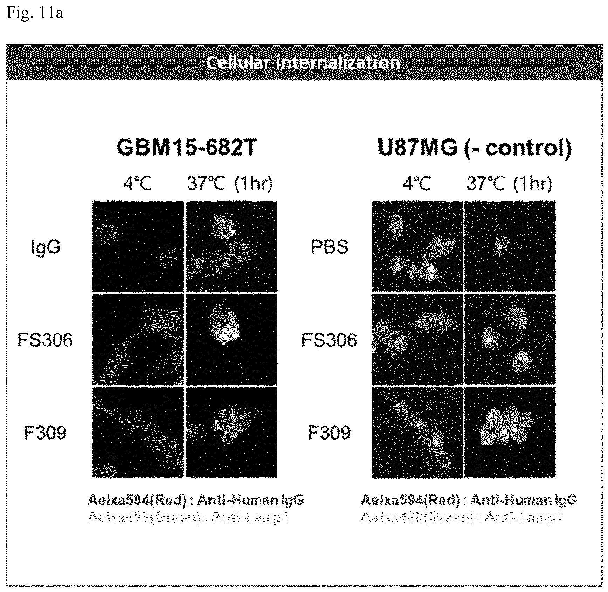

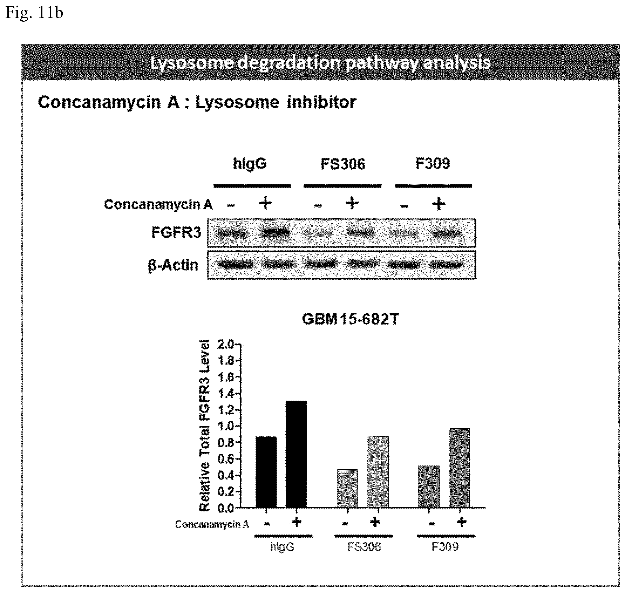

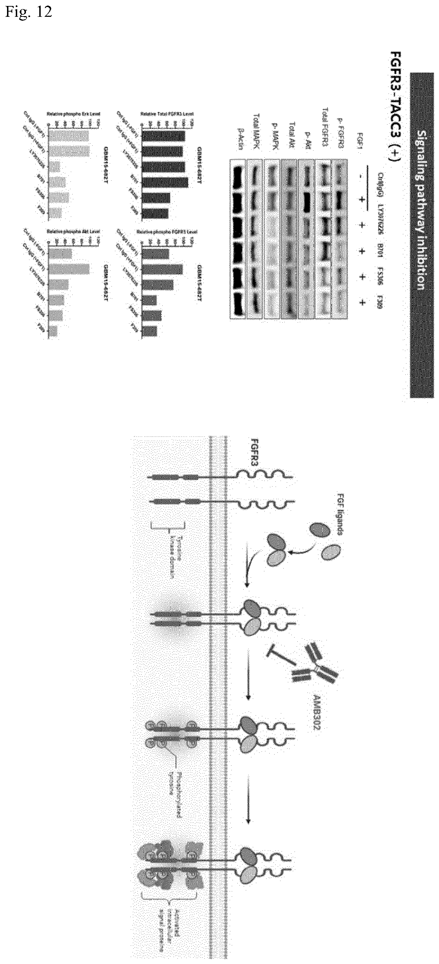

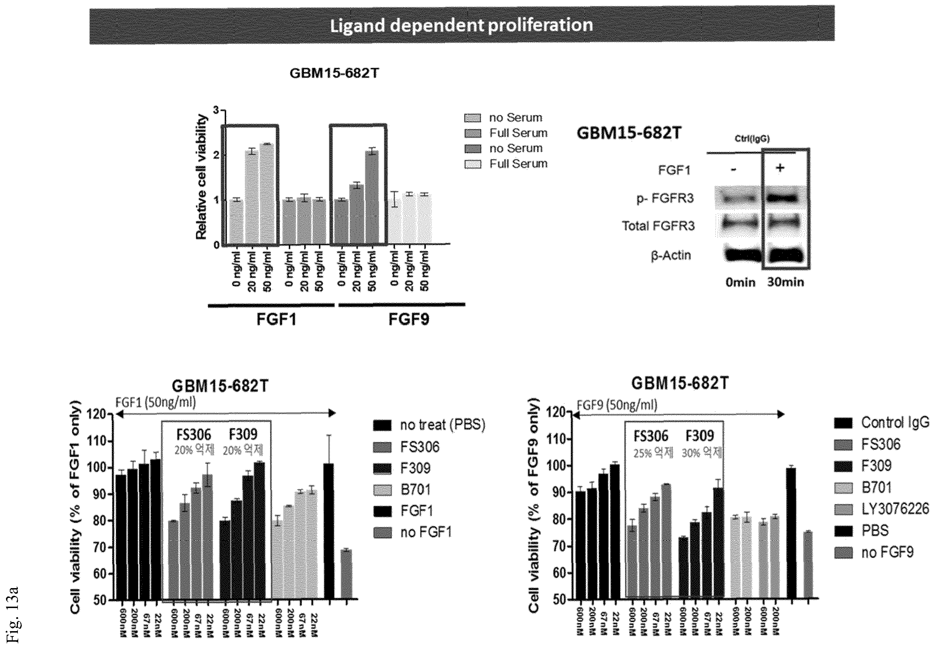

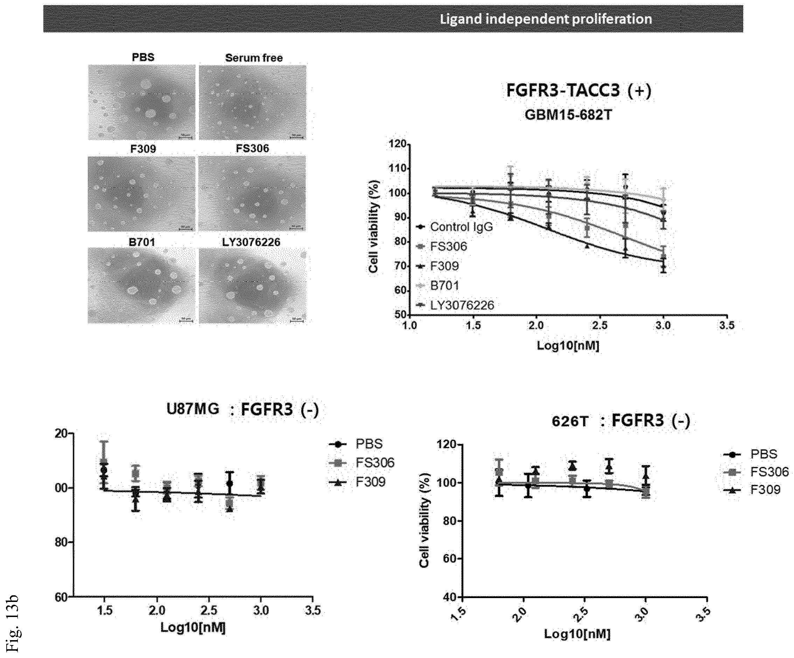

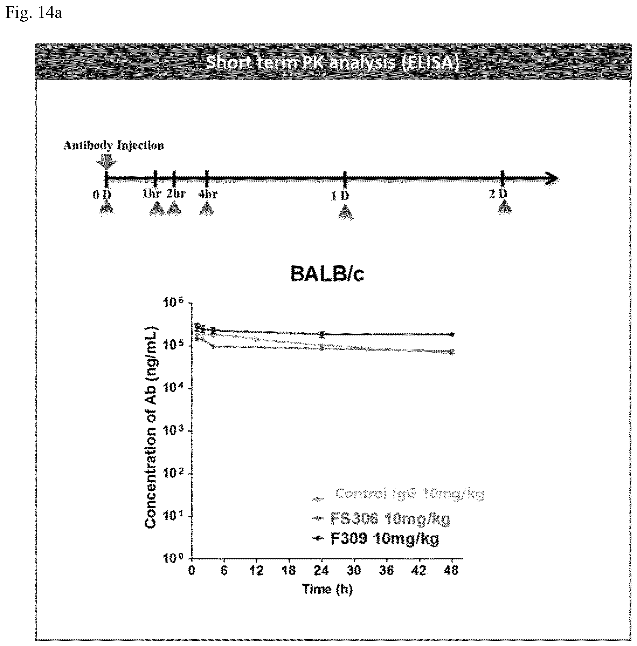

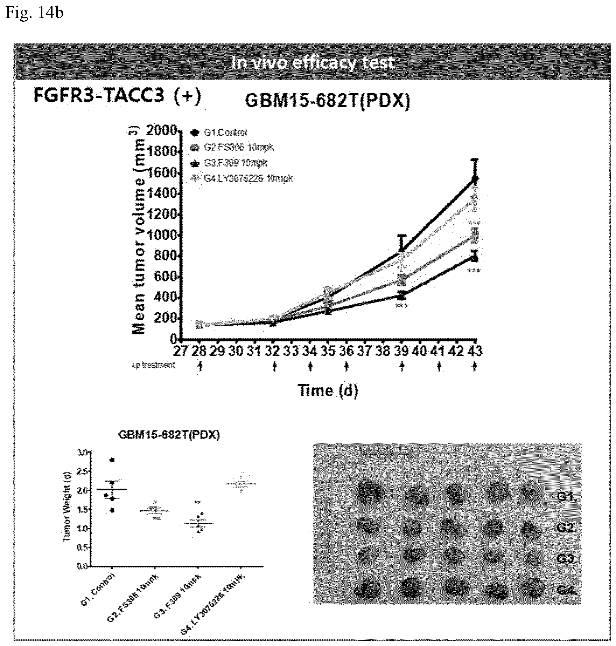

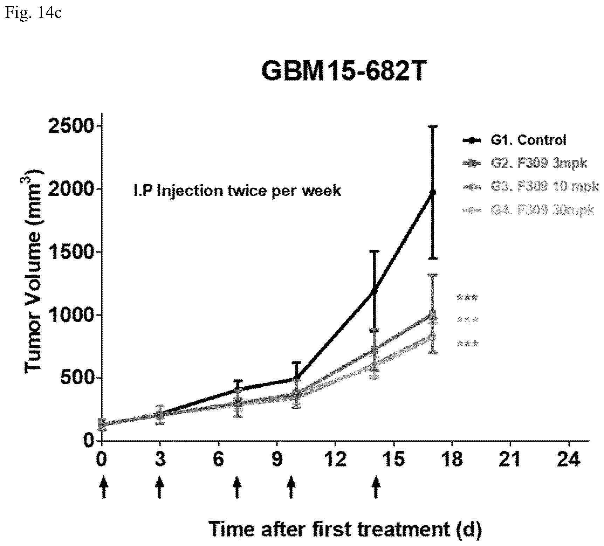

FIG. 10 shows the results of analysis of target resolution for FGFR3-TACC3 overexpressing brain tumor patient-derived cells and FGFR3 overexpressing cell lines of F309 and FS306 antibodies. FIG. 10A shows the results of analysis (FACS analysis) of cell surface FGFR3-TACC3 and FGFR3 resolution of F309 and FS306 antibodies. FIG. 10B shows results of analysis (Western blotting) of the total FGFR3-TACC3 and FGFR3 resolution of F309 and FS306 antibodies. FIG. 11 shows the results of analysis of cell internalization of antibodies F309 and FS306. FIG. 11A shows the results of analysis of cell internalization (analysis of ICC) in FGFR3-TACC3 overexpressing brain tumor patient-derived cells. FIG. 11B shows the result of identification of lysosomal degradation mechanism of F309 and FS306 antibodies through lysosome inhibitor treatment. FIG. 12 shows the results of analysis of FGFR3 sub-signaling inhibition of F309 and FS306 antibodies. FIG. 13A shows the results of ligand-dependent cell viability inhibition assay (FGF1, FGF9). FIG. 13B shows the results of ligand-independent cell growth inhibition assay (FGFR3-TACC3 overexpressing brain tumor patient-derived cells, FGFR3 low-expression cell lines or brain tumor patient-derived cells). FIG. 14A shows the results of short-term PK analysis of F309 and FS306. FIG. 14B shows the results of antibody efficacy evaluation in FGFR3-TACC3 overexpressing brain tumor patient-derived cell transplant mice (PDX). FIG. 14C shows the results of F309 antibody efficacy evaluation (FGFR3-TACC3 overexpressing brain tumor patient-derived cells) in the PDX model.
FIG. 15A is a schematic diagram illustrating 3D and 2D structures and strategic affinity improvement of the F309 antibody. FIG. 15B shows the results of affinity comparison between F309 affinity-modified antibodies through affinity ELISA. FIG. 15C shows the results of SPR analysis of the antibody with improved F309 affinity. FIG. 15C shows the results of the ligand-independent cell viability inhibition assay (FGFR3-TACC3 overexpressing brain tumor patient-derived cells) of the F309 improved antibody clones.
FIG. 16A is a schematic diagram for confirming the applicability to antibody-drug conjugate (ADC) of F309 antibody. FIG. 16B shows the results of cytotoxicity analysis of FGFR3-TACC3 overexpressing brain tumor patient-derived cells and FGFR3 overexpressing cell lines of F309-vc MMAE. FIG. 16C shows the results of cytotoxicity assay-1 depending on the FGFR3 expression level of F309-vc MMAE, and FIG. 16D shows the results of cytotoxicity assay-2 depending on the FGFR3 expression level of F309-vc MMAE.
FIG. 17 shows the results of Fv structures and epitope domain mapping of affinity matured antibodies and antigen-specific binding analysis.
FIG. 18 shows the results of analysis of the cell surface antigen expression levels of cells and cell lines of FGFR3-TACC3 brain tumor patient-derived cells.
FIG. 19 shows the results of analysis of the efficiency of cell internalization of affinity matured antibodies.
FIG. 20 shows the results of analysis of the FGFR3 (-TACC3) target resolution of affinity matured antibodies.
FIG. 21 shows the results of mouse PK analysis of affinity matured antibodies.
FIG. 22 shows the results of monkey PK analysis of affinity matured antibodies.
FIG. 23 is a schematic diagram illustrating preparation of an ADC including affinity matured antibodies (conjugation with topoisomerase I inhibitor).
FIG. 24 is a schematic diagram illustrating preparation of an ADC including affinity matured antibodies (conjugation with topoisomerase I inhibitor).
FIG. 25 shows results of analysis of the physical properties and binding capacity of ADCs including affinity matured antibodies.
FIG. 26 shows the results of analysis of physical properties of ADCs including affinity matured antibodies.
FIG. 27 shows the results of *in vitro* efficacy evaluation of ADCs including affinity matured antibodies.
FIG. 28 shows the results of *in vitro* efficacy evaluation of ADCs including affinity matured antibodies.
FIG. 29 shows the results of *in vivo* efficacy evaluation of ADCs including affinity matured antibodies.
FIG. 30 shows the results of *in vivo* efficacy evaluation of ADCs including affinity matured antibodies.
FIG. 31 shows the results of *in vivo* efficacy evaluation of ADCs including affinity matured antibodies.
FIG. 32 shows the results of evaluation of ADC efficacy of ADCs including affinity matured antibodies in a stereotactic animal model of FGFR3 (TACC3 fusion) brain tumors.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

### Anti-FGFR3 antibody

In one aspect, the present invention is directed to an antibody or antigen-binding fragment thereof specifically binding to FGFR3 (fibroblast growth factor receptor 3), including: a heavy chain CDR1 including at least one amino acid sequence selected from the group consisting of SEQ ID NOS: 1 to 18; a heavy chain CDR2 including at least one amino acid sequence selected from the group consisting of SEQ ID NOS: 19 to 41; a heavy chain CDR3 including at least one amino acid sequence selected from the group consisting of SEQ ID NOS: 42 to 66 and SEQ ID NOS: 186 to 198; a light chain CDR1 including at least one amino acid sequence selected from the group consisting of SEQ ID NOS: 67 to 88; a light chain CDR2 including at least one amino acid sequence selected from the group consisting of SEQ ID NOS: 89 to 109, and a light chain CDR3 including at least one amino acid sequence selected from the group consisting of SEQ ID NOS: 110 to 132 and SEQ ID NOS: 199 to 202.

As used herein, the term "antibody" refers to an anti-FGFR3 antibody that specifically binds to FGFR3. The scope of the present invention includes not only a complete antibody specifically binding to FGFR3 but also an antigen-binding fragment of the antibody molecule. In some cases, the antibody according to the present invention may target FGFR3-TACC3. In particular, the antibody according to the present invention was selected as an antibody having better efficacy against FGFR3-TACC3 overexpressing cancer cells than well-known anti-FGFR3 antibodies.

The complete antibody has a structure having two full-length light chains and two full-length heavy chains, wherein each light chain is linked to a corresponding heavy chain by a disulfide bond.

As used herein, the term "heavy chain" encompasses both a full-length heavy chain, which includes a variable domain (VH) containing an amino acid sequence having a variable region sequence sufficient for imparting specificity to an antigen and three constant domains (CH1, CH2 and CH3), and a fragment thereof. As used herein, the term "light chain" encompasses both a full-length light chain, which includes a variable domain (VL) containing an amino acid sequence having a variable region sequence sufficient for imparting specificity to an antigen and a constant domain (CL), and a fragment thereof.

The whole antibody includes IgA, IgD, IgE, IgM and IgG subtypes, and IgG includes subtypes of IgG1, IgG2, IgG3 and IgG4. The heavy-chain constant region has gamma (γ), mu (µ), alpha (α), delta (δ) and epsilon (ε) types, and is subclassified into gamma 1 (γ1), gamma 2 (γ2), gamma 3 (γ3), gamma 4 (γ4), alpha 1 (α1), and alpha 2 (α2). The light-chain constant region has kappa (κ) and lambda (λ) types.

The antigen-binding fragment of an antibody or antibody fragment refers to a fragment that has antigen-binding capacity, and includes Fab, F(ab'), F(ab')2, Fv and the like. Among the antibody fragments, Fab refers to a structure including a variable domain of each of the heavy chain and the light chain, the constant domain of the light chain, and the first constant domain (CH1) of the heavy chain, each having one antigen-binding site. Fab' is different from Fab in that it further includes a hinge region including at least one cysteine residue at the C-terminus of the CH1 domain of the heavy chain. F(ab')2 is created by a disulfide bond between cysteine residues in the hinge region of Fab'.

Fv is the minimal antibody fragment having only a heavy-chain variable domain and a light-chain variable domain. A two-chain Fv is a fragment wherein the variable domain of the heavy chain and the variable domain of the light chain are linked by a non-covalent bond, and a single-chain Fv (scFv) is a fragment wherein the variable domain of the heavy chain and the variable domain of the light chain are generally linked by a covalent bond via a peptide linker therebetween, or are directly linked at the C-terminus, forming a dimer-shaped structure, like the two-chain Fv. Such antibody fragments may be obtained using proteases (e.g., Fab can be obtained by restriction-cleaving the whole antibody with papain, and the F(ab')2 fragment can be obtained by restriction-cleaving the whole antibody with pepsin), and may be prepared using genetic recombination techniques.

The "Fv" fragment is an antibody fragment containing complete antibody recognition and binding sites. Such a region includes a dimer that consists of one heavy-chain variable domain and one light-chain variable domain linked to each other.

A "Fab" fragment contains a variable domain and a constant domain of the light-chain and a variable domain and a first constant domain (CH1) of the heavy-chain. A F(ab')2 antibody fragment generally includes a pair of Fab fragments covalently linked near the carboxyl terminal thereof via a hinge cysteine therebetween.

The "single-chain Fv" or "scFv" antibody fragment is a construct including a single polypeptide chain including VH and VL domains of the antibody. The scFv antibody fragment may further include a polypeptide linker between the VH domain and the VL domain in order for the scFv to form a desired structure for antigen binding.

In an embodiment, the antibody according to the present invention includes, but is not limited to, monoclonal antibodies, multispecific antibodies, human antibodies, humanized antibodies, chimeric antibodies, scFVs, Fab fragments, F(ab') fragments, disulfide-binding Fvs (sdFVs), anti-idiotypic (anti-Id) antibodies, epitope-binding fragments of such antibodies, and the like.

The heavy-chain constant region may be selected from gamma (γ), mu (u), alpha (α), delta (δ) and epsilon (c) isotypes. For example, the constant region may be gamma 1 (IgG1), gamma 3 (IgG3), or gamma 4 (IgG4). The light-chain constant region may be kappa- or lambda-type.

The term "monoclonal antibody" refers to an identical antibody, which is obtained from a population of substantially homogeneous antibodies, that is, each antibody constituting the population, excluding possible naturally occurring mutations that may be present in trivial amounts. Monoclonal antibodies are highly specific and are thus induced against a single antigenic site. Unlike conventional (polyclonal) antibody preparations that typically include different antibodies directed against different determinants (epitopes), each monoclonal antibody is directed against a single determinant on the antigen.

The term "epitope" refers to a protein determinant to which an antibody can specifically bind. Epitopes usually consist of a group of chemically active surface molecules, such as amino acid or sugar side chains, and generally have not only specific three-dimensional structural characteristics but also specific charge characteristics. Three-dimensional epitopes are distinguished from non-three-dimensional epitopes in that a bond to the former is broken in the presence of a denatured solvent, while a bond to the latter is not broken.

The antibody or antigen-binding fragment thereof according to the present invention may be, for example, bonded to any one, for example, D1, D2 or D3 domain, selected from the group consisting of three immune domains D1, D2 and D3 present in the extracellular region of FGFR3. In some cases, the antibody or antigen-binding fragment thereof according to the present invention may specifically bind to domain 1 (SEQ ID NO: 184) of FGFR3 of SEQ ID NO: 183 and may not compete for binding with the FGF ligand.

The non-human (*e.g*., murine) antibody of the "humanized" form is a chimeric antibody including minimal sequences derived from non-human immunoglobulins. In most cases, the humanized antibody is a human immunoglobulin (receptor antibody) in which a residue from the hypervariable region of a receptor is replaced with a residue from the hypervariable region of a non-human species (donor antibody) such as a mouse, rat, rabbit or non-human primate having the desired specificity, affinity and ability.

The term "human antibody" means a molecule derived from human immunoglobulin, wherein all of the amino acid sequences constituting the antibody including a complementarity-determining region and a structural region are composed of human immunoglobulin.

A part of the heavy chain and/or light chain is identical to or homologous with the corresponding sequence in an antibody derived from a particular species or belonging to a particular antibody class or subclass, while the other chain(s) include "chimeric" antibodies (immunoglobulins) which are identical to or homologous with corresponding sequences in an antibody derived from another species or belonging to another antibody class or subclass, as well as fragments of such antibody exhibiting the desired biological activity.

As used herein, the term "antibody variable domain" refers to the light- and heavy-chain regions of an antibody molecule including the amino acid sequences of a complementarity-determining region (CDR; i.e., CDR1, CDR2, and CDR3) and a framework region (FR). VH refers to a variable domain of the heavy chain. VL refers to a variable domain of the light chain.

The term "complementarity-determining region" (CDR) refers to an amino acid residue of the antibody variable domain, which is necessary for antigen binding. Each variable domain typically has three CDR regions, identified as CDR1, CDR2, and CDR3.

The anti-FGFR3 antibody or antigen-binding fragment thereof according to the present invention is selected as an antibody having better efficacy against FGFR3-TACC3 overexpressing cancer cells than well-known anti-FGFR3 antibodies and examples thereof include the following:
a heavy-chain variable region including a heavy chain CDR1 of SEQ ID NO: 1, a heavy chain CDR2 of SEQ ID NO: 19, and a heavy chain CDR3 of SEQ ID NO: 42, and a light-chain variable region including a light chain CDR1 of SEQ ID NO: 67, a light chain CDR2 of SEQ ID NO: 89, and a light chain CDR3 of SEQ ID NO: 110,
a heavy-chain variable region including a heavy chain CDR1 of SEQ ID NO: 2, a heavy chain CDR2 of SEQ ID NO: 20, and a heavy chain CDR3 of SEQ ID NO: 43, and a light-chain variable region including a light chain CDR1 of SEQ ID NO: 68, a light chain CDR2 of SEQ ID NO: 90, and a light chain CDR3 of SEQ ID NO: 111,
a heavy-chain variable region including a heavy chain CDR1 of SEQ ID NO: 3, a heavy chain CDR2 of SEQ ID NO: 21, and a heavy chain CDR3 of SEQ ID NO: 44, and a light-chain variable region including a light chain CDR1 of SEQ ID NO: 69, a light chain CDR2 of SEQ ID NO: 91, and a light chain CDR3 of SEQ ID NO: 112,
a heavy-chain variable region including a heavy chain CDR1 of SEQ ID NO: 4, a heavy chain CDR2 of SEQ ID NO: 22, and a heavy chain CDR3 of SEQ ID NO: 45, and a light-chain variable region including a light chain CDR1 of SEQ ID NO: 70, a light chain CDR2 of SEQ ID NO: 92, and a light chain CDR3 of SEQ ID NO: 113,
a heavy-chain variable region including a heavy chain CDR1 of SEQ ID NO: 5, a heavy chain CDR2 of SEQ ID NO: 23, and a heavy chain CDR3 of SEQ ID NO: 46, and a light-chain variable region including a light chain CDR1 of SEQ ID NO: 71, a light chain CDR2 of SEQ ID NO: 93, and a light chain CDR3 of SEQ ID NO: 114,
a heavy-chain variable region including a heavy chain CDR1 of SEQ ID NO: 5, a heavy chain CDR2 of SEQ ID NO: 23, and a heavy chain CDR3 of SEQ ID NO: 186, and a light-chain variable region including a light chain CDR1 of SEQ ID NO: 71, a light chain CDR2 of SEQ ID NO: 93, and a light chain CDR3 of SEQ ID NO: 114,
a heavy-chain variable region including a heavy chain CDR1 of SEQ ID NO: 5, a heavy chain CDR2 of SEQ ID NO: 23, and a heavy chain CDR3 of SEQ ID NO: 187, and a light-chain variable region including a light chain CDR1 of SEQ ID NO: 71, a light chain CDR2 of SEQ ID NO: 93, and a light chain CDR3 of SEQ ID NO: 114,
a heavy-chain variable region including a heavy chain CDR1 of SEQ ID NO: 5, a heavy chain CDR2 of SEQ ID NO: 23, and a heavy chain CDR3 of SEQ ID NO: 188, and a light-chain variable region including a light chain CDR1 of SEQ ID NO: 71, a light chain CDR2 of SEQ ID NO: 93, and a light chain CDR3 of SEQ ID NO: 114,
a heavy-chain variable region including a heavy chain CDR1 of SEQ ID NO: 5, a heavy chain CDR2 of SEQ ID NO: 23, and a heavy chain CDR3 of SEQ ID NO: 189, and a light-chain variable region including a light chain CDR1 of SEQ ID NO: 71, a light chain CDR2 of SEQ ID NO: 93, and a light chain CDR3 of SEQ ID NO: 114,
a heavy-chain variable region including a heavy chain CDR1 of SEQ ID NO: 5, a heavy chain CDR2 of SEQ ID NO: 23, and a heavy chain CDR3 of SEQ ID NO: 190, and a light-chain variable region including a light chain CDR1 of SEQ ID NO: 71, a light chain CDR2 of SEQ ID NO: 93, and a light chain CDR3 of SEQ ID NO: 114,
a heavy-chain variable region including a heavy chain CDR1 of SEQ ID NO: 5, a heavy chain CDR2 of SEQ ID NO: 23, and a heavy chain CDR3 of SEQ ID NO: 191, and a light-chain variable region including a light chain CDR1 of SEQ ID NO: 71, a light chain CDR2 of SEQ ID NO: 93, and a light chain CDR3 of SEQ ID NO: 114,
a heavy-chain variable region including a heavy chain CDR1 of SEQ ID NO: 5, a heavy chain CDR2 of SEQ ID NO: 23, and a heavy chain CDR3 of SEQ ID NO: 192, and a light-chain variable region including a light chain CDR1 of SEQ ID NO: 71, a light chain CDR2 of SEQ ID NO: 93, and a light chain CDR3 of SEQ ID NO: 114,
a heavy-chain variable region including a heavy chain CDR1 of SEQ ID NO: 5, a heavy chain CDR2 of SEQ ID NO: 23, and a heavy chain CDR3 of SEQ ID NO: 193, and a light-chain variable region including a light chain CDR1 of SEQ ID NO: 71, a light chain CDR2 of SEQ ID NO: 93, and a light chain CDR3 of SEQ ID NO: 114,
a heavy-chain variable region including a heavy chain CDR1 of SEQ ID NO: 5, a heavy chain CDR2 of SEQ ID NO: 23, and a heavy chain CDR3 of SEQ ID NO: 194, and a light-chain variable region including a light chain CDR1 of SEQ ID NO: 71, a light chain CDR2 of SEQ ID NO: 93, and a light chain CDR3 of SEQ ID NO: 114,
a heavy-chain variable region including a heavy chain CDR1 of SEQ ID NO: 5, a heavy chain CDR2 of SEQ ID NO: 23, and a heavy chain CDR3 of SEQ ID NO: 195, and a light-chain variable region including a light chain CDR1 of SEQ ID NO: 71, a light chain CDR2 of SEQ ID NO: 93, and a light chain CDR3 of SEQ ID NO: 114,
a heavy-chain variable region including a heavy chain CDR1 of SEQ ID NO: 5, a heavy chain CDR2 of SEQ ID NO: 23, and a heavy chain CDR3 of SEQ ID NO: 196, and a light-chain variable region including a light chain CDR1 of SEQ ID NO: 71, a light chain CDR2 of SEQ ID NO: 93, and a light chain CDR3 of SEQ ID NO: 114,
a heavy-chain variable region including a heavy chain CDR1 of SEQ ID NO: 5, a heavy chain CDR2 of SEQ ID NO: 23, and a heavy chain CDR3 of SEQ ID NO: 197, and a light-chain variable region including a light chain CDR1 of SEQ ID NO: 71, a light chain CDR2 of SEQ ID NO: 93, and a light chain CDR3 of SEQ ID NO: 114,
a heavy-chain variable region including a heavy chain CDR1 of SEQ ID NO: 5, a heavy chain CDR2 of SEQ ID NO: 23, and a heavy chain CDR3 of SEQ ID NO: 198, and a light-chain variable region including a light chain CDR1 of SEQ ID NO: 71, a light chain CDR2 of SEQ ID NO: 93, and a light chain CDR3 of SEQ ID NO: 114,
a heavy-chain variable region including a heavy chain CDR1 of SEQ ID NO: 5, a heavy chain CDR2 of SEQ ID NO: 23, and a heavy chain CDR3 of SEQ ID NO: 46, and a light-chain variable region including a light chain CDR1 of SEQ ID NO: 71, a light chain CDR2 of SEQ ID NO: 93, and a light chain CDR3 of SEQ ID NO: 199,
a heavy-chain variable region including a heavy chain CDR1 of SEQ ID NO: 5, a heavy chain CDR2 of SEQ ID NO: 23, and a heavy chain CDR3 of SEQ ID NO: 46, and a light-chain variable region including a light chain CDR1 of SEQ ID NO: 71, a light chain CDR2 of SEQ ID NO: 93, and a light chain CDR3 of SEQ ID NO: 200,
a heavy-chain variable region including a heavy chain CDR1 of SEQ ID NO: 5, a heavy chain CDR2 of SEQ ID NO: 23, and a heavy chain CDR3 of SEQ ID NO: 46, and a light-chain variable region including a light chain CDR1 of SEQ ID NO: 71, a light chain CDR2 of SEQ ID NO: 93, and a light chain CDR3 of SEQ ID NO: 201,
a heavy-chain variable region including a heavy chain CDR1 of SEQ ID NO: 5, a heavy chain CDR2 of SEQ ID NO: 23, and a heavy chain CDR3 of SEQ ID NO: 46, and a light-chain variable region including a light chain CDR1 of SEQ ID NO: 71, a light chain CDR2 of SEQ ID NO: 93, and a light chain CDR3 of SEQ ID NO: 202,
a heavy-chain variable region including a heavy chain CDR1 of SEQ ID NO: 4, a heavy chain CDR2 of SEQ ID NO: 24, and a heavy chain CDR3 of SEQ ID NO: 47, and a light-chain variable region including a light chain CDR1 of SEQ ID NO: 72, a light chain CDR2 of SEQ ID NO: 94, and a light chain CDR3 of SEQ ID NO: 115,
a heavy-chain variable region including a heavy chain CDR1 of SEQ ID NO: 6, a heavy chain CDR2 of SEQ ID NO: 25, and a heavy chain CDR3 of SEQ ID NO: 48, and a light-chain variable region including a light chain CDR1 of SEQ ID NO: 73, a light chain CDR2 of SEQ ID NO: 95, and a light chain CDR3 of SEQ ID NO: 112,
a heavy-chain variable region including a heavy chain CDR1 of SEQ ID NO: 7, a heavy chain CDR2 of SEQ ID NO: 26, and a heavy chain CDR3 of SEQ ID NO: 49, and a light-chain variable region including a light chain CDR1 of SEQ ID NO: 74, a light chain CDR2 of SEQ ID NO: 96, and a light chain CDR3 of SEQ ID NO: 116,
a heavy-chain variable region including a heavy chain CDR1 of SEQ ID NO: 8, a heavy chain CDR2 of SEQ ID NO: 27, and a heavy chain CDR3 of SEQ ID NO: 50, and a light-chain variable region including a light chain CDR1 of SEQ ID NO: 75, a light chain CDR2 of SEQ ID NO: 97, and a light chain CDR3 of SEQ ID NO: 117,
a heavy-chain variable region including a heavy chain CDR1 of SEQ ID NO: 9, a heavy chain CDR2 of SEQ ID NO: 28, and a heavy chain CDR3 of SEQ ID NO: 51, and a light-chain variable region including a light chain CDR1 of SEQ ID NO: 76, a light chain CDR2 of SEQ ID NO: 98, and a light chain CDR3 of SEQ ID NO: 118,
a heavy-chain variable region including a heavy chain CDR1 of SEQ ID NO: 10, a heavy chain CDR2 of SEQ ID NO: 29, and a heavy chain CDR3 of SEQ ID NO: 52, and a light-chain variable region including a light chain CDR1 of SEQ ID NO: 77, a light chain CDR2 of SEQ ID NO: 97, and a light chain CDR3 of SEQ ID NO: 119,
a heavy-chain variable region including a heavy chain CDR1 of SEQ ID NO: 11, a heavy chain CDR2 of SEQ ID NO: 30, and a heavy chain CDR3 of SEQ ID NO: 53, and a light-chain variable region including a light chain CDR1 of SEQ ID NO: 78, a light chain CDR2 of SEQ ID NO: 99, and a light chain CDR3 of SEQ ID NO: 120,
a heavy-chain variable region including a heavy chain CDR1 of SEQ ID NO: 11, a heavy chain CDR2 of SEQ ID NO: 31, and a heavy chain CDR3 of SEQ ID NO: 54, and a light-chain variable region including a light chain CDR1 of SEQ ID NO: 79, a light chain CDR2 of SEQ ID NO: 100, and a light chain CDR3 of SEQ ID NO: 121,
a heavy-chain variable region including a heavy chain CDR1 of SEQ ID NO: 12, a heavy chain CDR2 of SEQ ID NO: 32, and a heavy chain CDR3 of SEQ ID NO: 55, and a light-chain variable region including a light chain CDR1 of SEQ ID NO: 80, a light chain CDR2 of SEQ ID NO: 101, and a light chain CDR3 of SEQ ID NO: 122,
a heavy-chain variable region including a heavy chain CDR1 of SEQ ID NO: 3, a heavy chain CDR2 of SEQ ID NO: 33, and a heavy chain CDR3 of SEQ ID NO: 56, and a light-chain variable region including a light chain CDR1 of SEQ ID NO: 81, a light chain CDR2 of SEQ ID NO: 102, and a light chain CDR3 of SEQ ID NO: 123,
a heavy-chain variable region including a heavy chain CDR1 of SEQ ID NO: 13, a heavy chain CDR2 of SEQ ID NO: 34, and a heavy chain CDR3 of SEQ ID NO: 57, and a light-chain variable region including a light chain CDR1 of SEQ ID NO: 82, a light chain CDR2 of SEQ ID NO: 103, and a light chain CDR3 of SEQ ID NO: 124,
a heavy-chain variable region including a heavy chain CDR1 of SEQ ID NO: 14, a heavy chain CDR2 of SEQ ID NO: 35, and a heavy chain CDR3 of SEQ ID NO: 58, and a light-chain variable region including a light chain CDR1 of SEQ ID NO: 83, a light chain CDR2 of SEQ ID NO: 104, and a light chain CDR3 of SEQ ID NO: 125,
a heavy-chain variable region including a heavy chain CDR1 of SEQ ID NO: 1, a heavy chain CDR2 of SEQ ID NO: 36, and a heavy chain CDR3 of SEQ ID NO: 59, and a light-chain variable region including a light chain CDR1 of SEQ ID NO: 84, a light chain CDR2 of SEQ ID NO: 105, and a light chain CDR3 of SEQ ID NO: 126,
a heavy-chain variable region including a heavy chain CDR1 of SEQ ID NO: 6, a heavy chain CDR2 of SEQ ID NO: 23, and a heavy chain CDR3 of SEQ ID NO: 60, and a light-chain variable region including a light chain CDR1 of SEQ ID NO: 69, a light chain CDR2 of SEQ ID NO: 91, and a light chain CDR3 of SEQ ID NO: 127,
a heavy-chain variable region including a heavy chain CDR1 of SEQ ID NO: 15, a heavy chain CDR2 of SEQ ID NO: 37, and a heavy chain CDR3 of SEQ ID NO: 61, and a light-chain variable region including a light chain CDR1 of SEQ ID NO: 85, a light chain CDR2 of SEQ ID NO: 89, and a light chain CDR3 of SEQ ID NO: 128,
a heavy-chain variable region including a heavy chain CDR1 of SEQ ID NO: 16, a heavy chain CDR2 of SEQ ID NO: 38, and a heavy chain CDR3 of SEQ ID NO: 62, and a light-chain variable region including a light chain CDR1 of SEQ ID NO: 86, a light chain CDR2 of SEQ ID NO: 106, and a light chain CDR3 of SEQ ID NO: 129,
a heavy-chain variable region including a heavy chain CDR1 of SEQ ID NO: 17, a heavy chain CDR2 of SEQ ID NO: 39, and a heavy chain CDR3 of SEQ ID NO: 63, and a light-chain variable region including a light chain CDR1 of SEQ ID NO: 87, a light chain CDR2 of SEQ ID NO: 107, and a light chain CDR3 of SEQ ID NO: 130,
a heavy-chain variable region including a heavy chain CDR1 of SEQ ID NO: 4, a heavy chain CDR2 of SEQ ID NO: 19, and a heavy chain CDR3 of SEQ ID NO: 64, and a light-chain variable region including a light chain CDR1 of SEQ ID NO: 88, a light chain CDR2 of SEQ ID NO: 108, and a light chain CDR3 of SEQ ID NO: 131,
a heavy-chain variable region including a heavy chain CDR1 of SEQ ID NO: 18, a heavy chain CDR2 of SEQ ID NO: 40, and a heavy chain CDR3 of SEQ ID NO: 65, and a light-chain variable region including a light chain CDR1 of SEQ ID NO: 85, a light chain CDR2 of SEQ ID NO: 89, and a light chain CDR3 of SEQ ID NO: 128, or
a heavy-chain variable region including a heavy chain CDR1 of SEQ ID NO: 16, a heavy chain CDR2 of SEQ ID NO: 41, and a heavy chain CDR3 of SEQ ID NO: 66, and a light-chain variable region including a light chain CDR1 of SEQ ID NO: 72, a light chain CDR2 of SEQ ID NO: 109, and a light chain CDR3 of SEQ ID NO: 132.

The term "framework region" (FR) refers to a variable domain residue other than a CDR residue. Each variable domain typically has four FRs, identified as FR1, FR2, FR3, and FR4.

The binding affinity of the anti-FGFR3 antibody ranges from 10⁻⁵ M to 10⁻¹² M. For example, the binding affinity of the anti-FGFR3 antibody is 10⁻⁶ M to 10⁻¹² M, 10⁻⁷ M to 10⁻¹² M, 10⁻⁸ M to 10⁻¹² M, 10⁻⁹ M to 10⁻¹² M, 10⁻⁵ M to 10⁻¹¹ M, 10⁻⁶ M to 10⁻¹¹ M, 10⁻⁷ M to 10⁻¹¹ M, 10⁻⁸ M to 10⁻¹¹ M, 10⁻⁹ M to 10⁻¹¹ M, 10⁻¹⁰ M to 10⁻¹¹ M, 10⁻⁵ M to 10⁻¹⁰ M, 10⁻⁶ M to 10⁻¹⁰ M, 10⁻⁷ M to 10⁻¹⁰ M, 10⁻⁸ M to 10⁻¹⁰ M, 10⁻⁹ M to 10⁻¹⁰ M, 10⁻⁵ M to 10⁻⁹ M, 10⁻⁶ M to 10⁻⁹ M, 10⁻⁷ M to 10⁻⁹ M, 10⁻⁸ M to 10⁻⁹ M, 10⁻⁵ M to 10⁻⁸ M, 10⁻⁶ M to 10-⁸ M, 10⁻⁷ M to 10⁻⁸ M, 10⁻⁵ M to 10⁻⁷ M, 10⁻⁶ M to 10⁻⁷ M or 10⁻⁵ M to 10⁻⁶ M.

The antibody binding to FGFR3 or an antigen-binding fragment thereof may include a heavy-chain variable region including at least one amino acid sequence selected from the group consisting of SEQ ID NOS: 133 to 157, and SEQ ID NOS: 203 to 215. In addition, the antibody binding to FGFR3 or an antigen-binding fragment thereof may include a light-chain variable region including at least one amino acid sequence selected from the group consisting of SEQ ID NOS: 158 to 182, and SEQ ID NOS: 216 to 219.

In a specific embodiment according to the present invention, the antibody binding to FGFR3 or the antigen-binding fragment thereof may include:
the heavy-chain variable region of SEQ ID NO: 133 and the light-chain variable region of SEQ ID NO: 158;
the heavy-chain variable region of SEQ ID NO: 134 and the light-chain variable region of SEQ ID NO: 159;
the heavy-chain variable region of SEQ ID NO: 135 and the light-chain variable region of SEQ ID NO: 160;
the heavy-chain variable region of SEQ ID NO: 136 and the light-chain variable region of SEQ ID NO: 161;
the heavy-chain variable region of SEQ ID NO: 137 and the light-chain variable region of SEQ ID NO: 162;
the heavy-chain variable region of SEQ ID NO: 203 and the light-chain variable region of SEQ ID NO: 162;
the heavy-chain variable region of SEQ ID NO: 204 and the light-chain variable region of SEQ ID NO: 162;
the heavy-chain variable region of SEQ ID NO: 205 and the light-chain variable region of SEQ ID NO: 162;
the heavy-chain variable region of SEQ ID NO: 206 and the light-chain variable region of SEQ ID NO: 162;
the heavy-chain variable region of SEQ ID NO: 207 and the light-chain variable region of SEQ ID NO: 162;
the heavy-chain variable region of SEQ ID NO: 208 and the light-chain variable region of SEQ ID NO: 162;
the heavy-chain variable region of SEQ ID NO: 209 and the light-chain variable region of SEQ ID NO: 162;
the heavy-chain variable region of SEQ ID NO: 210 and the light-chain variable region of SEQ ID NO: 162;
the heavy-chain variable region of SEQ ID NO: 211 and the light-chain variable region of SEQ ID NO: 162;
the heavy-chain variable region of SEQ ID NO: 212 and the light-chain variable region of SEQ ID NO: 162;
the heavy-chain variable region of SEQ ID NO: 213 and the light-chain variable region of SEQ ID NO: 162;
the heavy-chain variable region of SEQ ID NO: 214 and the light-chain variable region of SEQ ID NO: 162;
the heavy-chain variable region of SEQ ID NO: 215 and the light-chain variable region of SEQ ID NO: 162;
the heavy-chain variable region of SEQ ID NO: 137 and the light-chain variable region of SEQ ID NO: 216;
the heavy-chain variable region of SEQ ID NO: 137 and the light-chain variable region of SEQ ID NO: 217;
the heavy-chain variable region of SEQ ID NO: 137 and the light-chain variable region of SEQ ID NO: 218;
the heavy-chain variable region of SEQ ID NO: 137 and the light-chain variable region of SEQ ID NO: 219;
the heavy-chain variable region of SEQ ID NO: 138 and the light-chain variable region of SEQ ID NO: 163;
the heavy-chain variable region of SEQ ID NO: 139 and the light-chain variable region of SEQ ID NO: 164;
the heavy-chain variable region of SEQ ID NO: 140 and the light-chain variable region of SEQ ID NO: 165;
the heavy-chain variable region of SEQ ID NO: 141 and the light-chain variable region of SEQ ID NO: 166;
the heavy-chain variable region of SEQ ID NO: 142 and the light-chain variable region of SEQ ID NO: 167;
the heavy-chain variable region of SEQ ID NO: 143 and the light-chain variable region of SEQ ID NO: 168;
the heavy-chain variable region of SEQ ID NO: 144 and the light-chain variable region of SEQ ID NO: 169;
the heavy-chain variable region of SEQ ID NO: 145 and the light-chain variable region of SEQ ID NO: 170;
the heavy-chain variable region of SEQ ID NO: 146 and the light-chain variable region of SEQ ID NO: 171;
the heavy-chain variable region of SEQ ID NO: 147 and the light-chain variable region of SEQ ID NO: 172;
the heavy-chain variable region of SEQ ID NO: 148 and the light-chain variable region of SEQ ID NO: 173;
the heavy-chain variable region of SEQ ID NO: 149 and the light-chain variable region of SEQ ID NO: 174;
the heavy-chain variable region of SEQ ID NO: 150 and the light-chain variable region of SEQ ID NO: 175;
the heavy-chain variable region of SEQ ID NO: 151 and the light-chain variable region of SEQ ID NO: 176;
the heavy-chain variable region of SEQ ID NO: 152 and the light-chain variable region of SEQ ID NO: 177;
the heavy-chain variable region of SEQ ID NO: 153 and the light-chain variable region of SEQ ID NO: 178;
the heavy-chain variable region of SEQ ID NO: 154 and the light-chain variable region of SEQ ID NO: 179;
the heavy-chain variable region of SEQ ID NO: 155 and the light-chain variable region of SEQ ID NO: 180;
the heavy-chain variable region of SEQ ID NO: 156 and the light-chain variable region of SEQ ID NO: 181; and
the heavy-chain variable region of SEQ ID NO: 157 and the light-chain variable region of SEQ ID NO: 182.

### scFv

The scFv is an antibody fragment having a construct of a single polypeptide chain including VH and VL domains of the antibody. The scFv may further include a polypeptide linker between the VH domain and the VL domain in order for the scFv to form a desired structure for antigen binding.

In one embodiment, in the single chain Fv (scFv) including the VH and VL domains of an antibody, the VH and VL domains may be linked to each other via a linker. The heavy-chain variable region including at least one amino acid sequence selected from the group consisting of SEQ ID NOS: 133 to 157 and SEQ ID NOS: 203 to 215 may be linked to a light-chain variable region including at least one amino acid sequence selected from the group consisting of SEQ ID NOS: 158 to 182 and SEQ ID NOS: 216 to 219 via a linker.

In a specific embodiment according to the present invention, the antibody binding to FGFR3 or the antigen-binding fragment thereof may include:
the heavy-chain variable region of SEQ ID NO: 133 and the light-chain variable region of SEQ ID NO: 158;
the heavy-chain variable region of SEQ ID NO: 134 and the light-chain variable region of SEQ ID NO: 159;
the heavy-chain variable region of SEQ ID NO: 135 and the light-chain variable region of SEQ ID NO: 160;
the heavy-chain variable region of SEQ ID NO: 136 and the light-chain variable region of SEQ ID NO: 161;
the heavy-chain variable region of SEQ ID NO: 137 and the light-chain variable region of SEQ ID NO: 162;
the heavy-chain variable region of SEQ ID NO: 203 and the light-chain variable region of SEQ ID NO: 162;
the heavy-chain variable region of SEQ ID NO: 204 and the light-chain variable region of SEQ ID NO: 162;
the heavy-chain variable region of SEQ ID NO: 205 and the light-chain variable region of SEQ ID NO: 162;
the heavy-chain variable region of SEQ ID NO: 206 and the light-chain variable region of SEQ ID NO: 162;
the heavy-chain variable region of SEQ ID NO: 207 and the light-chain variable region of SEQ ID NO: 162;
the heavy-chain variable region of SEQ ID NO: 208 and the light-chain variable region of SEQ ID NO: 162;
the heavy-chain variable region of SEQ ID NO: 209 and the light-chain variable region of SEQ ID NO: 162;
the heavy-chain variable region of SEQ ID NO: 210 and the light-chain variable region of SEQ ID NO: 162;
the heavy-chain variable region of SEQ ID NO: 211 and the light-chain variable region of SEQ ID NO: 162;
the heavy-chain variable region of SEQ ID NO: 212 and the light-chain variable region of SEQ ID NO: 162;
the heavy-chain variable region of SEQ ID NO: 213 and the light-chain variable region of SEQ ID NO: 162;
the heavy-chain variable region of SEQ ID NO: 214 and the light-chain variable region of SEQ ID NO: 162;
the heavy-chain variable region of SEQ ID NO: 215 and the light-chain variable region of SEQ ID NO: 162;
the heavy-chain variable region of SEQ ID NO: 137 and the light-chain variable region of SEQ ID NO: 216;
the heavy-chain variable region of SEQ ID NO: 137 and the light-chain variable region of SEQ ID NO: 217;
the heavy-chain variable region of SEQ ID NO: 137 and the light-chain variable region of SEQ ID NO: 218;
the heavy-chain variable region of SEQ ID NO: 137 and the light-chain variable region of SEQ ID NO: 219;
the heavy-chain variable region of SEQ ID NO: 138 and the light-chain variable region of SEQ ID NO: 163;
the heavy-chain variable region of SEQ ID NO: 139 and the light-chain variable region of SEQ ID NO: 164;
the heavy-chain variable region of SEQ ID NO: 140 and the light-chain variable region of SEQ ID NO: 165;
the heavy-chain variable region of SEQ ID NO: 141 and the light-chain variable region of SEQ ID NO: 166;
the heavy-chain variable region of SEQ ID NO: 142 and the light-chain variable region of SEQ ID NO: 167;
the heavy-chain variable region of SEQ ID NO: 143 and the light-chain variable region of SEQ ID NO: 168;
the heavy-chain variable region of SEQ ID NO: 144 and the light-chain variable region of SEQ ID NO: 169;
the heavy-chain variable region of SEQ ID NO: 145 and the light-chain variable region of SEQ ID NO: 170;
the heavy-chain variable region of SEQ ID NO: 146 and the light-chain variable region of SEQ ID NO: 171;
the heavy-chain variable region of SEQ ID NO: 147 and the light-chain variable region of SEQ ID NO: 172;
the heavy-chain variable region of SEQ ID NO: 148 and the light-chain variable region of SEQ ID NO: 173;
the heavy-chain variable region of SEQ ID NO: 149 and the light-chain variable region of SEQ ID NO: 174;
the heavy-chain variable region of SEQ ID NO: 150 and the light-chain variable region of SEQ ID NO: 175;
the heavy-chain variable region of SEQ ID NO: 151 and the light-chain variable region of SEQ ID NO: 176;
the heavy-chain variable region of SEQ ID NO: 152 and the light-chain variable region of SEQ ID NO: 177;
the heavy-chain variable region of SEQ ID NO: 153 and the light-chain variable region of SEQ ID NO: 178;
the heavy-chain variable region of SEQ ID NO: 154 and the light-chain variable region of SEQ ID NO: 179;
the heavy-chain variable region of SEQ ID NO: 155 and the light-chain variable region of SEQ ID NO: 180;
the heavy-chain variable region of SEQ ID NO: 156 and the light-chain variable region of SEQ ID NO: 181; and
the heavy-chain variable region of SEQ ID NO: 157 and the light-chain variable region of SEQ ID NO: 182.

The linker may be a peptide linker and may have a length of about 10-25 aa. For example, the linker may include hydrophilic amino acids such as glycine and/or serine, but is not limited thereto.

Specifically, the linker may include, for example, (GS)ₙ, (GGS)ₙ, (GSGGS)ₙ or (GnS)ₘ (in which n and m are each 1 to 10), but the linker may, for example, be (GₙS)ₘ (in which n and m are each 1 to 10) . Specifically, the linker may include GGGGS and may, for example, be GGGGSGGGGSGGGGS, represented by SEQ ID NO: 185, corresponding to three repeat units of GGGGS.

"Phage display" is a technique for displaying a mutant polypeptide as a fusion protein with at least a part of a coat protein on the surface of the particle of a phage, for example a fibrous phage. The usefulness of phage display is to rapidly and efficiently classify sequences that bind to target antigens with high affinity in large libraries of randomized protein mutants. Displaying peptides and protein libraries on phages has been used to screen millions of polypeptides in order to identify polypeptides with specific binding properties.

Phage display technology has offered a powerful tool for producing and screening novel proteins that bind to specific ligands (e.g., antigens). Using phage display technology, large libraries of protein mutants can be generated and sequences binding with high affinity to target antigens can be rapidly classified. The nucleic acid encoding mutant polypeptides is fused with a nucleic acid sequence encoding a viral coat protein, e.g., a gene III or gene VIII protein. A monophasic phage display system, in which a nucleic acid sequence encoding a protein or polypeptide is fused with a nucleic acid sequence encoding a part of the gene III protein, has been developed. In the monophasic display system, a fused gene is expressed at a low level, a wild-type gene III protein is also expressed, and thus particle infectivity is maintained.

It is important to demonstrate the expression of peptides on the fibrous phage surface and the expression of functional antibody fragments in the peripheral cytoplasm of *E. coli* for the development of antibody phage display libraries. Libraries of antibody- or antigen-binding polypeptides are produced through a number of methods, for example, methods of modifying a single gene by inserting a random DNA sequence or cloning a related gene sequence. Screening can be performed in the libraries regarding the expression of antibody- or antigen-binding proteins having desired characteristics.

Phage display technology has several advantages over conventional hybridomas and recombinant methods for producing antibodies having desired characteristics. This technique provides the generation of large antibody libraries with a variety of sequences within a short time without using animals. The production of hybridomas and the production of humanized antibodies may require a production time of several months. In addition, since no immunity is required, the phage antibody libraries can generate antibodies against antigens that are toxic or have low antigenicity. The phage antibody libraries can also be used to produce and identify novel therapeutic antibodies.

Techniques for generating human antibodies from immunized or non-immunized human, germline sequences, or naive B cell Ig repertoires using phage display libraries can be used. Naive or non-immunogenic antigen-binding libraries can be produced using various lymphatic tissues.

Techniques for identifying and separating high-affinity antibodies from phage display libraries are important for the isolation of new therapeutic antibodies. The isolation of high-affinity antibodies from the libraries depends on the size of the libraries, the production efficiency in bacterial cells and the variety of libraries. The size of the libraries is reduced by improper folding of the antibody- or antigen-binding protein and inefficient production due to the presence of the stop codon. Expression in bacterial cells may be inhibited by improper folding of the antibody- or antigen-binding domain. The expression can be improved by alternately mutating residues on the surface of the variable/constant interfaces or the selected CDR residues. The sequence of the framework region is an element that enables proper folding when generating antibody phage libraries in bacterial cells.

It is important to generate various libraries of antibody- or antigen-binding proteins in the separation of high-affinity antibodies. CDR3 regions have often been found to participate in antigen binding. Since the CDR3 region on the heavy chain varies considerably in terms of size, sequence and structural/dimensional morphology, various libraries can be produced using the same.

Also, diversity can be created by randomizing the CDR regions of variable heavy and light chains using all 20 amino acids at each position. The use of all 20 amino acids results in production of antibody sequences having increased diversity and an increased chance of identifying new antibodies.

The antibody or antibody fragment of the present invention may include the sequence of the anti-FGFR3 antibody mentioned herein as well as biological equivalents thereto, as long as it can specifically recognize FGFR3. For example, additional variations can be made to the amino acid sequence of the antibody in order to further improve the binding affinity and/or other biological properties of the antibody. Such variations include, for example, deletion, insertion and/or substitution of the amino acid sequence residues of the antibody. Such amino acid variations are based on the relative similarity of amino acid side chain substituents, such as the hydrophobicity, hydrophilicity, charge and size thereof. It can be seen through analysis of the size, shape and type of amino acid side chain substituents that all of arginine, lysine and histidine are positively charged residues; alanine, glycine and serine have similar sizes; and phenylalanine, tryptophan and tyrosine have similar shapes. Thus, based on these considerations, arginine, lysine and histidine; alanine, glycine and serine; and phenylalanine, tryptophan and tyrosine are considered to be biologically functional equivalents.

When taking into consideration variations having biologically equivalent activity, the antibody or a nucleotide molecule encoding the same according to the present invention is interpreted to include a sequence having substantial identity with the sequence set forth in the sequence number. The term "substantial identity" means that a sequence has a homology of at least 90%, most preferably a homology of at least 95%, at least 96%, at least 97%, at least 98% or at least 99%, when aligning the sequence of the present invention and any other sequence so as to correspond to each other as much as possible and analyzing the aligned sequence using algorithms commonly used in the art. Alignment methods for sequence comparison are well-known in the art. The NCBI Basic Local Alignment Search Tool (BLAST) is accessible through NCBI or the like, and can be used in conjunction with sequence analysis programs such as BLASTP, BLASTM, BLASTX, TBLASTN and TBLASTX over the Internet. BLAST is available at www.ncbi.nlm.nih.gov/BLAST/. A method of comparing sequence homology using this program can be found at www.ncbi.nlm.nih.gov/BLAST/blast help.html.

Based on this, the antibody or antigen-binding fragment thereof according to the present invention can have a homology of 90%, 91%, 92%, 93%, 94%, 95%, 96%, 97%, 98%, or 99% or more compared to the sequence disclosed herein or the entirety thereof. Such homology can be determined through sequence comparison and/or alignment by methods known in the art. For example, the percentage sequence homology of the nucleic acid or protein according to the present invention can be determined using a sequence comparison algorithm (i.e., BLAST or BLAST 2.0), manual alignment, or visual inspection.

In another aspect, the present invention is directed to a nucleic acid encoding the antibody or an antigen-binding fragment thereof. By isolating the nucleic acid encoding the antibody or antigen-binding fragment thereof according to the present invention, an antibody or antigen-binding fragment thereof can be produced in a recombinant manner.

The term "nucleic acid" is intended to encompass both DNA (gDNA and cDNA) and RNA molecules, and a nucleotide, which is a basic constituent unit of a nucleic acid, includes naturally derived nucleotides as well as analogues thereof, wherein sugar or base moieties are modified. The sequence of the nucleic acid encoding heavy- and light-chain variable regions of the present invention may vary. Such variation includes addition, deletion, or non-conservative or conservative substitution of nucleotides.

The DNA encoding the antibody can be easily separated or synthesized using conventional molecular biological techniques (for example, using an oligonucleotide probe capable of specifically binding to DNA encoding heavy- and light-chains of the antibody). Nucleic acids are isolated and inserted into replicable vectors for further cloning (amplification of DNA) or further expression. Based on this, in another aspect, the present invention is directed to a recombinant expression vector including the nucleic acid.

As used herein, the term "vector" refers to a means for expressing target genes in host cells, and includes plasmid vectors, cosmid vectors, and viral vectors such as bacteriophage vectors, adenovirus vectors, retroviral vectors and adeno-associated viral vectors. Vector components generally include, but are not limited to, one or more of the following components: signal sequences, replication origins, one or more antibiotic resistance marker genes, enhancer elements, promoters, and transcription termination sequences. The nucleic acid encoding the antibody is operably linked to promoters, transcription termination sequences or the like.

The term "operably linked" means a functional linkage between a nucleic acid expression regulation sequence (e.g., an array of promoter, signal sequence or transcription regulator binding sites) and another nucleic acid sequence, and enables the regulation sequence to regulate the transcription and/or translation of the other nucleic acid sequence.

When a prokaryotic cell is used as a host, it generally includes a potent promoter capable of conducting transcription (such as a tac promoter, a lac promoter, a lacUV5 promoter, an lpp promoter, a pLX promoter, a pRX promoter, a rac5 promoter, an amp promoter, a recA promoter, a SP6 promoter, a trp promoter, or a T7 promoter), a ribosome-binding site for initiation of translation, and a transcription/translation termination sequence. In addition, for example, when a eukaryotic cell is used as a host, it includes a promoter (*e.g.,* a metallothionein promoter, a β-actin promoter, a human hemoglobin promoter and a human muscle creatine promoter) derived from the genome of mammalian cells, or a promoter derived from a mammalian virus such as an adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, or Rous sarcoma virus (RSV) promoter, and generally has a polyadenylation sequence as a transcription termination sequence.

Optionally, the vector may be fused with another sequence in order to facilitate purification of the antibody expressed therefrom. The sequence to be fused therewith includes, for example, glutathione S-transferase (Pharmacia, USA), maltose-binding protein (NEB, USA), FLAG (IBI, USA), 6x His (hexahistidine; Qiagen, USA) and the like.

The vector includes antibiotic-resistance genes commonly used in the art as selectable markers, and examples thereof include genes conferring resistance to ampicillin, gentamycin, carbenicillin, chloramphenicol, streptomycin, kanamycin, geneticin, neomycin and tetracycline.

In another aspect, the present invention is directed to a cell transfected with the recombinant expression vector. The host cell used to produce the antibody of the present invention may be a prokaryote, yeast or higher eukaryotic cell, but is not limited thereto.

Prokaryotic host cells such as *Escherichia coli,* the genus *Bacillus,* such as *Bacillus subtilis* and *Bacillus thuringiensis, Streptomyces* spp., *Pseudomonas* spp. (for example, *Pseudomonas putida*)*, Proteus mirabilis* and *Staphylococcus* spp. (for example, *Staphylococcus carnosus*) can be used.

Interest in animal cells is the greatest, and examples of useful host cell lines include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

In another aspect, the present invention is directed to a method of producing an antibody or antigen-binding fragment thereof specifically binding to FGFR3 including culturing the host cells to produce an antibody, and isolating and purifying the produced antibody.

The host cells can be cultured in various media. Any commercially available medium can be used as a culture medium without limitation. All other essential supplements well-known to those skilled in the art may be included in appropriate concentrations. Culture conditions such as temperature and pH are those that are conventionally used with the host cells selected for expression, which will be apparent to those skilled in the art.

The recovery of the antibody or antigen-binding fragment thereof can be carried out, for example, by centrifugation or ultrafiltration to remove impurities and further purification of the resulting product using, for example, affinity chromatography. Other additional purification techniques such as anion or cation exchange chromatography, hydrophobic interaction chromatography and hydroxyapatite (HA) chromatography may be used.

### Bispecific or multispecific antibody

In another aspect, the present invention is directed to a bispecific antibody or multispecific antibody including the antibody or antigen-binding fragment thereof.

As used herein, the term "bispecific antibody" refers to an antibody having the ability to bind to or antagonize at least one target, and includes an antibody having the ability to bind to or antagonize two different targets, or an antibody in which an antibody having the ability to bind to one target is bound to a substance having the ability to antagonize another target.

As used herein, the term "multispecific antibody" refers to an antibody that has binding specificity for at least three different antigens. The multi-specific antibody may include a tri-specific or higher-specific antibody, for example, a tri-specific antibody, a tetra-specific antibody, or an antibody specific for five or more targets.

Antibodies belonging to bispecific or multispecific antibodies may be classified into scFv-based antibodies, Fab-based antibodies, and IgG-based antibodies. Bispecific or multispecific antibodies can inhibit or amplify two or more signals simultaneously and thus can be more effective than a monoantibody inhibiting/amplifying one signal. Compared to when each signal can be treated with each signal inhibitor, low-dose administration is possible and suppression/amplification of two or more signals in the same time and space is possible.

Methods of producing bispecific or multispecific antibodies are well known. Traditionally, recombinant production of bispecific antibodies is based on the co-expression of two or more immunoglobulin heavy/light chain pairs, under conditions in which the two or more heavy chains have different specificities.

In the scFv-based bispecific or multispecific antibodies, the VL and VH of each of different scFvs may be combined to produce hybrid scFv in the form of a heterodimer to thereby form a diabody, the different scFvs may be linked together to produce a tandem ScFv, the CH1 and CL of Fab may be expressed at the ends of each scFv to produce heterodimeric miniantibody, and some amino acids in the CH3 domain, which is the homodimeric domain of Fc, may be substituted to change into a heterodimeric structure in the form of "knob into hole" and express the changed CH3 domain in different ends of each scFv to produce a minibody in the form of a heterodimeric scFv.

A Fab-based bispecific or multispecific antibody may be produced in the form of a heterodimeric Fab by combining multiple Fabs for a specific antigen with each other using a disulfide bond or a mediator and may be produced in the form of a heterodimeric Fab with an antigen valency of 2 by expressing scFvs for different antigens in the terminal of the heavy or light chain of a specific Fab or in the form of a heterodimeric Fab with an antigen valency of 4 by forming a hinge region between Fab and scFv. In addition, a double-targeting bibody imparted with an antigen-binding valency of 3 by fusing scFvs for different antigens to the light and heavy chain ends of Fab, and a triple-targeting bibody imparted with an antigen-binding valency of 3 by fusing different scFvs to the light chain ends and heavy chain ends of Fab can be obtained by chemically conjugating three different Fabs.

A known method of producing IgG-based bispecific or multispecific antibodies includes producing hybrid hybridomas, also known as quadromas, by crossing mouse and rat hybridomas again by Trion Pharma Limited. In addition, bispecific antibodies may be produced in the so-called in a "Hole and Knob" manner, in a heterodimeric form by modifying some amino acids of the CH3 homodimeric domain of Fc for different heavy chains while sharing the light chain part. In addition to heterodimeric bispecific antibodies, two different types of scFvs can be fused to constant domains instead of the light and heavy chain variable domains of IgG, respectively, followed by expression, to produce homodimeric (scFv)4-IgG. In addition, ImClone reported a bispecific antibody prepared based on IMC-1C11, a chimeric monoclonal antibody against human VEGFR-2, by fusing only a single variable domain for a mouse platelet-derived growth factor receptor-a to the amino terminal of the light chain of the antibody. In addition, antibodies having multiple antigen-binding valencies to CD20 can be produced as an antibody through the so-called a "dock and lock (DNL)" method using the dimerization and docking domain (DDD) of the protein kinase A (PKA) R subunit and the anchoring domain of PKA.

A wide variety of recombinant antibody formats have been developed, for example bispecific or multispecific antibodies that are bivalent, trivalent or tetravalent or higher. For example, the recombinant antibody formats include bispecific or multispecific antibodies that are bivalent, trivalent or tetravalent or higher described in International Patent Application Publication Nos. WO 2001/077342, WO 2009/080251, WO 2009/080252, WO 2009/080253, WO 2009/080254, WO 2010/112193, WO 2010/115589, WO 2010/136172, WO 2010/145792, WO 2010/145793, and WO 2011/117330. Antibodies that are bivalent or higher, trivalent or higher and tetravalent or higher mean that two or more binding domains, three or more binding domains, or four or more binding domains, respectively, are present in the antibody molecule.

In a specific embodiment, the bispecific or multispecific antibody according to the present invention may include the anti-FGFR3 antibody or antigen-binding fragment, specifically, in the form of IgG complete antibody or a fragment thereof, for example, single-chain Fv, VH domain and/or VL domain, Fab or (Fab)2.

In addition, the bispecific or multispecific antibody may include an antibody that binds to an antibody targeting FGFR3 and an antibody targeting another substance, for example, an antibody targeting one or more selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptor, c-Met, EGFR, HER2, KDR, PDGFRa, NRP1, and MARCO, specifically, in the form of an IgG complete antibody or a fragment thereof, for example, single-chain Fv, a VH domain and/or a VL domain, Fab or (Fab)2.

The bispecific or multispecific antibody according to the present invention may include additional binding specificity induced or mediated by a target other than FGFR3.

For example, the bispecific antibody according to the present invention may simultaneously target FGFR3, and at least one selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptors, c-Met, EGFR, HER2, KDR, PDGFRa, NRP1, MARCO, and MARCO.

For example, the multispecific antibody according to the present invention may simultaneously target FGFR3, and at least one selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278(ICOS), CD27, CD154 (CD40L), CD357 (GITR), CD30, DR3, CD226 (DNAM1), CD96, CD200, CD200R, transferrin receptors, c-Met, EGFR, HER2, KDR, PDGFRa, NRP1, and MARCO.

### Immune cell engaging-bispecific or multispecific antibodies

In another aspect, the present invention is directed to an immune cell-engaging bispecific or multispecific antibody including a scFv including a scFv of the antibody and a second binding domain including at least one scFv of an antibody that binds to an immune cell-activating antigen.

A cytolytic synapse between cytotoxic T cells and cancer target cells is temporarily induced through the immune cell-engaging bispecific or multispecific antibody to release toxic substances.

In one embodiment, the immune cells may include one or more selected from the group consisting of T cells, NK cells, cytokine induced killer cells (CIK), activated cytotoxic T lymphocytes (CTL), macrophages, tumor-infiltrating lymphocytes (TIL), and dendritic cells.

In one embodiment, the immune cell-activating antigen may be selected from the following and an antibody that binds to the antigen may act as an immune cell engager:
T cell-activating antigens including CD3, TCRα, TCRβ, TCRγ, TCRξ, ICOS, CD28, CD27, HVEM, LIGHT, CD40, 4-1BB, OX40, DR3, GITR, CD30, TIM1, SLAM, CD2, or CD226;
NK cell activating antigens including NKp30, NKp40, NKp44, NKp46, NKG2D, DNAM1, DAP10, CD16 (e.g., CD16a, CD16b), CRTAM, CD27, PSGL1, CD96, CD100 (SEMA4D), NKp80, CD244 (SLAMF4 or 2B4), SLAMF6, SLAMF7, KIR2DS2, KIR2DS4, KIR3DS1, KIR2DS3, KIR2DS5, KIR2DS1, CD94, NKG2C, NKG2E or CD160;
B-cell activating antigens including CX40, CD40 or CD70;
macrophage activating antigens including CD2 agonist, CD40, CD70, Toll-like receptor (TCR) agonist, CD47, STING or OX40L; or
dendritic cell-activating antigens including CD2 agonist, OX40, OX40L, 41BB agonist, TCR agonist, CD47 agonist, or STING agonist.

Immune cell engagers are described in detail in US Patent Application Publication No. 2017/0368169, which is incorporated herein by reference.

Specifically, the immune cell-engaging bispecific or multispecific antibody includes a tandem scFv and may bind to the following antigens and surface antigens on cancer cells. The surface antigen on the cancer cell is FGFR3 targeted by the antibody according to the present invention:
CD3, TCRα, TCRβ, TCRγ, TCRξ, ICOS, CD28, CD27, HVEM, LIGHT, CD40, 4-1BB, OX40, DR3, GITR, CD30, TIM1, SLAM, CD2, or CD226;
NKp30, NKp40, NKp44, NKp46, NKG2D, DNAM1, DAP10, CD16 (e.g., CD16a, CD16b), CRTAM, CD27, PSGL1, CD96, CD100 (SEMA4D), NKp80, CD244 (SLAMF4 or 2B4), SLAMF6, SLAMF7, KIR2DS2, KIR2DS4, KIR3DS1, KIR2DS3, KIR2DS5, KIR2DS1, CD94, NKG2C, NKG2E or CD160;
OX40, CD40 or CD70;
CD2 agonist, CD40, CD70, TCR (toll-like receptor) agonist, CD47, STING or OX40L; or
CD2 agonist, OX40, OX40L, 41BB agonist, TCR agonist, CD47 agonist, or STING agonist.

The immune cell-engaging bispecific or multispecific antibody may, for example, include a structure of VL(FGFR3)-VH(FGFR3)-VH(CD3 or CD16A)-VL(CD3 or CD16A), VH(FGFR3)-VL(FGFR3)-VH(CD3 or CD16A)-VL(CD3 or CD16A), VH(CD3 or CD16A)-VL(CD3 or CD16A)-VH(FGFR3)-VL(FGFR3) or VH(CD3 or CD16A)-VL(CD3 or CD16A)-VL(FGFR3)-VH(FGFR3).

The scFv may include a heavy-chain variable region including at least one amino acid sequence selected from the group consisting of SEQ ID NOS: 133 to 157, and SEQ ID NOS: 203 to 215, and a light-chain variable region including at least one amino acid sequence selected from the group consisting of SEQ ID NOS: 158 to 182, and SEQ ID NOS: 216 to 219. The heavy-chain variable region and the light-chain variable region may be linked via a linker.

The linker may be a peptide linker and may have a length of about 10-25 aa. For example, the linker may include hydrophilic amino acids such as glycine and/or serine.

Specifically, the linker may include, for example, (GS)ₙ, (GGS)ₙ, (GSGGS)ₙ or (GnS)ₘ (in which n and m are each 1 to 10), but the linker may, for example, be (GnS)ₘ (in which n and m are each 1 to 10). Specifically, the linker may include GGGGS and may, for example, be GGGGSGGGGSGGGGS represented by SEQ ID NO: 185, corresponding to three repeat units of GGGGS.

Examples of the immune cell-engaging bispecific or multispecific antibody include: blinatumomab (Amgen) that binds to CD3 and CD19; solitomab (Amgen) that binds to CD3 and EpCAM; MEDI 565 (MedImmune, Amgen) that binds to CD3 and CEA; and BAY2010112 (Bayer, Amgen) that binds CD3 and PSMA. Examples of DART include: MGD006 (Macrogenics) that binds to CD3 and CD123; and MGD007 (Macrogenics) that bind to CD3 and gpA33. Examples of TandAb include: Affimed Therapeutics (AFM11) that binds to CD3 and CD19; and Affimed Therapeutics (AFM13) that binds to CD30 and CD16A.

### Antibody-drug conjugate (ADC)

In another aspect, the present invention is directed to an antibody-drug conjugate (ADC) including a drug and the antibody or antigen-binding fragment thereof conjugated to each other.

The antibody-drug conjugate requires that the anticancer drug stably bind to the antibody until the anticancer drug is delivered to the target cancer cell. The drug delivered to the target should be released from the antibody to thereby induce the death of the target cell. For this purpose, the drug should stably bind to the antibody, and should have sufficient cytotoxicity to induce the death of the target cell when released from the target cell.

In an embodiment, the antibody may be bound to the drug via a linker. The linker is a site for linking the anti-FGFR3 antibody to the drug, and allows the drug to be released from the antibody in a form that can be cleaved under intracellular conditions, that is, in the intracellular environment. In addition, the antibody should be stable during systemic circulation, and the binding of the linker to the drug should not affect the stability and pharmacokinetics of the antibody.

For example, the linker may include a cleavable linker or a non-cleavable linker. The cleavable linker such as a peptide linker may be cleaved by intracellular peptidases or proteases, such as lysosome or endosome proteases. In the non-cleavable linker, for example, a thioether linker, the drug may be released after the antibody is non-selectively cleaved by intracellular hydrolysis.

In one embodiment, the cleavable linker may include a peptide linker. The peptide linker has at least two amino acid lengths. For example, the cleavable linker may include a dipeptide of Val-Cit, Val-Ala, Val-Cit or Phe-Leu, or Gly-Phe-Leu-Gly. Examples of linkers are described in detail in International Patent Application Publication No. WO 2004/010957, which may be incorporated herein by reference.

The antibody region of the antibody-drug conjugate binds to the antigen of the target cancer cell to form an ADC-antigen complex and then is internalized into the cancer cell through an endosome-lysosome pathway. In this case, the intracellular release of cytotoxic drugs is regulated by the internal environment of endosomes/lysosomes.

In an embodiment, the cleavable linker is sensitive to pH and may be sensitive to hydrolysis at a predetermined pH. Generally, the pH-sensitive linker is a linker that may be hydrolyzed under acidic conditions. Examples of acid-labile linkers that may be hydrolyzed in lysosomes may include hydrazone, semicarbazone, thiosemicarbazone, cis-aconitic amide, orthoester, acetal, ketal, and the like.

In another embodiment, the linker may also be cleaved under reducing conditions, and may, for example, be a disulfide linker. A variety of disulfide bonds may be formed using N-succinimidyl-S-acetylthioacetate (SATA), N-succinimidyl-3-(2-pyridyldithio) propionate (SPDP), N-succinimidyl-3-(2-pyridyldithio)butyrate (SPDB) and N-succinimidyl-oxycarbonyl-alpha-methyl-alpha-(2-pyridyl-dithio)toluene (SMPT). This disulfide linker may be cleaved by disulfide exchange with thiols of intracellular glutathione.

The drug and/or drug linker may be randomly conjugated through the lysine of the antibody, or may be conjugated through cysteine, which is exposed when the disulfide bond chain is reduced. In some cases, the drug-linker may be conjugated through cysteine present in a genetically engineered tag, *e.g.,* a peptide or protein. The genetically engineered tag, *e.g.,* peptide or protein, may include an amino acid motif that can be recognized, for example, by an isoprenoid transferase. The peptide or protein has a deletion at the carboxyl terminus of the peptide or protein or an addition at the carboxyl (C) terminus of the peptide or protein through a covalent bond of the spacer unit.

The peptide or protein may be directly covalently bonded to an amino acid motif, or may be linked to the amino acid motif through a covalent bond with the spacer unit. The amino acid spacer unit includes 1 to 20 amino acids, and is preferably a glycine unit.

The isoprenoid transferase may be, for example, farnesyl protein transferase (FTase) or geranylgeranyl transferase (GGTase), and FTase and GGTase I may recognize a CAAX motif in the aforementioned Formula 1, and GGTase II may recognize XXCC, XCXC or CXX motifs, wherein C is cysteine, A is an aliphatic amino acid, and X is an amino acid that determines the substrate specificity of isoprenoid transferase.

In another embodiment, the linker may include a beta-glucuronide linker that is present in multiple copies in the lysosome, or is recognized and hydrolyzed by beta-glucuronidase that is overexpressed in some tumor cells. Unlike the peptide linker, this linker has an advantage of increasing the solubility of the antibody-drug composite when bound to a drug having high hydrophobicity due to the high hydrophilicity thereof.

In this regard, in the present invention, the beta-glucuronide linker disclosed in International Patent Publication No. WO 2015-182984, which is incorporated herein by reference, is, for example, a beta-glucuronide linker including a self-immolative group.

In addition, the linker may be, for example, a non-cleavable linker, and the drug may be capable of being released merely through a single step of hydrolyzing the antibody to produce, for example, an amino acid-linker-drug conjugate. This type of linker may be a thioether group or a maleimidocaproyl group and may remain stable in the blood.

In one embodiment of the present invention, the linker and the drug may be linked randomly through a cysteine exposed when the disulfide chain of the antibody is reduced or by introduction of an antibody terminal-binding peptide having the sequence GGGGGGGCVIM.

The drug (including D of Formula 1) is an agent that exhibits a pharmacological effect and may be bound to an antibody. Specifically, the drug may be a chemotherapeutic agent, toxin, microRNA (miRNA), siRNA, shRNA or a radioactive isotope. The chemotherapeutic agent may be a cytotoxic agent or an immunosuppressive agent. Specifically, the chemotherapeutic agent may include a microtubulin inhibitor, a mitotic inhibitor, a topoisomerase inhibitor, or a chemotherapeutic agent capable of functioning as a DNA intercalator. The chemotherapeutic agent may also include an immunomodulatory compound, an anticancer agent, an antiviral agent, an antibacterial agent, an antifungal agent, an anthelmintic agent or a combination thereof.

The anticancer agent may, for example, include at least one selected from the group consisting of maytansinoid, auristatin (including MMAE, and MMAF), aminopterin, actinomycin, bleomycin, thalidomide, camptothecin, N8-acetylspermidine, 1-(2 chloroethyl)-1,2-dimethyl sulfonyl hydrazide, esperamicin, etoposide, 6-mercaptopurine, dolastatin, trichothecene, calicheamicin, taxol, taxane, paclitaxel, docetaxel, methotrexate, vincristine, vinblastine, doxorubicin, melphalan, mitomycin A, mitomycin C, chlorambucil, duocarmycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, Cytoxan, etoposide, 5-fluorouracil, bis-chloroethylnitrosourea (CNU), irinotecan, camptothecin, bleomycin, idarubicin, daunorubicin, dactinomycin, plicamycin, mitoxantrone, asparaginase, vinorelbine, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, EICAR (5-ethynyl-1-beta-D-ribofuranosylimidazole-4-carboxamide), hydroxyurea, deferoxamine, floxuridine, doxifluridine, raltitrexed, cytarabine(ara C), cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, interferon-a, interferon-γ, tumor necrosis factors, gemcitabine, Velcade, Revlimid, Thalomid, lovastatin, 1-methyl-4-phenylpyridinium ion, staurosporine, actinomycin D, dactinomycin, bleomycin A2, bleomycin B2, peplomycin, epirubicin, pirarubicin, zorubicin, mitoxantrone, verapamil, and thapsigargin, nucleases and toxins derived from bacteria or plants and animals, but is not limited thereto.

The anti-inflammatory agent may include, but is not limited to, steroid agents that bind to glucocorticoid receptors to reduce inflammation or swelling, non-steroidal anti-inflammatory drugs (NSAIDs) that relieve pain in response to cyclic oxygenase (COX) that synthesizes prostaglandins that cause inflammation, or immune-specific anti-inflammatory drugs (ImSAIDs) that change activation and transport of inflammatory cells, but are not limited thereto.

The therapeutic agent for immune diseases may include azathioprine, chlorambucil, cyclophosphamide, cyclosporine, mycophenolate, azathioprine, or methotrexate, but is not limited thereto.

Specifically, the immunosuppressive agent may be tacrolimus as a calcineurin inhibitor, mycophenolate mofetil, as a gene synthesis inhibitor, or prednisone as an anti-inflammatory steroid, but is not limited thereto.

In some cases, the drug may have at least one nucleophile group selected from the group consisting of amine, thiol, hydroxyl, hydrazide, oxime, hydrazine, thiosemicarbazone, hydrazine carboxylate and aryl hydrazide groups, which can react with an electrophilic group on the linker and the linker reagent to form a covalent bond.

In a specific embodiment according to the present invention, an ADC was prepared by linking the antibody or antigen-binding fragment thereof according to the present invention to a drug, for example, auristatin (MMAE), through the MC-vc-PAB linker. It was found that the ADC exhibited the desired cytotoxicity.

### Chimeric antigen receptor (CAR)

In another aspect, the present invention is directed to a chimeric antigen receptor (CAR) including an extracellular domain including an antigen-binding site, a transmembrane domain, and an intracellular signaling domain, wherein the antigen-binding site of the extracellular domain is a scFv of the antibody.

A chimeric antigen receptor (CAR) is a synthetic construct designed to induce an immune response against a target antigen and cells expressing the antigen. The CAR includes an extracellular domain, a transmembrane domain and an intracellular signaling domain. Cancer cells can be killed by introducing a gene encoding a receptor that recognizes a cancer cell surface antigen that is specifically expressed on the surface of cancer cells into immune cells. Through immune cells containing receptors that bind to antigens specifically expressed in cancer cells, an immune response can be induced by targeting only cancer cells. The CAR includes the scFv of the anti-FGFR3 antibody according to the present invention as an antigen recognition site in the extracellular domain.

The 1^{st}-generation CAR includes an extracellular domain including an antigen recognition site specifically expressed in cancer cells, a transmembrane domain, and an intracellular signaling domain, and uses only CD3ζ as the signaling domain, but has drawbacks of insignificant therapeutic effect on cancer and short duration. Such 1^{st} generation CARs are specifically described in US Pat. No. 6,319,494, which is incorporated herein by reference.

In order to improve reactivity to immune cells, a 2^{nd}-generation CAR was prepared by combining a costimulatory domain (CD28 or CD137/4-1BB) with CD3ζ. Compared to the 1^{st}-generation CAR, the 2^{nd}-generation CAR has a greatly increased number of CAR-containing immune cells remaining in the body. The 2^{nd}-generation CAR used one costimulatory domain, whereas the 3^{rd}-generation CAR used two or more costimulatory domains. In order to achieve expansion and persistence of immune cells containing the CAR *in vivo,* the costimulatory domain may be combined with 4-1BB, CD28, or OX40. The 2^{nd}-generation CAR is specifically described in U.S. Patent Nos. 7,741,465, 7,446,190 or 9,212,229 and the 3^{rd}-generation CAR is specifically described in U.S. Patent No. 8,822,647, which is incorporated herein by reference.

The 4^{th}-generation CAR includes additional genes encoding cytokines such as IL-12 or IL-15 to additionally express CAR-based immune proteins of cytokines, and the 5^{th} generation CAR contains an interleukin receptor chain such as IL-2Rβ to enhance immune cells. The 4^{th} generation CAR and the 5^{th} generation CAR are described in detail in US Patent No. 10,316,102 and US Patent No. 10,336,810, respectively, which are incorporated herein by reference.

In an embodiment, the antigen-binding site of the extracellular domain is an antibody scFv. The VH and VI domains in the scFv including VH and VI domains of the antibody may be linked via a linker. The heavy-chain variable region including at least one amino acid sequence selected from the group consisting of SEQ ID NOS: 133 to 157, and SEQ ID NOS: 203 to 215 may be linked to the light-chain variable region including at least one amino acid sequence selected from the group consisting of SEQ ID NOS: 158 to 182, and SEQ ID NOS: 216 to 219 via a linker.

The linker may be a peptide linker and may have a length of about 10-25 aa. For example, the linker may include hydrophilic amino acids such as glycine and/or serine.

Specifically, the linker may include, for example, (GS)ₙ, (GGS)ₙ, (GSGGS)ₙ or (GnS)ₘ (in which n and m are each 1 to 10), but the linker may, for example, be (GnS)ₘ (in which n and m are each 1 to 10). Specifically, the linker may include GGGGS and may, for example, be GGGGSGGGGSGGGGS represented by SEQ ID NO: 185, corresponding to three repeat units of GGGGS.

The transmembrane domain may be derived from natural or synthetic sources. When the source is natural, the domain may be derived from any membrane-bound or transmembrane protein. The transmembrane domain may include T-cell receptors, CD28, CD3 epsilon, CD45, CD4, CD5, CD8, CD9, CD16, CD22, CD33, CD37, CD64, CD80, CD86, CD134, CD137, CD154, alpha, beta or zeta chain of ICOS. When the transmembrane domain is synthesized, it may include hydrophobic residues such as leucine and valine, or may include peptides including phenylalanine, tryptophan, and valine at each end. A short oligo- or polypeptide linker, between 2 and 10 amino acids in length, may form a bond between the transmembrane domain and the cytoplasmic signaling domain of the CAR. A glycine-serine peptide may be used as a linker.

The signaling domain may induce activation of the normal effector function of the immune cell in which the CAR is located. For example, the signaling domain may induce cytolytic activation or helper activation through secretion of cytokines. The signaling domain may include a truncated fragment of an intracellular signaling domain sufficient to transduce an effector function signal.

The signaling domain may include the cytoplasm of co-receptor and the T cell receptor (TCR) that interact with each other to initiate signaling after antigen receptor engagement.

It is also known that signals generated through the TCR alone are insufficient and co-stimulatory signals are required for full activation of T cells. Thus, T cell activation may involve initiating antigen-dependent primary activation via the TCR and acting in an antigen-dependent manner to provide secondary or co-stimulatory signals. Primary cytoplasmic signaling sequences regulate primary activation of the TCR complex either in a stimulatory manner or in an inhibitory manner. Primary cytoplasmic signaling sequences that act in the stimulatory manner may contain signaling motifs known as immunoreceptor tyrosine-based activation motifs or ITAMs. Examples of ITAMs containing primary cytoplasmic signaling sequences may include TCR zeta, FcR gamma, FcR beta, CD3 gamma, CD3 delta, CD3 epsilon, CD5, CD22, CD79a, CD79b, and CD66d.

Optionally, the cytoplasmic domain of the CAR may include a portion of the CD3 zeta chain and a costimulatory signaling region. The costimulatory signaling region refers to a portion of a CAR that includes the intracellular domain of a costimulatory molecule. For example, the cytoplasmic domain of the CAR may include CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, and a ligand that specifically binds to CD83, and the like. The cytoplasmic signaling sequence within the cytoplasmic signaling portion of the CAR may be linked via a peptide linker including 2 to 10 amino acids, for example, glycine-serine.

In another aspect, the present invention is directed to an immune cell into which the chimeric antigen receptor is introduced.

The immune cells are capable of inducing immunity to induce a desired cancer treatment effect and may, for example, include, but are not limited to, one or more selected from the group consisting of T cells, NK cells, cytokine induced killer cells (CIK), activated cytotoxic T lymphocytes (CTL), macrophages, tumor-infiltrating lymphocytes (TIL) and dendritic cells.

In some cases, the immune cell may further include a chimeric antigen receptor (CAR) containing, as an antigen-binding site of the extracellular domain, a scFv for an antibody other than the anti-FGFR3 antibody.

The antibody other than the anti-FGFR3 antibody may, for example, be an antibody or antigen-binding fragment thereof targeting at least one selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptors, c-Met, EGFR, HER2, KDR, PDGFRa, NRP1, and MARCO.

### Endoplasmic reticulum (vesicle) and exosomes

In another aspect, the present invention is directed to an endoplasmic reticulum (vesicle) in which the antibody or antigen-binding fragment thereof is exposed outside or present therein.

The vesicle can directly introduce a target protein into cells through a cell membrane, and includes an antibody or antigen-binding fragment thereof according to the present invention.

The vesicle according to the present invention may be in the form of a cell membrane and a target agent to be delivered. Examples of the vesicle are described in detail in US Patent Publication No. 2018/0177725, which is incorporated herein by reference.

The vesicle may have an average diameter of, for example, 40 to 500 nm, specifically 100 to 300 nm, 80 to 200 nm, or 100 nm. The vesicle may have an average diameter of, for example, about 100, 200, 300, 400 or 500 nm.

The vesicle may not include other intracellular organelles such as mitochondria, lysosomes, or the Golgi apparatus in addition to the cell membrane and the antibody or antigen-binding fragment thereof according to the present invention.

The vesicle may be divided into internal and external regions via a membrane interposed therebetween and the antibody or antigen-binding fragment thereof may exist in the membrane and be exposed to the outside or present inside, or may be isolated from the membrane and present inside the vesicle.

The membrane of the vesicle may vary depending on the target to which the antibody or antigen-binding fragment thereof is applied, and may be, for example, a cancer cell membrane in order to deliver cancer cells expressing FGFR3 to which the antibody or antigen-binding fragment thereof according to the present invention binds, but is not limited thereto.

The cancer cells may, for example, include cancer cells that induce Hodgkin's lymphoma, non-Hodgkin's lymphoma (e.g., B cell lymphoma, diffuse large B cell lymphoma, follicular lymphoma, chronic lymphocytic leukemia, mantle cell lymphoma, marginal zone B cell lymphoma, Burkitt's lymphoma, lymphoplasmacytic lymphoma, and hairy cell leukemia), acute myelogenous leukemia, chronic myelogenous leukemia, myelodysplastic syndrome, multiple myeloma, or acute lymphocytic leukemia.

In addition, the cancer cells may, for example, include cancer cells that induce solid cancers such as ovarian cancer, rectal cancer, gastric cancer, testicular cancer, anal cancer, uterine cancer, colon cancer, rectal cancer, renal cell carcinoma, liver cancer, lung non-small cell carcinoma, small intestine cancer, esophageal cancer, melanoma, Kaposi's sarcoma, endocrine cancer, thyroid cancer, parathyroid cancer, adrenal cancer, bone cancer, pancreatic cancer, skin cancer, head and neck cancer, skin or intraocular malignant melanoma, cervical cancer, brain stem glioma, pituitary adenocarcinoma, epidermal cancer, cervical squamous cell carcinoma, fallopian tube carcinoma, endometrial carcinoma, vaginal carcinoma, soft tissue sarcoma, urethral cancer, vulvar carcinoma, penile cancer, bladder cancer, kidney or ureter cancer, renal pelvic carcinoma, spinal tumor, neoplasia of the central nervous system (CNS), primary CNS lymphoma, angiogenic tumor, and metastatic lesion of the cancers.

Furthermore, the cancer cells may include, for example, cancer cells that cause glioblastoma, lung cancer, bladder cancer, oral cancer, head and neck squamous cell cancer, gallbladder cancer or cervical cancer.

The vesicle may be, for example, an exosome. The exosome refers to a small membrane-structured vesicle having a size of 50 to 200 nm that contains proteins, DNA, RNA, or the like in order to transmit signals between cells and is secreted out of the cell.

The exosome may include various proteins, DNA, RNA, and the like in cells. Substances included in these exosomes and secreted out of cells are re-introduced into other cells by fusion with the cell membrane or endocytosis, and serve as intercellular communicators. In addition, substances included in these exosomes and secreted out of cells may be analyzed and used to diagnose a specific disease.

According to the present invention, the antibody or antigen-binding fragment thereof is exposed outside the exosome or present therein. The exosome has a membrane structure to distinguish between the inside and outside, and the antibody or antigen-binding fragment thereof exists in the exosome membrane and is exposed to the outside or present inside, or is isolated from the membrane and is present inside the exosome.

The exosomes are produced by loading specific drugs on immune cell/stem cell-derived artificial exosomes having various targeting characteristics to deliver the result to the desired lesion tissue in the human body. As a result, it is possible to dramatically reduce side effects on other tissues and maximize therapeutic efficacy using a small amount of drug. For example, such an exosome is described in detail, for example, in International Patent Application Publication No. WO 2011/002239, which is incorporated herein by reference.

When the antibody or antigen-binding fragment thereof is present in the exosome membrane and exposed to the outside or present inside, the antibody or antigen-binding fragment thereof is expressed in the form of a fusion to an exosome-specific marker, and the antibody or antigen-binding fragment thereof can be captured inside the exosome. When the exosome membrane is fused to the cell membrane of the cell in which FGFR3 is expressed, the antibody or antigen-binding fragment thereof may be delivered to the cell.

The antibody or antigen-binding fragment thereof may be displayed on the surface of the exosome. For example, the PTGFRN (prostaglandin F2 receptor negative regulator) protein may be expressed on the surface of exosome and other target proteins may be fused to the PTGFRN protein. Such exosomes are described in detail in International Patent Application Publication Nos. WO 2014/076137 and WO 2018/09119, which are incorporated herein by reference.

Exosomes where the antibody or antigen-binding fragment thereof is present inside may be prepared by temporarily binding the antibody or antigen-binding fragment thereof to the marker protein using a light-specific binding protein. The target protein may be captured inside the exosome such that it is not bound to the membrane. When the exosomes are delivered into cells by internalization, the exosomes may be degraded and the antibody or antigen-binding fragment thereof may be delivered to the cells.

The process of allowing the antibody or antigen-binding fragment thereof to exist inside is described in detail in International Patent Application Publication Nos. WO 2016/178532 and WO 2018/062973, which are incorporated herein by reference.

For example, the light-specific binding proteins CIBN and CRY2 may be used. Specifically, the CIBN is expressed in the form of a fusion with CD9, which is one of the marker proteins, and the CRY2 is expressed in the form of a fusion with the antibody or antigen-binding fragment thereof. For this purpose, genes encoding this fusion protein may be introduced into exosome-producing cells. The CIBN-CD9 fusion protein expressed in the exosome-producing cells is incorporated into the exosomes due to CD9. At this time, when the cells are irradiated with blue LED light, the target protein-CRY2 fusion protein expressed in the exosome-producing cells is bound to the CIBN domain fused to CD9, a reversibly inducible fusion protein in the form of target protein-CRY2-CIBN-CD9 is formed, and this fusion protein may be incorporated inside the exosome due to CD9. After the exosomes in which the target protein is present are produced, if the blue light is not irradiated any further, CIBN-CRY2 binding is released, and the target protein may be present inside such that it is not bound to the cell membrane of the exosomes.

### Therapeutic composition

In another aspect, the present invention is directed to a composition for treating cancer, or an inflammatory or immune disease containing the antibody or antigen-binding fragment thereof, the bispecific or multispecific antibody including the antibody or antigen-binding fragment thereof, the antibody-drug conjugate including the antibody or antigen-binding fragment thereof, the chimeric antigen receptor including the antibody or antigen-binding fragment thereof, or immune cellsincluding the chimeric antigen receptor.

In another aspect, the present invention is directed to a pharmaceutical composition for preventing or treating cancer including: (a) a pharmaceutically effective amount of the antibody to FGFR3 or antigen-binding fragment thereof according to the present invention, the bispecific or multispecific antibody including the antibody or antigen-binding fragment thereof, the antibody-drug conjugate including the antibody or antigen-binding fragment thereof, the chimeric antigen receptor including the antibody or antigen-binding fragment thereof, or immune cellsincluding the chimeric antigen receptor; and (b) a pharmaceutically acceptable carrier. In another aspect, the present invention is directed to a method for preventing or treating cancer including administering, to a cancer patient, the antibody to FGFR3 or antigen-binding fragment thereof according to the present invention, the bispecific or multispecific antibody including the antibody or antigen-binding fragment thereof, the antibody-drug conjugate including the antibody or antigen-binding fragment thereof, the chimeric antigen receptor including the antibody or antigen-binding fragment thereof, or immune cellsinto including the chimeric antigen receptor.

As used herein, the term "prevention" refers to any action causing the suppression of growth of cancer or the delay of progression of cancer by administration of the composition according to the present invention. The term "treatment" means suppression of the progression of cancer or alleviation or elimination of cancer.

The cancer may, for example, include Hodgkin's lymphoma, non-Hodgkin's lymphoma (e.g., B cell lymphoma, diffuse large B cell lymphoma, follicular lymphoma, chronic lymphocytic leukemia, mantle cell lymphoma, marginal zone B cell lymphoma, Burkitt's lymphoma, lymphoplasmacytic lymphoma, and hairy cell leukemia), acute myelogenous leukemia, chronic myelogenous leukemia, myelodysplastic syndrome, multiple myeloma, or acute lymphocytic leukemia.

The cancer may, for example, include ovarian cancer, rectal cancer, gastric cancer, testicular cancer, anal cancer, uterine cancer, colon cancer, rectal cancer, renal cell carcinoma, liver cancer, lung non-small cell carcinoma, small intestine cancer, esophageal cancer, melanoma, Kaposi's sarcoma, endocrine cancer, thyroid cancer, parathyroid cancer, adrenal cancer, bone cancer, pancreatic cancer, skin cancer, head and neck cancer, skin or intraocular malignant melanoma, cervical cancer, brain stem glioma, pituitary adenocarcinoma, epidermal cancer, cervical squamous cell carcinoma, fallopian tube carcinoma, endometrial carcinoma, vaginal carcinoma, soft tissue sarcoma, urethral cancer, vulvar carcinoma, penile cancer, bladder cancer, kidney or ureter cancer, renal pelvic carcinoma, spinal tumor, neoplasia of the central nervous system (CNS), primary CNS lymphoma, angiogenic tumor, metastatic lesion of the cancers and combinations of the cancers.

The cancer may, for example, be glioblastoma, lung cancer, bladder cancer, oral cancer, head and neck squamous cell cancer, gallbladder cancer or cervical cancer. In particular, in the case of glioblastoma, the antibody or antigen-binding fragment thereof should pass through the blood-brain barrier, which is a barrier formed by tight junction in the capillary endothelial cell membrane of the brain present between the brain and spine, and the surrounding circulatory system. The antibody or antigen-binding fragment thereof may be connected to a transporter in order for it to pass through the BBB. In order to deliver drugs through the BBB, the osmotic pressure of the BBB may be disrupted using a method such as bradykinin or high density focus ultrasound (HIFU). The method may also include the use of glucose and amino acid transporters internal to cells or delivery systems such as receptor-mediated transcytosis of insulin or transferrin, or blocking active efflux transporters of glycoproteins.

Such inflammation is, for example, chronic obstructive pulmonary disease, chronic digestive disorder, conjunctivitis, otitis media, allergic rhinitis, gingivitis, pharyngitis, bronchitis, gastroesophageal reflux disease (GERD), esophagitis, gastritis, enteritis, peptic ulcer, inflammatory bowel disease (IBD), Crohn's disease, irritable bowel syndrome (IBS), enteritis or allergies, urethritis, cystitis, vaginitis, rectal proctitis, eosinophilic gastroenteritis, or rheumatoid arthritis, but is not limited thereto.

The immune disease may be, for example, acquired immunodeficiency, autoimmune disease, systemic lupus erythematosus, scleroderma, Sjogren's syndrome, polymyositis and dermatomyositis, polymyalgia rheumatica, temporal arteritis, polyarteritis nodosa, or Behçet's syndrome.

The pharmaceutically acceptable carrier contained in the composition according to the present invention may include a pharmaceutically acceptable carrier commonly used in preparations, and may include, but is not limited to, lactose, dextrose, sucrose, sorbitol, mannitol, starch, gum acacia, calcium phosphate, alginate, gelatin, calcium silicate, microcrystalline cellulose, polyvinylpyrrolidone, water, syrup, methylcellulose, methyl hydroxybenzoate, propyl hydroxybenzoate, talc, magnesium stearate, mineral oil, and the like. The composition according to the present invention may further contain a lubricant, a wetting agent, a sweetener, a flavoring agent, an emulsifier, a suspension agent, a preservative, or the like, in addition to the ingredients described above.

The pharmaceutical composition according to the present invention may be administered orally or parenterally. The parenteral administration may be intravenous injection, subcutaneous injection, intramuscular injection, intraperitoneal injection, endothelial administration, topical administration, intranasal administration, pulmonary administration, rectal administration, or the like.

Upon oral administration, since proteins or peptides are digested, an oral composition should be coated with an active drug or formulated so as to protect the same from degradation in the stomach. In addition, the pharmaceutical composition may be administered using any device capable of delivering the active substance to target cells.

The suitable dose of the pharmaceutical composition according to the present invention may vary depending on factors such as the formulation method, administration method, and age, body weight, gender, pathological conditions, diet, administration time, administration route, excretion rate, and responsiveness of the patient, and a general physician of ordinary skill can easily determine and prescribe a dose effective for the desired treatment or prevention. For example, the daily dose of the pharmaceutical composition according to the present invention may be within the range of 0.0001 to 100 mg/kg. The term "pharmaceutically effective amount" as used herein may mean an amount sufficient to prevent or treat cancer.

The pharmaceutical composition according to the present invention may be prepared into a unit dose form, or may be incorporated into a multi-dose container through formulation using a pharmaceutically acceptable carrier and/or excipient according to a method that can be easily implemented by those skilled in the art to which the present invention pertains. Here, the formulation may be in the form of a solution, a suspension or an emulsion in an oil or aqueous medium, or may be in the form of an extract, a powder, a suppository, a granule, a tablet, or a capsule. The composition may further contain a dispersant or a stabilizer.

### Combination therapy

In another aspect, the present invention is directed to a composition for combination therapy containing immune cells and a drug other than an anti-FGFR3 antibody.

In an embodiment, the drug other than the anti-FGFR3 antibody may include a chemotherapeutic agent or an antibody other than the anti-FGFR3 antibody.

The drug may, for example, include at least one selected from the group consisting of maytansinoid, auristatin (including MMAE and MMAF), aminopterin, actinomycin, bleomycin, thalidomide, camptothecin, N8-acetylspermidine, 1-(2 chloroethyl)-1,2-dimethyl sulfonyl hydrazide, esperamicin, etoposide, 6-mercaptopurine, dolastatin, trichothecene, calicheamicin, taxol, taxane, paclitaxel, docetaxel, methotrexate, vincristine, vinblastine, doxorubicin, melphalan, mitomycin A, mitomycin C, chlorambucil, duocarmycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, Cytoxan, etoposide, 5-fluorouracil, bis-chloroethylnitrosourea (CNU), irinotecan, camptothecin, bleomycin, idarubicin, daunorubicin, dactinomycin, plicamycin, mitoxantrone, asparaginase, vinorelbine, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, EICAR (5-ethynyl-1-beta-D-ribofuranosylimidazole-4-carboxamide), hydroxyurea, deferoxamine, floxuridine, doxifluridine, raltitrexed, cytarabine(ara C), cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, interferon-a, interferon-γ, tumor necrosis factors, gemcitabine, Velcade, Revlimid, Thalomid, lovastatin, 1-methyl-4-phenylpyridinium ion, staurosporine, actinomycin D, dactinomycin, bleomycin A2, bleomycin B2, peplomycin, epirubicin, pirarubicin, zorubicin, mitoxantrone, verapamil, and thapsigargin, nucleases and toxins derived from bacteria or plants and animals, but the present invention is not limited thereto.

In an embodiment, the antibody other than anti-FGFR3 antibody may be an antibody or an antigen-binding fragment thereof targeting one or more selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptor, c-Met, EGFR, HER2, KDR, PDGFRa, NRP1, and MARCO.

In another aspect, the present invention is directed to a composition for combination therapy including the antibody or antigen-binding fragment thereof and at least one selected from the group consisting of:
(i) an immune cell;
(ii) an immune cell containing a chimeric antigen receptor (CAR) including a scFv fragment of an antibody other than the anti-FGFR3 antibody as an extracellular domain; and
(iii) an immune checkpoint inhibitor.

In accordance with another aspect of the present invention, provided is a composition for combination therapy including the immune cell-engaging bispecific or multispecific antibody and at least one selected from the group consisting of:
(i) an immune cell;
(ii) an immune cell containing a chimeric antigen receptor (CAR) including a scFv fragment of an antibody other than the anti-FGFR3 antibody as an extracellular domain; and
(iii) an immune checkpoint inhibitor.

The immune cells are capable of inducing immunity to induce a desired cancer treatment effect and may, for example, include, but are not limited to, one or more selected from the group consisting of T cells, NK cells, cytokine induced killer cells (CIK), activated cytotoxic T lymphocytes (CTL), macrophages, tumor-infiltrating lymphocytes (TIL) and dendritic cells.

The antibody other than anti-FGFR3 is an antibody targeting a subject other than FGFR3 and may be an antibody or antigen-binding fragment thereof binding to PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, a transferrin receptor, c-Met, EGFR, HER2, KDR, PDGFRa, NRP1, MARCO, or MARCO, but is not limited thereto.

In another aspect, the present invention is directed to a composition for combination therapy containing the antibody or antigen-binding fragment thereof and at least one selected from the group consisting of:
(i) an immune cell;
(ii) an immune cell containing a chimeric antigen receptor (CAR) including a scFv fragment of an antibody other than the anti-FGFR3 antibody as an extracellular domain; and
(iii) an immune checkpoint inhibitor.

In accordance with another aspect of the present invention, provided is a composition for combination therapy including the immune cell-engaging bispecific or multispecific antibody and at least one selected from the group consisting of:
(i) an immune cell;
(ii) an immune cell containing a chimeric antigen receptor (CAR) including a scFv fragment of an antibody other than the anti-FGFR3 antibody as an extracellular domain; and
(iii) an immune checkpoint inhibitor.

The immune cells are capable of inducing immunity to induce a desired cancer treatment effect and may, for example, include, but are not limited to, one or more selected from the group consisting of T cells, NK cells, cytokine induced killer cells (CIK), activated cytotoxic T lymphocytes (CTL), macrophages, tumor-infiltrating lymphocytes (TIL) and dendritic cells.

The antibody other than the anti-FGFR3 antibody is an antibody targeting a subject other than FGFR3 and may be an antibody or antigen-binding fragment thereof binding to PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptors, c-Met, EGFR, HER2, KDR, PDGFRa, NRP1, MARCO, or MARCO, but is not limited thereto.

The immune checkpoint inhibitor refers to an agent capable of inducing T cell activation by blocking a T cell inhibitory signal in a site where antigen presenting cells (APCs) react with immune cells, for example, T cells. The immune checkpoint inhibitor may, for example, be a drug targeting PD-1, PD-L1, BTLA, CTLA-4, VISTA, LAG3, TIM3, CD137 (4-1BB), CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, or CD200R, but is not limited thereto.

A first component and a second component for administration in combination may be administered simultaneously. In addition, the first component and the second component administered in combination may be separately administered at a predetermined time interval. The second component may be separately administered before or after administration of the first component to the subject for the combination administration.

### Companion diagnostics (CDx)

Companion diagnosis is a molecular diagnostic technique to predict the response of the patient to a specific drug treatment. In the application of drugs, the selection criteria for patients to be applied are provided and criteria can be provided to allow treatment of drugs suitable for the patient in consideration of specificity and sensitivity of diagnostic, prognostic and predictive biomarkers suitable for the criteria.

Anticancer drugs are highly effective in patients who respond thereto, but the proportion of patients who respond thereto is low. For this reason, it is important to select patients for whom the anticancer drugs will be effective. Through companion diagnosis, it is possible to select patients who respond to anticancer drugs and increase the therapeutic efficiency of patients.

In another aspect, the present invention is directed to a composition for treating cancer determined that a fusion gene of FGFR3 and TACC3 is amplified, the fusion gene has mutation, the fusion gene is overexpressed, or the fusion gene is constitutively activated, containing the antibody or antigen-binding fragment thereof, the bispecific or multispecific antibody including the antibody or antigen-binding fragment thereof, the antibody-drug conjugate including the antibody or antigen-binding fragment thereof, the chimeric antigen receptor including the antibody or antigen-binding fragment thereof, or immune cellsincluding the chimeric antigen receptor.

In another aspect, the present invention is directed to a method of treating cancer including determining, in a patient-derived sample, whether or not a fusion gene of FGFR3 and TACC3 is amplified, the fusion gene has mutation, the fusion gene is overexpressed, or the fusion gene is constitutively activated, and administering, to the patient, the antibody or antigen-binding fragment thereof, the bispecific or multispecific antibody including the antibody or antigen-binding fragment thereof, the antibody-drug conjugate including the antibody or antigen-binding fragment thereof, the chimeric antigen receptor including the antibody or antigen-binding fragment thereof, or immune cellsincluding the chimeric antigen receptor, when it is determined that the fusion gene of FGFR3 and TACC3 is amplified, the fusion gene has mutation, the fusion gene is overexpressed, or the fusion gene is constitutively activated.

The sample refers to a biological subject obtained from a tissue or bodily fluid of a patient. The source of the tissue sample may be solid tissue such as tissue derived from a frozen and/or preserved organ, tissue sample, biopsy, or aspirate; blood or any blood ingredient (e.g., serum, plasma); bone marrow or any bone marrow ingredient; or a bodily fluid such as urine, cerebrospinal fluid, whole blood, plasma or serum. The sample may include a non-cellular fraction (e.g., urine, plasma, serum or other non-cellular body fluid). The sample may include a bodily fluid such as blood (e.g., whole blood). Specifically, the sample may be a whole blood sample, a whole bone marrow sample, a whole peripheral blood sample, or a whole tumor sample obtained from a patient. The "whole" sample refers to a sample that is substantially free of ingredients (e.g., cells) that have been removed or separated from the sample. The sample, e.g., a blood sample, may be diluted (e.g., in a physiologically compatible buffer or medium) prior to use. In other embodiments, the "whole" sample such as a whole tissue sample or a whole tumor sample may substantially retain the microenvironment from the tissue of origin, e.g., substantially retain the structure of a tumor or immune microenvironment. Specifically, a sample, such as a tumor sample, may be processed (e.g., ground, minced, blended, ground, or the like) into smaller pieces and diluted (e.g., in a physiologically compatible buffer or medium).

Identification of the expression of the gene is a process of determining the presence and determining expression level of the gene and may be performed by polymerase chain reaction (PCR). In some cases, this may be performed by transcriptional polymerase reaction (RT-PCR), competitive RT-PCR, real-time RT-PCR, RNase protection assay (RPA), northern blotting, or DNA chip-based methods.

Polymerase chain reaction (PCR) may be performed through a gene amplification reaction using primers. Multiplex PCR, real-time PCR, differential display PCR (DD-PCR), rapid amplification of cDNA ends (RACE), inverse polymerase chain reaction chain reaction (IPCR), vectorette PCR, TAIL-PCR (thermal asymmetric interlaced PCR) and the like may be performed.

Whether or not a protein is expressed by genetic coding may be detected. This is a process of determining the presence and expression level of the protein expressed from the gene. The amount of protein may be determined using an antibody, oligopeptide, ligand, PNA (peptide nucleic acid) or aptamer that specifically binds to the protein.

Analysis methods for this include Western blot, enzyme linked immunosorbent assay (ELISA), radioimmunoassay (RIA), radio immunodiffusion, Ouchterlony immunodiffusion, rocket immunoelectrophoresis, tissue immunostaining, immunoprecipitation assay, complement fixation assay, fluorescence activated cell sorter (FACS), a protein chip-based method and the like.

### Example

Hereinafter, the present invention will be described in more detail with reference to examples. However, it is obvious to those skilled in the art that these examples are provided only for illustration of the present invention and should not be construed as limiting the scope of the present invention.

### Example 1: Selection of phage antibodies specific to FGFR3, cell panning (phage display technology)

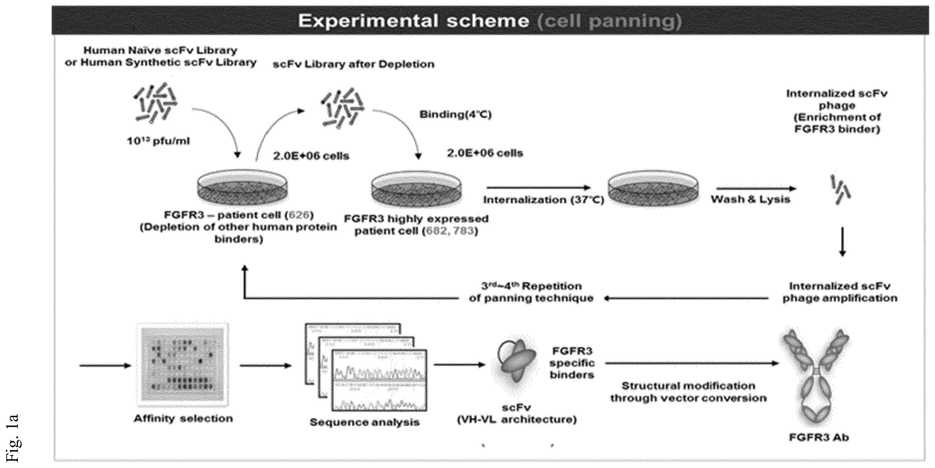

The present inventors performed cell panning to select antibodies with cell internalization function using a synthetic scFv phage library *{(1)*Bai, Xuelian & Kim, Jihye & Kang, Seungmin & Kim, Wankyu & Shim, Hyunbo. (2015). A Novel Human scFv Library with Non-Combinatorial Synthetic CDR Diversity. PloS one. 10. e0141045. 10.1371/journal.pone.0141045*., (2)* Yang, Hye & Kang, Kyung & TCW, Julia & Shim, Hyunbo. (2009). Construction of a large synthetic human scFv library with six diversified CDRs and high functional diversity. Molecules and cells. 27. 225-35. 10.1007/s10059-009-0028-9*.}.* FGFR3 over-expressing cells (682T) and FGFR3 low-expressing cells (626T) were verified by RPKM analysis values and protein expression confirmation through western blotting and the cells were selected. The FGFR3 low-expressing cells were treated with the phage library stock collected in advance and reacted at 4°C for 1 hour, only the supernatant was recovered through a centrifuge, and the FGFR3-overexpressing cells prepared in advance were treated with the supernatant and reacted at 4°C for 1 hour. Then, non-specific phage was removed through the culture medium and incubated at 37°C for 30 minutes, and the antibody internalized into cells was recovered. The recovery was performed by washing with low pH buffer to remove the non-specific cell surface-bound phage and then lysing the cells with lysis buffer to recover antibodies with cell internalization function through FGFR3-specific binding inside the cells. The infected *E. coli* was amplified again, was subjected to the same cell panning twice to perform cell panning a total of three times, and then was subjected to ELISA screening to select FGFR3-specific antibodies. The schematic diagram of the experiment is shown in [FIG. 1A].

### Example 2: Selection of phage antibodies specific to FGFR3, immobilized antigen panning (phage display technology)

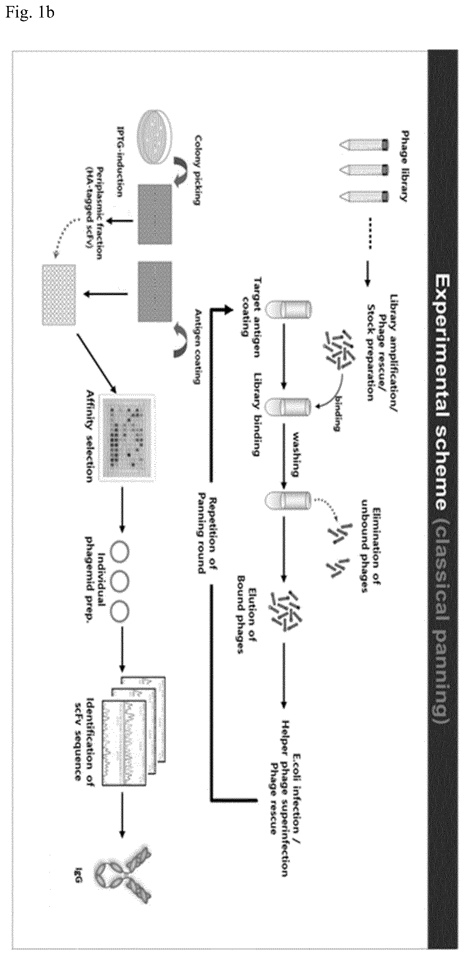

Immobilized antigen panning widely used in general was performed using the same phage library. An immune tube was coated with FGFR3-IIIc/Fc antigen and negative/Fc antigen at 3 pg/ml (overnight, 4°C), and the previously recovered phage library stock was first reacted with pre-blocked negative/Fc antigen at room temperature for 1 hour. Then, the supernatant was recovered and reacted at room temperature for 1 hour in a pre-blocked FGFR3-IIIc/Fc tube. Then, the result was washed with PBST to remove non-specific phages and phages specific to the FGFR3-lllc/Fc antigen were recovered using low pH buffer. E. coli was infected with the phages, amplified and subjected to the same panning three times to perform biopanning a total of four times, and then subjected to ELISA screening to select FGFR3-specific antibodies. A schematic diagram of the experiment is shown in [FIG. 1B].

### Example 3: Selection of phage antibodies specific for FGFR3, magnetic bead panning; Semi-automated biopanning (phage display technology)

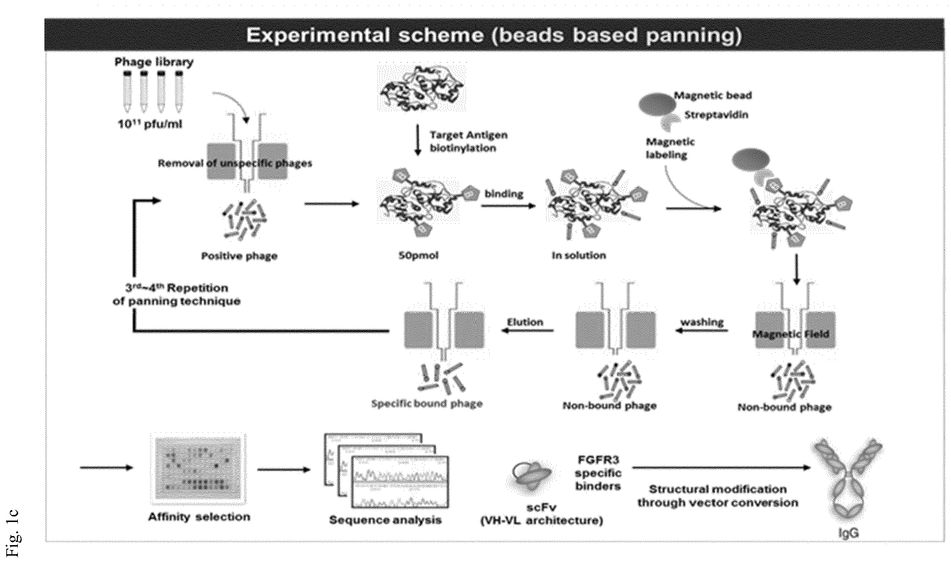

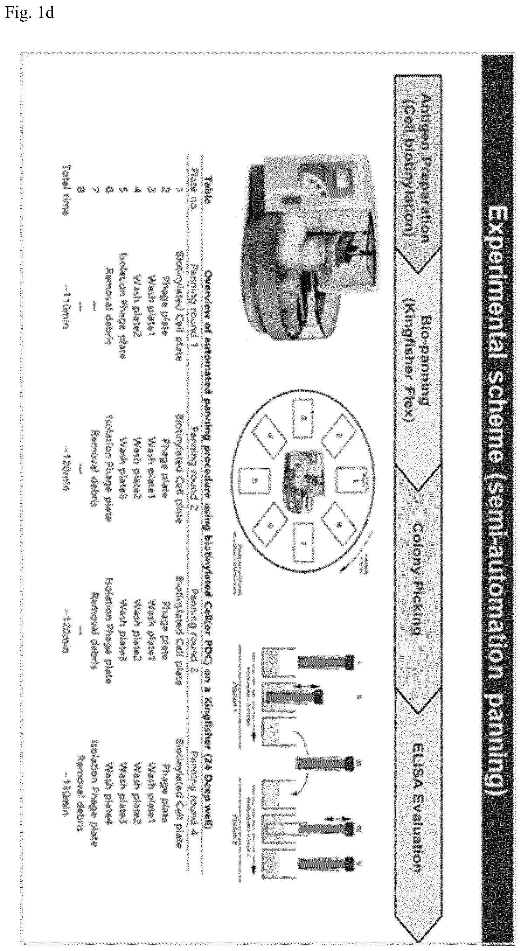

Semi-automated biopanning was performed using the same phage library. This method is magnetic bead-based panning, which is a conventionally used manual method that includes treating biotinylated target antigens with phages, binding, washing with columns or magnetic chambers and recovering antigen-specific phages [FIG. 1C]. The present inventor introducing the series of processes into a semi-automated system. Reagents such as phage library stock, biotinylated FGFR3-IIIc/Fc antigen, streptavidin-M280 Dynabeads, 0.1% PBST wash buffer, and elution buffer were seeded into a Kingfisher Flex 24 deep well in a Kingfisher Flex (Thermo scientific) device and biopanning was performed through the pre-produced software protocol (BindIt^{™} Software) [FIG. 1D]. Biopanning was repeated 3 to 4 times to amplify phages specifically binding to the FGFR3 antigen and recover the same. ELISA screening was performed using this to select FGFR3-specific antibodies.

### Example 4: Affinity ELISA-based antibody screening

The product of each biopanning method is an *E. coli* host cell (TG1), which was seeded on agar LB/ampicillin medium as a single clone, optionally inoculated into SB/ampicillin culture medium and cultured (3 hours, 37°C). Then, each well was treated with 1 mM IPTG at a final concentration for induction of scFv-pIII protein and cultured at 30°C overnight. The next day, the supernatant was removed from the cultured 96-well plate using a centrifuge, and only *E. coli* was collected, treated with 1xTES solution (20% w/v sucrose, 50 mM Tris, 1 mM EDTA, pH 8.0), suspended at 4°C for 1 hour or more, treated with 0.2xTES solution, suspended for 2 hours or more, and the scFv-pIII protein present in the periplasmic region of E. *coli* was extracted. A 96-well plate was coated with FGFR3 recombinant protein and negative recombinant protein at 1 µg/ml overnight at 4°C the day before and was reacted with the extracted scFv-pIII at room temperature for 1 hour. After the reaction, the product was washed with PBST, treated with the HRP-conjugated anti-HA antibody for 1 hour and washed again to induce a color reaction (TMB substrate) and the O.D. value was measured at a wavelength of 450 nm. The O.D ratio of FGFR3 recombinant protein/negative recombinant protein was calculated to select FGFR3-specific scFv-pIII clones. The selection was made on clones having a relative ratio (positive/negative) of 2 or more and antibody sequence analysis was performed thereon.

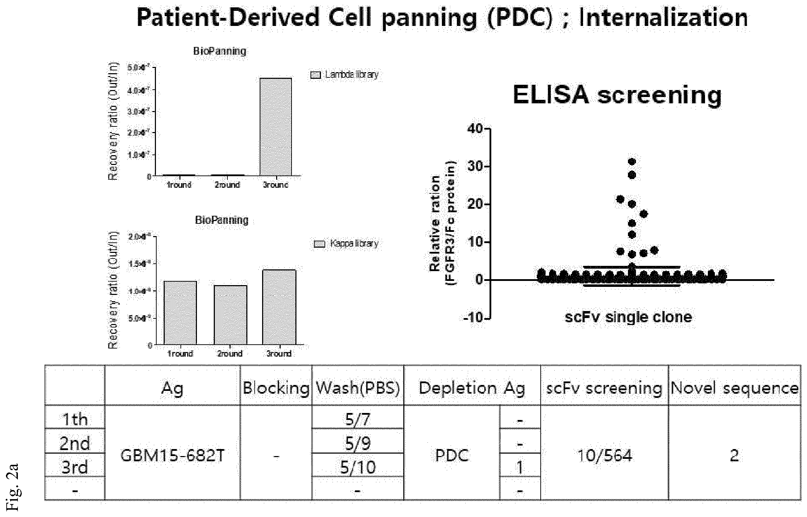

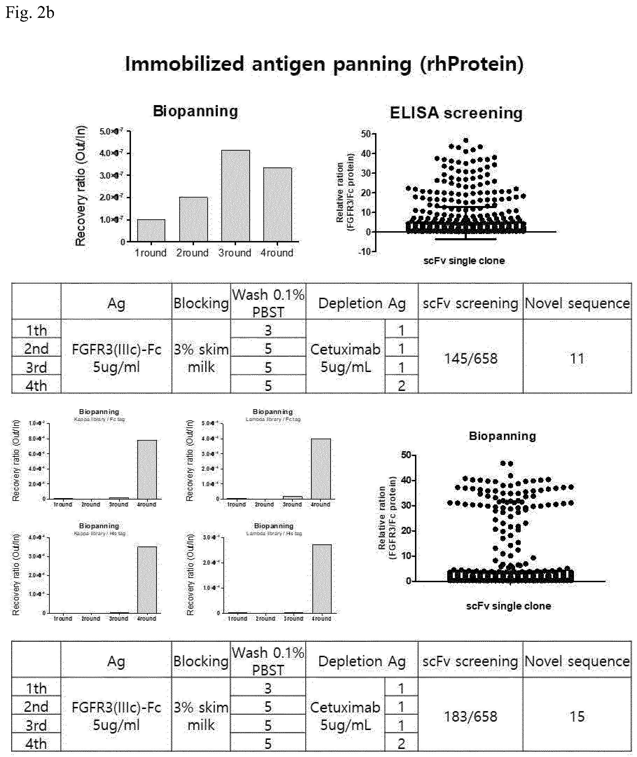

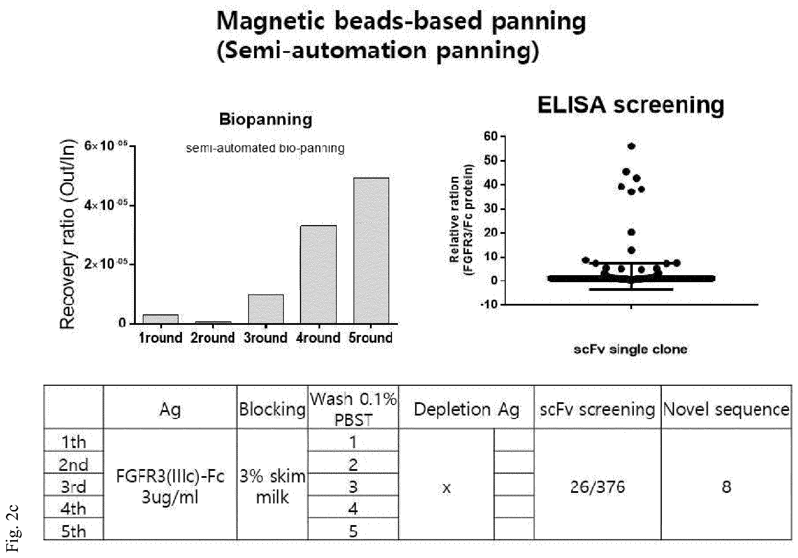

In biopanning using patient-derived cells, ELISA screening was performed on 564 single clones of the completed output phages. Thereamong, 10 positive clones were identified [FIG. 2A]. In immobilized antigen panning, ELISA screening was performed on 658 single clones each in duplicate independent experiments (1,316 in total) and thereamong, 328 positive clones were identified [FIG. 2B]. After completion of the magnetic-based semi-automated biopanning, ELISA screening was performed on 376 single clones and 26 positive clones were identified [FIG. 2C].

### Example 5: Antibody Sequencing

Antibody sequences of clones found to have an O.D. ratio (FGFR3 antigen/negative antigen) of 2 or more in affinity ELISA screening analysis were analyzed. After culturing and growing *E. coli* containing the phagemid vector of the selected clone in a 14 ml round tube in LB/ampicillin medium, DAN prep (mini-prep) was performed to extract plasmid DNA. The extracted DNA was analyzed using a primer matching the scFv sequence of the phagemid vector.

As a result of sequence analysis according to each method, 2 antibody-specific sequences were analyzed for cell panning, 26 antibody-specific sequences were analyzed for immobilized antigen panning, and 8 antibody-specific sequences were analyzed for semi-automated panning. Based on this, repeated ELISA in scFv form was performed on respective corresponding clones. As a result, 25 clones having FGFR3-specific binding ability were finally identified and the sequences for the heavy chain and light chain were analyzed.

**[Table 1]**

| SEQ IN No. | VH CDR1 | SEQ IN No. | VH CDR2 | SEQ IN No. | VH CDR3 |
|---|---|---|---|---|---|
| 1 | SYYMS | 19 | AISPGSSNKYYADS VKG | 42 | DWSKFDY |
| 2 | DYYMS | 20 | AIYHGDGSTYYADS VKG | 43 | DSRLCQTSGCYSDDA MDV |
| 3 | GYAMS | 21 | AISHGGSSKYYADS VKG | 44 | SRLESPFLAQSYAYA MDV |
| 4 | NYYMS | 22 | GISPGSGSIYYADS VKG | 45 | DWTRFDY |
| 5 | SYDMS | 23 | GIYSGDGSIYYADS VKG | 46 | DAPTNEMSRFDY |
| 4 | NYYMS | 24 | GIYYGSGNIYYADS VKG | 47 | GGGGFDY |
| 6 | DYDMS | 25 | AISSSDGSTYYADS VKG | 48 | HLPNFDY |
| 7 | NYAIH | 26 | SIIPISGTANYAQK FQG | 49 | GGYGFDY |
| 8 | NYWIG | 27 | IIHPGGSDSRYSPS FQG | 50 | WRAWFDS |
| 9 | DIAMH | 28 | TIYSSDSTYYNPSL KG | 51 | GVGYTTSSRFDY |
| 10 | SYAIS | 29 | SIYYSGSTYYNPSL KS | 52 | GASWMDM |
| 11 | IYGMH | 30 | SIYYTGDTYSNPSL KS | 53 | GYATALDY |
| 11 | IYGMH | 31 | SIYYSGDTYYNPSL ES | 54 | DIGSSWGFDY |
| 12 | NYWMT | 32 | WINTYSGNTNYNPS LKT | 55 | GRGMFDY |
| 3 | GYAMS | 33 | GINWRGATTAYADS LKG | 56 | SDYGLFDI |
| 13 | NYPMS | 34 | SVSGSGTSTYYADS VKG | 57 | HTQRWGAFDP |
| 14 | SYAMS | 35 | GVSYSGNNKYYADS LQS | 58 | NRNAIFDY |
| 1 | SYYMS | 36 | GIYPDDGSIYYADS VKG | 59 | DPLIYELPIGYSSYG MDV |
| 6 | DYDMS | 23 | GIYSGDGSIYYADS VKG | 60 | GPNSHIYPYFYY |
| 15 | GYDMS | 37 | VISPSGGNIYYADS VKG | 61 | GILFMTITLPYSSDG MDV |
| 16 | NYDMS | 38 | VIFHDSGNIYYADS VKG | 62 | HPLLSIFSLPYYSDA MDV |
| 17 | GYYMS | 39 | AISSGGGNTYYADS VKG | 63 | KWPTFDY |
| 4 | NYYMS | 19 | AISPGSSNKYYADS VKG | 64 | DWEQFDY |
| 18 | NYAMS | 40 | GISSDNSSKYYADS VKG | 65 | HTVIEALEAAYYDDG MDV |
| 16 | NYDMS | 41 | VIYHNGSSKYYADS VKG | 66 | HGGDETNYFQSYYDG MDV |

**[Table 2]**

| Clone | SEQ IN No. | VH |
|---|---|---|
| F303 | 133 | EVQLLESGGGLVQPGGSLRLSCAASGFTFSSYYMSWVRQAPGKG LEWVSAISPGSSNKYYADSVKGRFTISRDNSKNTLYLQMNSLRA EDTAVYYCAKDWSKFDYWGQGTLVTVSS |
| F304 | 134 | EVQLLESGGGLVQPGGSLRLSCAASGFTFSDYYMSWVRLAPGKG LEWVSAIYHGDGSTYYADSVKGRFTISRDNSKNTLYLQMNSLRA EDTAVYYCARDSRLCQTSGCYSDDAMDVWGQGTLVTVSS |
| F305 | 135 | EVQLLESGGGLVQPGGSLRLSCAASGFTFSGYAMSWVRQAPGKG LEWVSAISHGGSSKYYADSVKGRFTISRDNSKNTLYLQMNSLRA EDTAVYYCARSRLESPFLAQSYAYAMDVWGQGTLVTVSS |
| F308 | 136 | EVQLLESGGGLVQPGGSLRLSCAASGFTFSNYYMSWVRQAPGKG LEWVSGISPGSGSIYYADSVKGRFTISRDNSKNTLYLQMNSLRA EDTAVYYCAKDWTRFDYWGQGTLVTVSS |
| F309 | 137 | EVQLLESGGGLVQPGGSLRLSCAASGFTFSSYDMSWVRQAPGKG LEWVSGIYSGDGSIYYADSVKGRFTISRDNSKNTLYLQMNSLRA EDTAVYYCAKDAPTNEMSRFDYWGQGTLVTVSS |
| F310 | 138 | EVQLLESGGGLVQPGGSLRLSCAASGFTFSNYYMSWVRQAPGKG LEWVSGIYYGSGNIYYADSVKGRFTISRDNSKNTLYLQMNSLRA EDTAVYYCARGGGGFDYWGQGTLVTVSS |
| F311 | 139 | EVQLLESGGGLVQPGGSLRLSCAASGFTFSDYDMSWVRQAPGKG LEWVSAISSSDGSTYYADSVKGRFTISRDNSKNTLYLQMNSLRA EDTAVYYCAKHLPNFDYWGQGTLVTVSS |
| FS301 | 140 | EVQLLESGGGLVQPGGSLRLSCAASGFTFSNYAIHWVRQAPGKG LEWVSSIIPISGTANYAQKFQGRFTISRDNSKNTLYLQMNSLRA EDTAVYYCSRGGYGFDYWGQGTLVTVSS |
| FS303 | 141 | EVQLLESGGGLVQPGGSLRLSCAASGFTFSNYWIGWVRQAPGKG LEWVSIIHPGGSDSRYSPSFQGRFTISRDNSKNTLYLQMNSLRA EDTAVYYCARWRAWFDSWGQGTLVTVSS |
| FS306 | 142 | EVQLLESGGGLVQPGGSLRLSCAASRFTFSDIAMHWVRQAPGKG LEWVSTIYSSDSTYYNPSLKGRFTISRDNSKNTLYLQMNSLRAE DTAVYYCAKGVGYTTSSRFDYWGQGTLVTVSS |
| FS307 | 143 | EVQLLESGGGLVQPGGSLRLSCAASGFTFSSYAISWVRQAPGKG LEWVSSIYYSGSTYYNPSLKSRFTISRDNSKNTLYLQMNSLRAE DTAVYYCAQGASWMDMWGQGTLVTVSS |
| FS309 | 144 | EVQLLESGGGLVQPGGSLRLSCAASGFTFSIYGMHWVRQAPGKG LEWVSSIYYTGDTYSNPSLKSRFTISRDNSKNTLYLQMNSLRAE DTAVYYCARGYATALDYWGQGTLVTVSS |
| FS310 | 145 | EVQLLESGGGLVQPGGSLRLSCAASGFTFSIYGMHWVRQAPGKG LEWVSSIYYSGDTYYNPSLESRFTISRDNSKNTLYLQMNSLRAE DTAVYYCARDIGSSWGFDYWGQGTLVTVSS |
| FS312 | 146 | EVQLLESGGGLVQPGGSLRLSCAASGFTFSNYWMTWVRQAPGKG LEWVSWINTYSGNTNYNPSLKTRFTISRDNSKNTLYLQMNSLRA EDTAVYYCARGRGMFDYWGQGTLVTVSS |
| FS313 | 147 | EVQLLESGGGLVQPGGSLRLSCAASGFTFSGYAMSWVRQAPGKG LEWVSGINWRGATTAYADSLKGRFTISRDNSKNTLYLQMNSLRA EDTAVYYCARSDYGLFDIWGQGTLVTVSS |
| FS314 | 148 | EVQLLESGGGLVQPGGSLRLSCAASGFTFSNYPMSWVRQAPGKG LEWVSSVSGSGTSTYYADSVKGRFTISRDNSKNTLHLQMNSLRA EDTAVYYCARHTQRWGAFDPWGQGTLVTVSS |
| FS316 | 149 | EVQLLESGGGLVQPGGSLRLSCAASGFTFSSYAMSWVRQAPGKG LEWVSGVSYSGNNKYYADSLQSRFTISRDNSKNTLYLQMNSLRA EDTAVYYCARNRNAIFDYWGQGTLVTVSS |
| FA001 | 150 | EVQLLESGGGLVQPGGSLRLSCAASGFTFSSYYMSWVRQAPGKG LEWVSGIYPDDGSIYYADSVKGRFTISRDNSKNTLYLQMNSLRA EDTAVYYCAKDPLIYELPIGYSSYGMDVWGQGTLVTVSS |
| FA002 | 151 | EVQLLESGGGLVQPGGSLRLSCAASGFTFSDYDMSWVRQAPGKG LEWVSGIYSGDGSIYYADSVKGRFTISRDNSKNTLYLQMNSLRA EDTAVYYCAKGPNSHIYPYFYYWGQGTLVTVSS |
| FA003 | 152 | EVQLLESGGGLVQPGGSLRLSCAASGFTFSGYDMSWVRQAPGKG LEWVSVISPSGGNIYYADSVKGRFTISRDNSKNTLYLQMNSLRA EDTAVYYCARGILFMTITLPYSSDGMDVWGQGTLVTVSS |
| FA004 | 153 | EVQLLESGGGLVQPGGSLRLSCAASGFTFSNYDMSWVRQAPGKG LEWVSVIFHDSGNIYYADSVKGRFTISRDNSKNTLYLQMNSLRA EDTAVYYCAKHPLLSIFSLPYYSDAMDVWGQGTLVTVSS |
| FA005 | 154 | EVQLLESGGGLVQPGGSLRLSCAASGFTFSGYYMSWVRQAPGKG LEWVSAISSGGGNTYYADSVKGRFTISRDNSKNTLYLQMNSLRA EDTAVYYCARKWPTFDYWGQGTLVTVSS |
| FA006 | 155 | EVQLLESGGGLVQPGGSLRLSCAASGFTFSNYYMSWVRQAPGKG LEWVSAISPGSSNKYYADSVKGRFTISRDNSKNTLYLQMNSLRA EDTAVYYCAKDWEQFDYWGQGTLVTVSS |
| FA007 | 156 | EVQLLESGGGLVQPGGSLRLSCAASGFTFSNYAMSWVRQAPGKG LEWVSGISSDNSSKYYADSVKGRFTISRDNSKNTLYLQMNSLRA EDTAVYYCAKHTVIEALEAAYYDDGMDVWGQGTLVTVSS |
| FA008 | 157 | EVQLLESGGGLVQPGGSLRLSCAASGFTFSNYDMSWVRQAPGKG LEWVSVIYHNGSSKYYADSVKGRFTISRDNSKNTLYLQMNSLRA EDTAVYYCAKHGGDETNYFQSYYDGMDVWGQGTLVTVSS |

**[Table 3]**

| Clon e | SEQ IN No. | VL CDR1 | SEQ IN No. | VL CDR2 | SEQ IN No. | VL CDR3 |
|---|---|---|---|---|---|---|
| F303 | 67 | SGSSSNIGSNDV T | 89 | DDNHRP S | 110 | GTWDASLSAY V |
| F304 | 68 | TGSSSNIGNNYV S | 90 | YNSHRP S | 111 | ATWDDSLSGY V |
| F305 | 69 | SGSSSNIGNNDV S | 91 | SNSHRP S | 112 | GTWDDSLNGY V |
| F308 | 70 | TGSSSNIGNNAV S | 92 | DDSKRP S | 113 | GAWDYSLSAY V |
| F309 | 71 | SGSSSNIGNNYV S | 93 | ADSKRP S | 114 | ASWDYSLSGY V |
| F310 | 72 | SGSSSNIGNNDV Y | 94 | ADNHRP S | 115 | GAWDASLNGY V |
| F311 | 73 | TGSSSNIGNNTV S | 95 | YDSHRP S | 112 | GTWDDSLNGY V |
| FS30 1 | 74 | RASQSISTFLN | 96 | DATNRA T | 116 | QQANSFPLT |
| FS30 3 | 75 | RASQSITDYLA | 97 | DASNRA T | 117 | QQSYSTPFT |
| FS30 6 | 76 | RASQSISSYLN | 98 | DASNRA A | 118 | CQQYSSFPLT |
| FS30 7 | 77 | QASQDISNYLN | 97 | DASNRA T | 119 | QQSYSTPIT |
| FS30 9 | 78 | SGSSSNIGSNTV N | 99 | DTSNKH S | 120 | AAWDNSLNGQ L |
| FS31 0 | 79 | TAAASDIASNYV Q | 100 | EDTKRP S | 121 | ATWDDSLRGW V |
| FS31 2 | 80 | SDNIGSKSVH | 101 | SNNQRP S | 122 | QSYDSSTVV |
| FS31 3 | 81 | GGNNIGSKSVH | 102 | QDTKRP S | 123 | QSYDSRQLV |
| FS31 4 | 82 | GGDNIGTKSVH | 103 | GNSNRP S | 124 | QSYDSSLSGY V |
| FS31 6 | 83 | SRNNIGSKSMH | 104 | DNNKRP S | 125 | SSYTSSSTLV |
| FA00 1 | 84 | SGSSSNIGSNDV N | 105 | YDSNRP S | 126 | AAWDSSLSGY V |
| FA00 2 | 69 | SGSSSNIGNNDV S | 91 | SNSHRP S | 127 | GTWDYSLSGY V |
| FA00 3 | 85 | SGSSSNIGSNAV T | 89 | DDNHRP S | 128 | GAWDDSLSAY V |
| FA00 4 | 86 | TGSSSNIGNNDV S | 106 | SDNHRP S | 129 | GAWDSSLNAY V |
| FA00 5 | 87 | TGSSSNIGSNTV Y | 107 | ANNQRP S | 130 | GTWDDSLSGY V |
| FA00 6 | 88 | SGSSSNIGSNTF N | 108 | SNSQRP S | 131 | GAWDSSLSAY V |
| FA00 7 | 85 | SGSSSNIGSNAV T | 89 | DDNHRP S | 128 | GAWDDSLSAY V |
| FA00 8 | 72 | SGSSSNIGNNDV Y | 109 | SDSNRP S | 132 | GTWDYSLNGY V |

**[Table 4]**

| Clon e | SEQ IN No. | VL |
|---|---|---|
| F303 | 158 | QSVLTQPPSASGTPGQRVTISCSGSSSNIGSNDVTWYQQLPGTAPKLL IYDDNHRPSGVPDRFSGSKSGTSASLAISGLRSEDEADYYCGTWDASL SAYVFGGGTKLTVL |
| F304 | 159 | QSVLTQPPSASGTPGQRVTISCTGSSSNIGNNYVSWYQQLPGTAPKLL IYYNSHRPSGVPDRFSGSKSGTSASLAISGLRSEDEADYYCATWDDSL SGYVFGGGTKLTVL |
| F305 | 160 | QSVLTQPPSASGTPGQRVTISCTGSSSNIGNNYVSWYQQLPGTAPKLL IYYNSHRPSGVPDRFSGSKSGTSASLAISGLRSEDEADYYCATWDDSL SGYVFGGGTKLTVL |
| F308 | 161 | QSVLTQPPSASGTPGQRVTISCTGSSSNIGNNAVSWYQQLPGTAPKLL IYDDSKRPSGVPDRFSGSKSGTSASLAISGLRSEDEADYYCGAWDYSL SAYVFGGGTKLTVL |
| F309 | 162 | QSVLTQPPSASGTPGQRVTISCSGSSSNIGNNYVSWYQQLPGTAPKLL IYADSKRPSGVPDRFSGSKSGTSASLAISGLRSEDEADYYCASWDYSL SGYVFGGGTKLTVL |
| F310 | 163 | QSVLTQPPSASGTPGQRVTISCSGSSSNIGNNDVYWYQQLPGTAPKLL IYADNHRPSGVPDRFSGSKSGTSASLAISGLRSEDEADYYCGAWDASL NGYVFGGGTKLTVL |
| F311 | 164 | QSVLTQLPSASGTPGQRVTISCTGSSSNIGNNTVSWYQQLPGTAPKLL IYYDSHRPSGVPDRFSGSKSGTSASLAISGLRSEDEADYYCGTWDDSL NGYVFGGGTKLTVL |
| FS30 1 | 165 | EIVLTQSPGTLSLSPGERATLSCRASQSISTFLNWYQQKPGQAPRLLI YDATNRATGIPDRFSGSGSGTDFTLTISRLEPEDFAVYYCQQANSFPL TFGQGTKVEIK |
| FS30 3 | 166 | EIVLTQSPGTLSLSPGERATLSCRASQSITDYLAWYQQKPGQAPRLLI YDASNRATGIPDRFSGSGSGTDFTLTISRLEPEDFAVYYCQQSYSTPF TFGQGTKVEIK |
| FS30 6 | 167 | EIVLTQGPGTLSLSPGERATLSCRASQSISSYLNWYQQKPGQAPRLLV YDASNRAAGIPDRFSGSGSGTDFTLTISRLEPEDFAVYYCQQYSSFPL TFGQGTKVEIK |
| FS30 7 | 168 | EIVLTQSPGTLSLSPGERATLSCQASQDISNYLNWYQQKPGQAPRLLI YDASNRATGIPDRFSGSGSGTDFTLTISRLEPEDFAVYYCQQSYSTPI TFGQGTKVEIK |
| FS30 9 | 169 | QSVLTQPPSASGTPGQRVTISCSGSSSNIGSNTVNWYQQLPGTAPKLL IYDTSNKHSGDRFSGSKSGTSASLAISGLRSEDEADYYCAAWDNSLNG QLFGGGTKLTVL |
| FS31 0 | 170 | QSVLTQPPSASGTPGQRVTISCTAAASDIASNYVQWYQQLPGTAPKLL IYEDTKRPSGVPYRFSGSKSGTSASLAISGLRSEDEADYYCATWDDSL RGWVFGGGTKLTVL |
| FS31 2 | 171 | QSVLTQPPSASGTPGQRVTISCSDNIGSKSVHWYQQLPGTAPKLLIYS NNQRPSGVSDRFSGSKSGTSASLAISGLRSEDEADYYCQSYDSSTVVF GGGTKLTVL |
| FS31 3 | 172 | QSVLTQPPSASGAPGQRVTISCGGNNIGSKSVHWYQQLPGTAPKLLIY QDTKRPSGVPNRFSGSKSGTSASLAISGLRSEDEADYYCQSYDSRQLV FGGGTKLTVL |
| FS31 4 | 173 | QSVLTQPPSASGTPGQRVTISCGGDNIGTKSVHWYQQLPGTAPKLLIY GNSNRPSGVPNRFSGSKSGTSASLAISGLRSEDEADYYCQSYDSSLSG YVFGGGTKLTVL |
| FS31 6 | 174 | QSVLTQPPSASATPGQRVTISCSRNNIGSKSMHWYQQLPGTAPKLLIY DNNKRPSGVSDRFSGSKSGTSASLAISGLRSEDEADYYCSSYTSSSTL VFGGGTKLTVL |
| FA00 1 | 175 | QSVLTQPPSASGTPGQRVTISCSGSSSNIGSNDVNWYQQLPGTAPKLL IYYDSNRPSGVPDRFSGSKSGTSASLAISGLRSEDEADYYCAAWDSSL SGYVFGGGTKLTVL |
| FA00 2 | 176 | QSVLTQPPSASGTPGQRVTISCSGSSSNIGNNDVSWYQQLPGTAPKLL IYSNSHRPSGVPDRFSGSKSGTSASLAISGLRSEDEADYYCGTWDYSL SGYVFGGGTKLTVL |
| FA00 3 | 177 | QSVLTQPPSASGTPGQRVTISCSGSSSNIGSNAVTWYQQLPGTAPKLL IYDDNHRPSGVPDRFSGSKSGTSASLAISGLRSEDEADYYCGAWDDSL SAYVFGGGTKLTVL |
| FA00 4 | 178 | QSVLTQPPSASGTPGQRVTISCTGSSSNIGNNDVSWYQQLPGTAPKLL IYSDNHRPSGVPDRFSGSKSGTSASLAISGLRSEDEADYYCGAWDSSL NAYVFGGGTKLTVL |
| FA00 5 | 179 | QSVLTQPPSASGTPGQRVTISCTGSSSNIGSNTVYWYQQLPGTAPKLL IYANNQRPSGVPDRFSGSKSGTSASLAISGLRSEDEADYYCGTWDDSL SGYVFGGGTKLTVL |
| FA00 6 | 180 | QSVLTQPPSASGTPGQRVTISCSGSSSNIGSNTFNWYQQLPGTAPKLL IYSNSQRPSGVPDRFSGSKSGTSASLAISGLRSEDEADYYCGAWDSSL SAYVFGGGTKLTVL |
| FA00 7 | 181 | QSVLTQPPSASGTPGQRVTISCSGSSSNIGSNAVTWYQQLPGTAPKLL IYDDNHRPSGVPDRFSGSKSGTSASLAISGLRSEDEADYYCGAWDDSL SAYVFGGGTKLTVL |
| FA00 8 | 182 | QSVLTQPPSASGTPGQRVTISCSGSSSNIGNNDVYWYQQLPGTAPKLL IYSDSNRPSGVPDRFSGSKSGTSASLAISGLRSEDEADYYCGTWDYSL NGYVFGGGTKLTVL |

### Example 6: Conversion of scFv antibody fragments to IgG and production of antibody

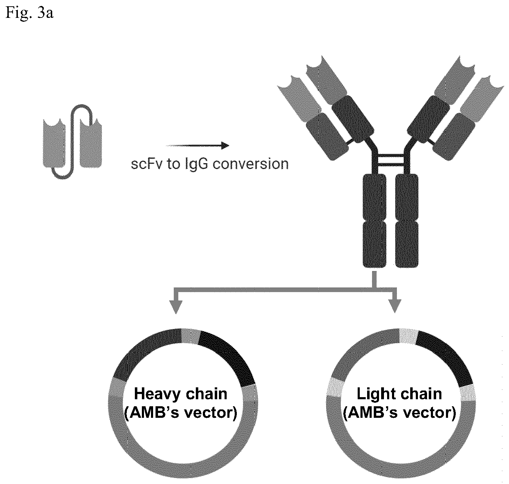

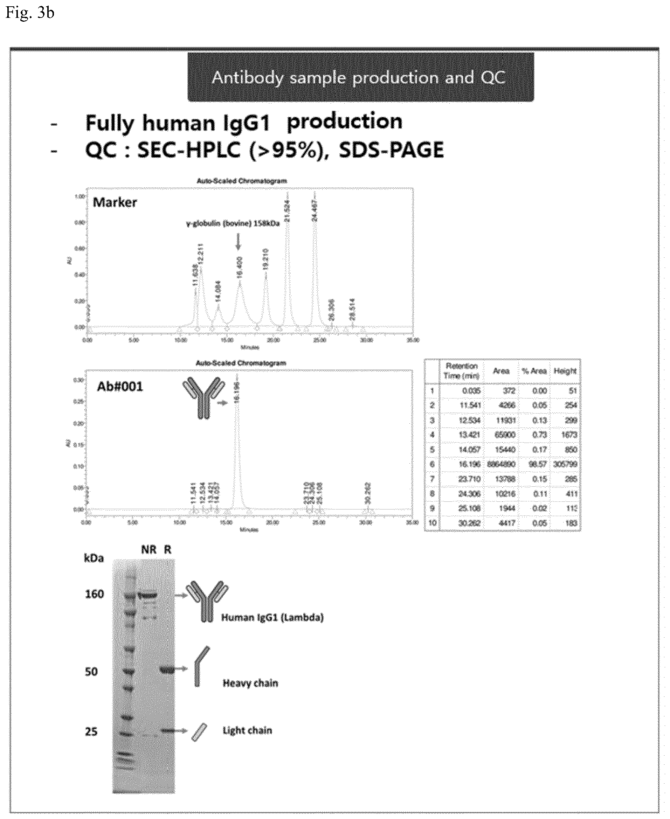

Based on the FGFR3 antibody sequence identified through sequencing, the present inventors performed cloning into a heavy chain production vector and a light chain production vector to construct an IgG production vector. Based on the constructed production vector, FGFR3 antibody was produced using a mammalian protein expression system (Expi293F) and high-purity antibody was recovered and purified with MabSelect SuRe^{™}, an affinity chromatography column based on AKTA Prime Plus equipment. The high-purity sample characteristics of the finished sample were analyzed based on SDS-PAGE and size-exclusion chromatography HPLC (SEC-HPLC) analysis (FIGS. 3A and 3B).

### Example 7: Evaluation of antigen-binding ability of FGFR3 antibody; ELISA

Affinity ELISA was performed to determine the binding ability of the produced antibody (IgG) to the FGFR3 antigen. A 96-well plate (4°C, overnight) was coated with 1-2 µg/ml of FGFR3 antigen and blocked with 3% skim milk (PBS) (room temperature, 1 hour). Then, the plate was treated with previously serially diluted antibody samples in duplicate and triplicate, and reacted at room temperature for 1 hour. Then, the result was washed with PBST 2 to 3 times, treated with the HRP-conjugated anti-human Fab antibody diluted in skim milk (room temperature, 1 hour), and washed again with PBST, and a color reaction (TMB substrate) was performed. Then, O.D. was measured at 450 nm.

The binding ability of the produced antibody (IgG) to human FGFR3 IIIb (1264-FR-050, R&D), IIIc (766-FR-050, R&D), mouse FGFR3 (9089-FR, R&D), and monkey FGFR3 (90313-C02H, Sino Biological) antigens was determined in the same manner as above.

The binding ability to each antibody was determined by concentration (FIGS. 4A to 4D) and the OD value was converted into a relative value and graphed. KD values were calculated based on EC₅₀ (Tables 5A to 5D).

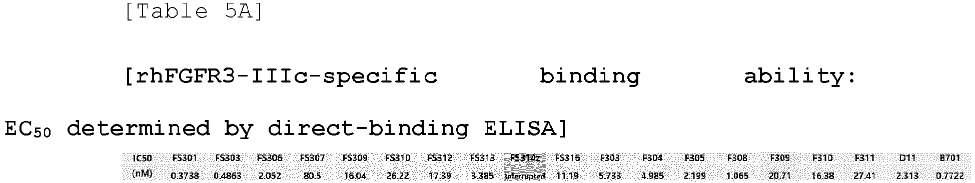

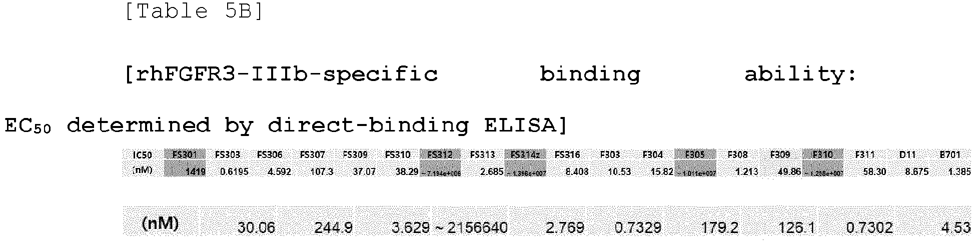

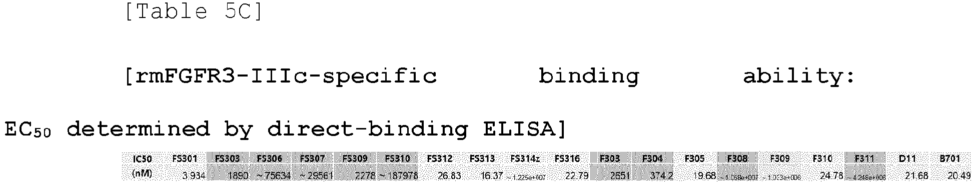

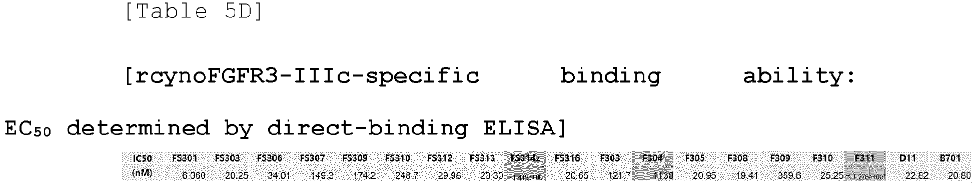

### Example 8: Evaluation of antigen-binding ability of FGFR3 antibody; SPR

SPR analysis was performed to evaluate the binding ability of the produced antibody (IgG) to the FGFR3 subtype and subfamily. The measurement of SRP-based affinity of FGFR3 antibody candidates for the FGFR3 antigen was performed using a Biacore-T200 instrument. The same antigens as described with reference to ELISA binding capacity were directly immobilized on a CM5 biosensor chip by amine coupling to achieve approximately 300 RU. Two-fold serial dilutions were injected at a flow rate of 30 pg/min at a concentration of approximately 20 nM for an association time of 180 seconds (25°C) and then separated at the same flow rate for 180 seconds. The CM5 biosensor chip was regenerated by injecting 10 mM glycine-HCl as a buffer between each injection. Association and dissociation rates were calculated using a one-to-one Langmuir binding model. The equilibrium dissociation constant (KD) was calculated as a ratio Koff/Kon (FIG. 5, Table 6).

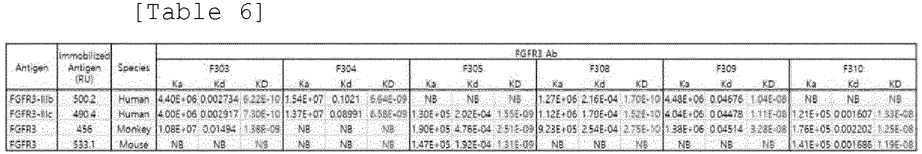

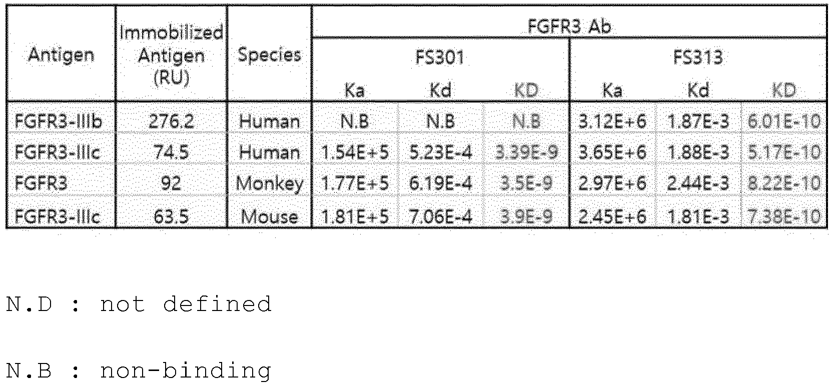

### Example 9: Confirmation of FGFR3 protein expression level; Western blotting, FACS analysis

Western blotting and FACS analysis were performed to select FGFR3-overexpressing cells for cell surface binding and other assays of the FGFR3 antibody discovered in the present invention. Patient-derived cells and cancer cell lines were selected at mRNA expression levels (RPKM) and a certain number of cells was secured through subculture of each cell. In Western blotting, lysate was prepared and verified, and in FACS analysis, direct cell surface FGFR3 expression was confirmed. First, in Western blotting, each cell was lysed with cOmplete^{™} Lysis-M (Roche), followed by Bradford protein quantitative analysis and western blotting of 20 pg of the lysate. Total FGFR3 (sc-13121, Santa Cruz) and beta-actin were detected to compare relative FGFR3 protein expression levels between the cell lines. In FACS, each subcultured cell was seeded at 3.0-5.0E+05 cells/tube, reacted with FGFR3-PE direct antibody (FAB766P, R&D) at 4°C for 1 hour, washed 2 to 3 times and analyzed with BD FACSAria^{™} III (BD Biosciences).

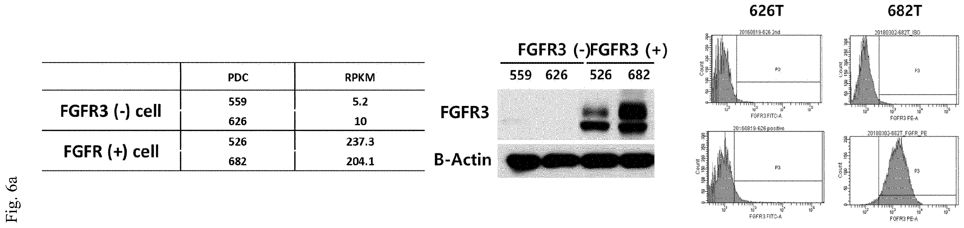

As a result of Western blotting, 682T and 526T patient-derived cells were identified as FGFR3 overexpressing cells. On the other hand, 626T and 559T were found to express almost no FGFR3. The results of FACS results showed the same behaviors. In particular, 682T cells are considered to express a very high level of FGFR3 on the surface thereof (FIG. 6A).

### Example 10: FGFR3-TACC3 fusion verification; RT-PCR

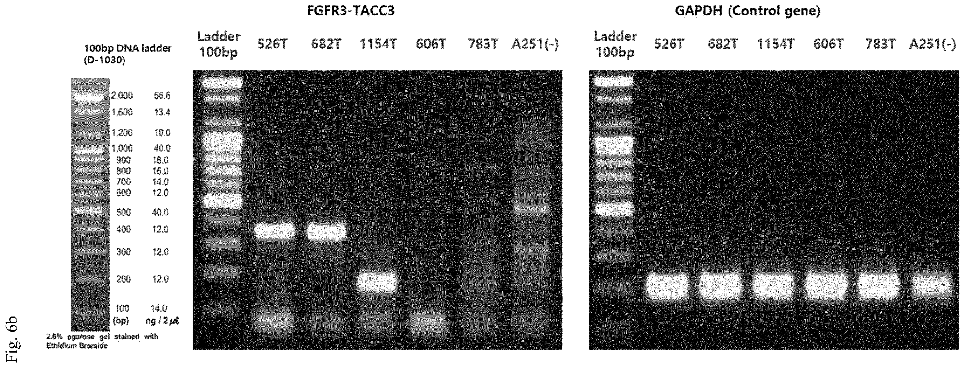

FGFR3 was overexpressed, amplified, mutated, or fused in various carcinomas. In particular, patients with FGFR3-TACC3 fusion have been reported to have poor prognosis in bladder cancer and brain tumor. Therefore, a therapeutic agent for these diseases has the potential to overcome unmet medical needs. Therefore, in the present invention, the patient-derived cells were grouped based on the FGFR3-TACC3 fusion analysis, which was identified using RT-PCR. Patient-derived cells GBM15-682T, GBM14-526T, BT16-1154T, GBM14-606T, and NS10-783T were proliferated through subculture, and predetermined amounts of the cells were recovered and RNA was extracted using RNeasy Mini Kit (Qiagen). Since the extracted RNA was unstable, cDNA was synthesized by RT-PCR (superscript IV Reverse transcriptase) . To analyze the fusion site of FGFR3-TACC3, the fusion sites researched were identified using the UCSC genome browser and primers were constructed to recognize the FGFR3-TACC3-specific regions (FW: 5'-TGATCATGCGGGAGTGCTG-3', RV: 5'-CTCCTCACACCTGCTCCTC-3'). It was determined whether or not the FGFR3-TACC3 fusion-specific region was amplified through a PCR experiment on cDNA using the prepared primers. At the same time, GAPDH was also used as a control group. As a result, GBM15-682T, GBM14-526T, and BT16-1154T were identified as FGFR3-TACC3 fusions (PCR expected size, 400-500 bp). Thereamong, GBM15-682T and GBM14-526T were expected to be the same fusion, and BT16-1154T was expected to be a different fusion [FIG. 6B]. The characteristics and anticancer effects of additional FGFR3 antibody clones were analyzed based on FGFR3-TACC3 fusion brain tumor patient-derived cells.

### Example 11. Evaluation of cell-binding ability of FGFR3 antibody; FACS analysis]

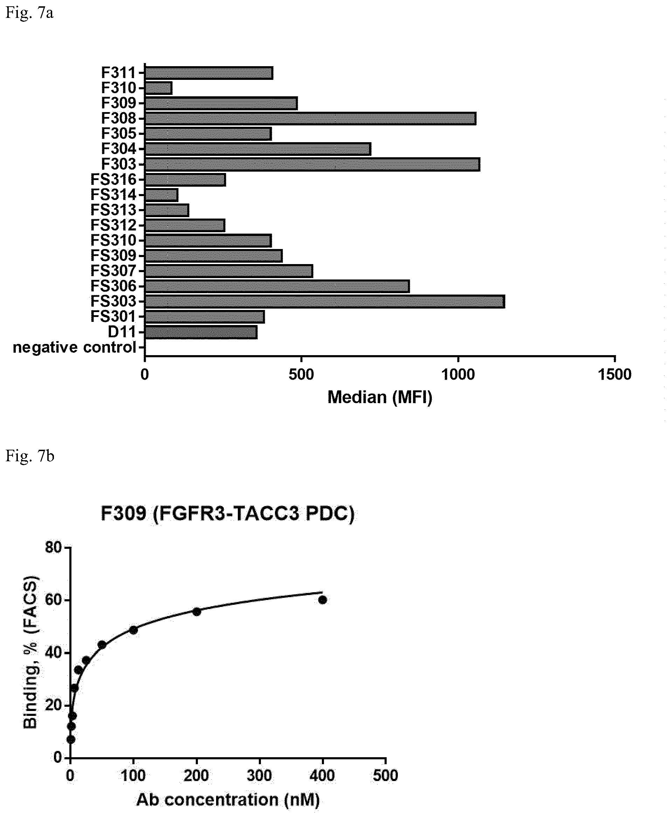

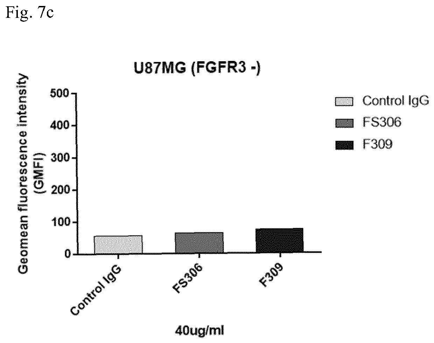

FACS analysis was performed to determine the binding ability of the antibody to the FGFR3-TACC3 overexpressing patient-derived cells identified in the present invention. Respective cells were seeded at 3.0-5.0E+05 cells/tube, washed with PBS, and treated with 100 to 200 nM of the discovered antibody at 4°C. Then, the non-adherent antibody sample was removed by the same washing method, the residue was treated with an anti-human Alexa-488 conjugate (Catalog# A-11013, Invitrogen) antibody to detect the antibody attached to the cell surface by BD FACSAria^{™}III (BD Biosciences) and to determine cell surface binding ability thereof. The discovered antibodies exhibited cell binding ability of different affinities (FIG. 7A). The cell surface binding ability of the F309 clone was analyzed depending on the concentration under the same conditions. It was found that the antibody had cell surface binding ability in proportion to concentration, which indicates that the antibody had FGFR3-specific binding ability (FIG. 7B). It was also found that the antibody did not bind to cell lines in which FGFR3 was not expressed (FIG. 7C).

### Example 12: Evaluation of target resolution of FGFR3 antibodies; FACS

FGFR3 target resolution of the candidate antibodies discovered in the present invention was screened through FACS. Representatively, GBM15-682T patient-derived cells, which are FGFR3 overexpressing cells, were subcultured and seeded at 3.0E+05 cells/tube and treated with each candidate antibody at 40 µg/ml (2 hours, 37°C). Then, the cells were washed 2 to 3 times, treated with FGFR3-PE (FAB766P, R&D) and reacted at 4°C for 1 hour. The cells were washed 2 to 3 times in the same manner as above and were analyzed with BD FACSAria^{™}III (BD Biosciences). As a result, the total FGFR3 expression level on the cell surface was compared after antibody treatment. Comparative analysis was performed based on the Geomean value compared to the IgG control. The cells were treated with the antibody for each time (4 hours, 2 hours, 1 hour, 30 minutes, 15 minutes) in the same manner as above and the cell surface FGFR3 degrading ability of the discovered antibody was determined over time. The same process was performed on the KMS11 cell line.

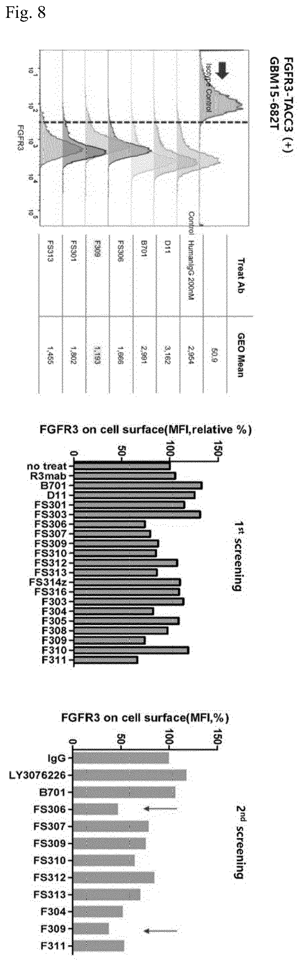

Relative comparison was performed at one concentration between preferentially discovered antibodies under the same conditions. Based on this, it was found that the F309 and FS306 antibodies effectively reduced FGFR3 on the cell surface (FIG. 8). In addition, the F309 and FS306 clones reduced the total expression of FGFR3 on the cell surface over time, which indicates that the corresponding clones were further found to have FGFR3 resolution. In addition, F309 and FS306 are considered to have superior resolution to other antibodies, B701 and LY3076226 (D11) antibodies (FIG. 10A).

### Example 13: FGFR3 epitope domain mapping

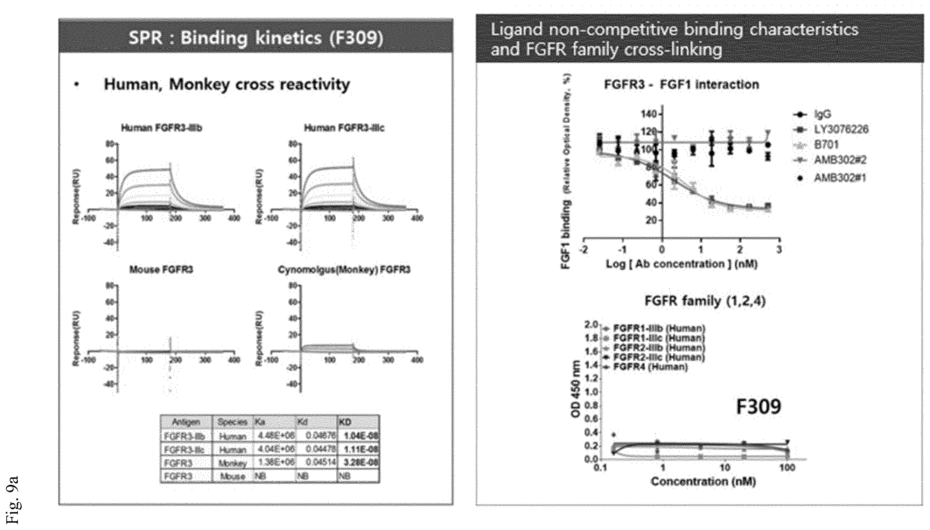

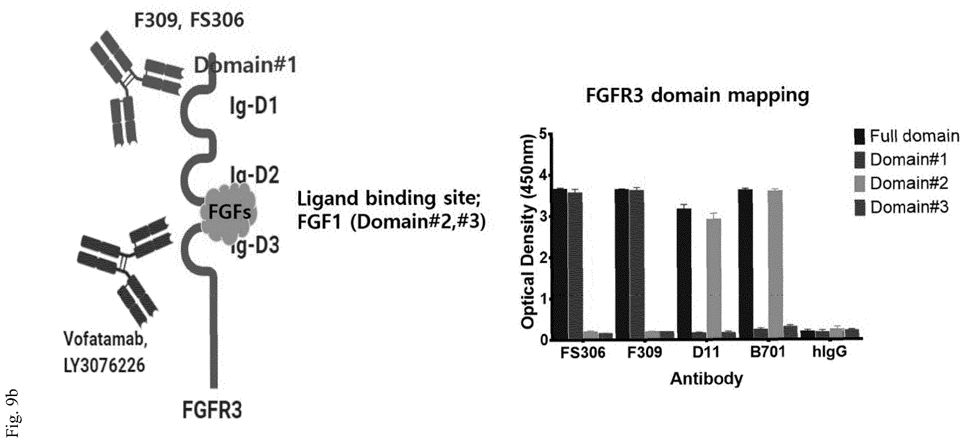

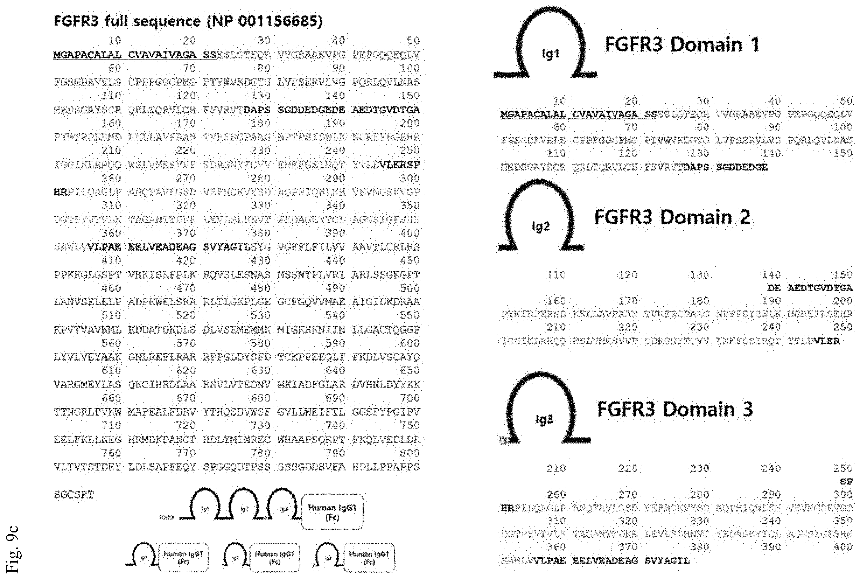

FGFR3 is composed of three domains, so each domain (FGFR3 Ig domain I, domain II, domain III) is attached with an Fc tag to analyze the FGFR3-binding domain of the discovered antibody, and produced and purified through a mammalian cell protein expression system (FIG. 9C). Analysis of the binding ability of the antibody to the purified domain was performed in the same manner as the ELISA described above. 1 µg/ml of an antigen was coated and treated with the antibody to induce a TMB color reaction. F309 and FS306 clones were found to specifically bind to FGFR3 domain 1 (FIG. 9B). Comparative antibodies B701 (Vofatamab, Rainier Therapeutics) and D11 (LY3076226, Eli Lilly) were found to bind between domains 2 and 3 as reported in the literature. However, the epitope of the comparative antibody is included in domain No. 2, which was conducted according to the present invention. In addition, ligands of FGF1 and FGF9, and FGFR3 have been reported to bind to Ig domain II and domain III of FGFR3. Based on this, competition ELISA assay was conducted. As a result, the B701 and D11 antibodies were observed to bind to FGFR3 competitively with the FGF ligand, as expected, whereas the F309 antibody was observed to bind to FGFR3 regardless of the FGF ligand. This supports binding of F309 antibody to the aforementioned FGFR3 Ig domain I (FIG. 9A).

### Example 14: Evaluation of target resolution of FGFR3 antibodies; Western blotting

The FGFR3 target resolution of the candidate antibodies discovered in the present invention was determined through Western blotting. GBM15-682T patient-derived cells were used as representative cells similar to the FACS method. The cells were seeded at 2.0E+06 cells/well in a 6-well plate, treated with each antibody at a concentration of 30 pg/ml, and reacted at 37°C for 3 hours. Then, a lysate was prepared, 20 pg of each sample was calculated using the Bradford quantification method, and western blotting was performed. Total FGFR3 was observed with the sc-13121 antibody. Experiments were performed on the KMS11 cell line at the same concentration under reaction conditions. Like the results of FACS, it was found that the entire FGFR3 resolution of the F309 and FS306 clones was excellent (FIG. 10B).

### Example 15: Antibody cellular internalization; Immunocytochemistry (ICC)

The cellular internalization mechanism of the antibody was further verified through the ICC method. GBM15-682T was used as FGFR3 over-expressing cells and U87MG was used as low-expressing cells. GBM15-682T patient-derived cells were not adhered to an ICC slide, so the slide was coated with a PLO reagent in advance and then the cells were cultured thereon. U87MG, as an adherent cell, was used without treatment with PLO treatment. First, cell internalization conditions were as follows. The cells attached to the ICC slide were treated with 30 µg/ml of each antibody and reacted at 37°C for 2 hours. Cell internalization inactivation was performed for 2 hours at 4°C at the same concentration. Then, the cells were washed and immobilized with 2% PFA (10 minutes, 4°C). The cells were washed in the same manner once more and reacted with 0.1% Triton-X at room temperature for 3 minutes to permeate the cells, and washed again. Then, the cells were blocked with 1% BSA at room temperature for 30 minutes, and treated with the primary antibody overnight at 4°C. Primary antibodies used herein were lysosomal marker LAMP-1 antibody and anti-human IgG. The next day, the cells were washed and reacted with the secondary antibody in the absence of light at 4°C for 1 hour. The secondary antibody used herein was a fluorescent antibody that probes the primary antibody (Alexa 488; Anti-human LAMP1, Alexa 594; Anti-human IgG). After washing and DAPI staining, each sample was analyzed through a confocal microscope. The result of the analysis showed that the F309 and FS306 clones were attached to the surface of GBM15-682T, FGFR3-overexpressing cells when treated at 4°C, but were observed at the same location as the lysosomal (LAMP-1) location because cell internalization proceeds under the condition of 37°C. Based on this, the clones are expected to be internalized into cells after binding to cell surface FGFR3 and degraded in lysosomes (FIG. 11A).

### Example 16: Mechanism of target degradation of FGFR3 antibodies; analysis of antibody mechanism through lysosomal inhibition

In the present invention, verification was conducted through a lysosomal inhibitor in order to identify the FGFR3 target decomposition mechanism of the previously described discovery antibody. A mechanism in which, when an antibody binds to a specific antigen on the cell surface, receptor-mediated endocytosis occurs, and the receptor and the antibody are degraded together in lysosomes has been reported. Therefore, degradation in lysosomes of FGFR3 antibodies found to have FGFR3-degrading ability was verified, and total FGFR3 levels of samples treated with and without treatment with a lysosomal inhibitor were detected through western blotting. GBM15-682T was seeded at 1.5E+06 cells/well in a 6-well plate and treated with Concanamycin, known as a lysosomal inhibitor, at a concentration of 100 nM at 37°C for 2 hours. Treatment with DMSO as a control was performed. After 2 hours, the cells were treated with 30 ug/ml of the antibody at 37°C for 3 hours. Then, the total FGFR3 level was determined in the same manner as in the western blotting method described above. The treatment with concanamycin did not reduce a lysosomal inhibitor, total FGFR3, even when treatment with F309 and FS306 clones was performed. On the other hand, the non-treatment with concanamycin significantly reduced total FGFR3. This indicates that F309 and FS306 clones are considered to be attached to the cell surface FGFR3 and then internalized into the cell and degraded together with FGFR3 in the lysosome (FIG. 11B).

### Example 17: Signaling pathway inhibition assay; Western blotting

FGFR3 downstream signaling inhibition assay was analyzed by western blotting. In the same manner as the western blotting method described above, GBM15-682T patient-derived cells were seeded into a 6-well plate and treated with 30 µg/ml of each antibody (37°C, 3 hours). Then, in order to activate FGFR3-related downstream signaling, the cells were treated with FGF1 and FGF9 representative ligands at 50 ng/ml (treated together with 50 µg/ml of heparin sulfate) and reacted at 37°C for 30 minutes. Then, lysate was prepared and western blotting was performed on 20 µg of each sample. Phosphorylation and activation of the FGFR3 downstream signaling pathway were verified using the following antibodies; p-FGFR3 (ab155960, Abeam), p-ERK (4370s, CST), p-AKT (9271s, CST). The F309 and FS306 clones were found to inhibit p-FGFR3, p-ERK, and p-AKT, which are FGFR3 downstream signaling pathways (FIG. 12) .

### Example 18: Analysis of cancer cell growth inhibition ability; ligand-dependent environment

In order to determine the ability of the antibody discovered in the present invention to inhibit cancer cell growth in a ligand-dependent environment, GBM15-682T was seeded on a 96-well black-flat bottom plate at 10,000 cells/well under serum-free conditions, starved for one day, treated with ligand-in FGF1 (232-FA, R&D) and FGF9 (273-F9, R&D) at a final concentration of 50 ng/ml, and then treated with serially diluted antibodies at different concentrations. Then, the cells were cultured in a 37°C CO₂ incubator for 7 days. Cell growth was measured through CellTiter-Glo^{®} luminescent cell viability assay, and cell growth of the antibody-treated group was compared with that of the control group. When the cells were treated with the ligand, cancer growth was accelerated by about 1.5 to 2 times by the ligand and when the cells were treated with the clone at each concentration, cell growth was inhibited by 20 to 30% compared to the IgG control (control group) (FIG. 13A).

### Example 19: Analysis of cancer cell growth inhibition ability; ligand independent environment

Based on the cell growth measurement method described in the present invention, the ability of the antibody to inhibit cancer cell growth was evaluated in a ligand-independent environment. GBM15-682T patient-derived cells were seeded at 10,000 cells/well on a 96-well black-flat bottom plate, and treated with serially diluted antibodies at different concentrations. Then, the cells were cultured in a 37°C CO₂ incubator for 7 days. Cell growth was measured through CellTiter-Glo^{®} luminescent cell viability assay, and cell growth of the antibody-treated group was compared with that of the control group. When the cells were treated with the antibody in a ligand-free environment, the F309 and FS306 clones inhibited cell growth by 30 to 40% compared to the IgG control (control group). The FGFR3 low-expressing cell line U87MG and patient-derived cells (626T) did not have the ability to inhibit cancer cell growth. This is considered to inhibit growth due to FGFR3 expression (FIG. 13B).

### Example 20: In vivo short-term PK analysis and efficacy evaluation in xenotransplantation model

Short-term PK was performed to determine the initial clearance when the discovered antibody was administered *in vivo.* 10 mg/kg of the antibody was administered to BALB/c mice, and immediately after administration, blood samples of subjects were collected at intervals of 1 hour, 2 hours, 4 hours, 1 day and 2 days. To remove blood cells from blood, plasma was obtained through centrifugation and antibody titer in plasma was assayed based on ELISA. The result showed that, similar to the control antibody, both F309 and FS306 antibodies had an initial PK of normal antibodies without an initial clearance pattern (FIG. 14A).

To evaluate the *in vivo* efficacy of the discovered antibody, the present inventors constructed a xenotransplantation model. GBM15-682T (1.0E+06 cells) was injected into the flank of BALB/c nude mice via subcutaneous injection to form a tumor and mice were grouped again into 5 subjects when the tumor average volume reached 100 to 150 mm³. Antibodies and vehicles for each group were intraperitoneally injected 3 times a week or 2 times a week for each concentration. Tumor volume was calculated using the formula V = 0.5a × b² (a: the length of the long axis of the tumor, b: the length of the short axis of the tumor) using calipers. The weight of the mouse was also measured on the same day as the tumor measurement. It was found that the F309 and FS306 clones inhibited cancer growth by about 50% and 40%, respectively, when administered at 10 mg/kg (three times a week), respectively (FIG. 14A). F309 is considered to inhibit cancer growth based on excellent anticancer effects when administered twice a week at each concentration (30 mpk, 10 mpk, 3 mpk) (FIG. 14B).

### Example 21: F309 antibody affinity maturation engineering

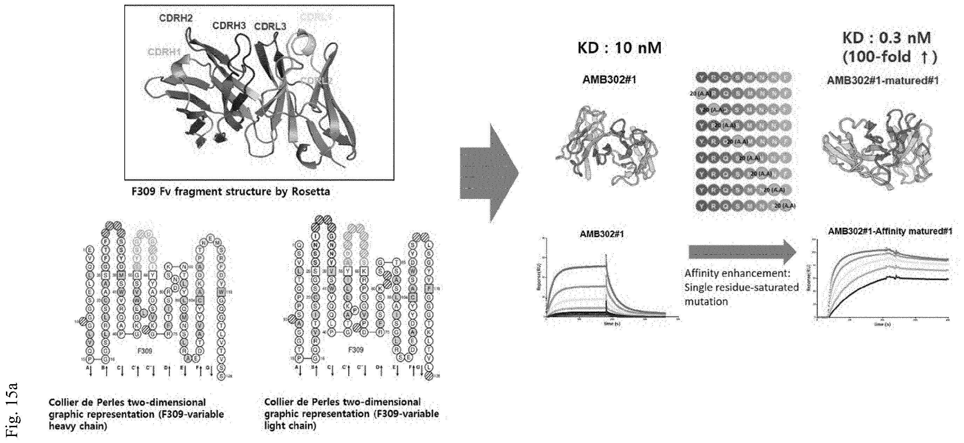

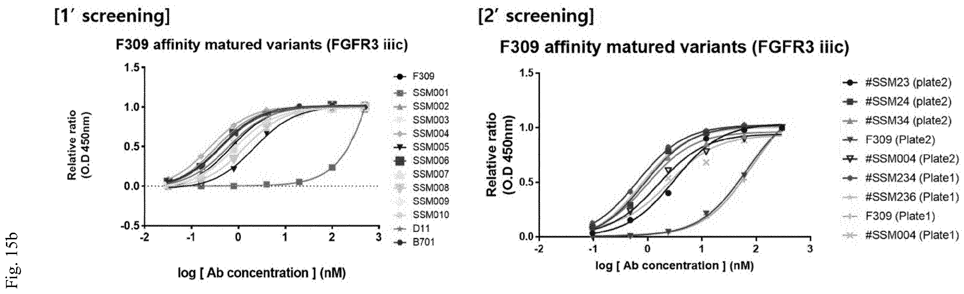

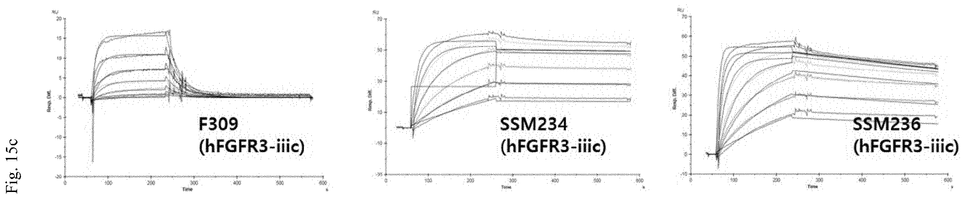

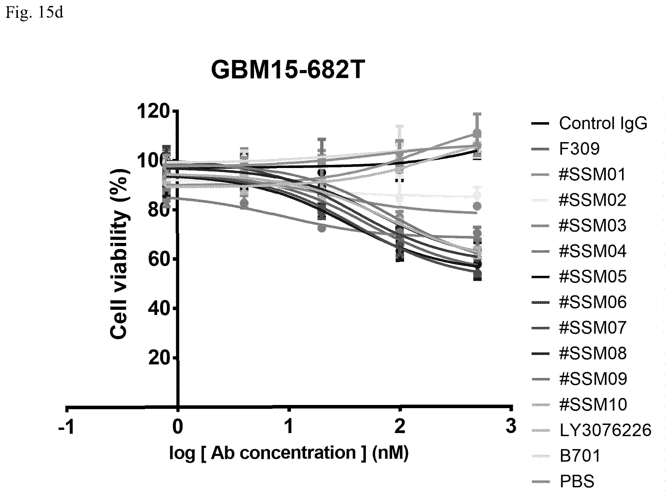

In order to further improve excellent FGFR3 resolution and cancer growth inhibitory ability of F309, affinity maturation was performed. A phagemid vector displaying the F309 scFv fragment (heavy chain variable region; VH, light chain variable region; VL) on the surface of M13 bacteriophage was constructed and used as a library template. For affinity maturation, CDR H1, H2 and H3 and CDR L1, L2 and L3 were selected based on antibody Kabat numbering, and an F309 variant library was constructed using primers containing the NNK degeneration codon for each CDR (FIG. 15A). For each library, biopanning (immobilized antigen panning) described above was performed 3 to 4 times, and based on this, antibodies with higher affinity than the F309 parent antibody were obtained by ELISA-based affinity screening. The obtained clones were re-verified by a reproduction experiment. Finally, affinity matured variants of F309 were obtained through antibody sequence analysis. The obtained F309 variants are determined to significantly increase the specific binding ability to the FGFR3 recombinant protein compared to the parent antibody (FIG. 15B). Furthermore, additional variants SSM023, SSM024, SSM026, SSM034, SSM036, SSM234, and SSM236 were designed and prepared by combination of the amino acid residues for the SSM001 to SSM010 variants. ELISA was performed to verify the FGFR3 antigen-specific binding ability of these variants to the antibodies. As a result, further increased FGFR3 antigen affinity was determined [FIGS. 15B and 15CJ. Analysis of the biological activity of the variant antibodies was performed in GBM15-682T. The result showed that the variant antibodies increased cancer growth inhibition compared to the F309 parent antibody (FIG. 15D).

**[Table 7]**

| Clone | SEQ IN No. | VH CDR1 | SEQ IN No. | VH CDR2 | SEQ IN No. | VH CDR3 |
|---|---|---|---|---|---|---|
| F309#SSM0 01 | 5 | SYDM S | 23 | GIYSGDGSIYYADS VKG | 186 | DYPTNEMSRF DY |
| F309#SSM0 02 | | | | | 187 | DGPTNEMSRF DY |
| F309#SSM0 03 | | | | | 188 | DAPMNEMSRF DY |
| F309#SSM0 04 | | | | | 189 | DAPTNEISRF DY |
| F309#SSM0 05 | | | | | 190 | DAPTNEPSRF DY |
| F309#SSM0 06 | | | | | 191 | DAPTNEESRF DY |
| F309#SSM0 23 | | | | | 192 | DGPMNEMSRF DY |
| F309#SSM0 24 | | | | | 193 | DGPTNEISRF DY |
| F309#SSM0 26 | | | | | 194 | DGPTNEESRF DY |
| F309#SSM0 34 | | | | | 195 | DAPMNEISRF DY |
| F309#SSM0 36 | | | | | 196 | DAPMNEESRF DY |
| F309#SSM2 34 | | | | | 197 | DGPMNEISRF DY |
| F309#SSM2 36 | | | | | 198 | DGPMNEESRF DY |

**[Table 8]**

| Clone | SEQ IN No. | VH |
|---|---|---|
| **F309#SSM0 01** | 203 | EVQLLESGGGLVQPGGSLRLSCAASGFTFSSYDMSWVRQAPG KGLEWVSGIYSGDGSIYYADSVKGRFTISRDNSKNTLYLQMN SLRAEDTAVYYCAKDYPTNEMSRFDYWGQGTLVTVSS |
| **F309#SSM0 02** | 204 | EVQLLESGGGLVQPGGSLRLSCAASGFTFSSYDMSWVRQAPG KGLEWVSGIYSGDGSIYYADSVKGRFTISRDNSKNTLYLQMN SLRAEDTAVYYCAKDGPTNEMSRFDYWGQGTLVTVSS |
| **F309#SSM0 03** | 205 | EVQLLESGGGLVQPGGSLRLSCAASGFTFSSYDMSWVRQAPG KGLEWVSGIYSGDGSIYYADSVKGRFTISRDNSKNTLYLQMN SLRAEDTAVYYCAKDAPMNEMSRFDYWGQGTLVTVSS |
| **F309#SSM0 04** | 206 | EVQLLESGGGLVQPGGSLRLSCAASGFTFSSYDMSWVRQAPG KGLEWVSGIYSGDGSIYYADSVKGRFTISRDNSKNTLYLQMN SLRAEDTAVYYCAKDAPTNEISRFDYWGQGTLVTVSS |
| **F309#SSM0 05** | 207 | EVQLLESGGGLVQPGGSLRLSCAASGFTFSSYDMSWVRQAPG KGLEWVSGIYSGDGSIYYADSVKGRFTISRDNSKNTLYLQMN SLRAEDTAVYYCAKDAPTNEPSRFDYWGQGTLVTVSS |
| **F309#SSM0 06** | 208 | EVQLLESGGGLVQPGGSLRLSCAASGFTFSSYDMSWVRQAPG KGLEWVSGIYSGDGSIYYADSVKGRFTISRDNSKNTLYLQMN SLRAEDTAVYYCAKDAPTNEESRFDYWGQGTLVTVSS |
| **F309#SSM0 23** | 209 | EVQLLESGGGLVQPGGSLRLSCAASGFTFSSYDMSWVRQAPG KGLEWVSGIYSGDGSIYYADSVKGRFTISRDNSKNTLYLQMN SLRAEDTAVYYCAKDGPMNEMSRFDYWGQGTLVTVSS |
| **F309#SSM0 24** | 210 | EVQLLESGGGLVQPGGSLRLSCAASGFTFSSYDMSWVRQAPG KGLEWVSGIYSGDGSIYYADSVKGRFTISRDNSKNTLYLQMN SLRAEDTAVYYCAKDGPTNEISRFDYWGQGTLVTVSS |
| **F309#SSM0 26** | 211 | EVQLLESGGGLVQPGGSLRLSCAASGFTFSSYDMSWVRQAPG KGLEWVSGIYSGDGSIYYADSVKGRFTISRDNSKNTLYLQMN SLRAEDTAVYYCAKDGPTNEESRFDYWGQGTLVTVSS |
| **F309#SSM0 34** | 212 | EVQLLESGGGLVQPGGSLRLSCAASGFTFSSYDMSWVRQAPG KGLEWVSGIYSGDGSIYYADSVKGRFTISRDNSKNTLYLQMN SLRAEDTAVYYCAKDAPMNEISRFDYWGQGTLVTVSS |
| **F309#SSM0 36** | 213 | EVQLLESGGGLVQPGGSLRLSCAASGFTFSSYDMSWVRQAPG KGLEWVSGIYSGDGSIYYADSVKGRFTISRDNSKNTLYLQMN SLRAEDTAVYYCAKDAPMNEESRFDYWGQGTLVTVSS |
| **F309#SSM2 34** | 214 | EVQLLESGGGLVQPGGSLRLSCAASGFTFSSYDMSWVRQAPG KGLEWVSGIYSGDGSIYYADSVKGRFTISRDNSKNTLYLQMN SLRAEDTAVYYCAKDGPMNEISRFDYWGQGTLVTVSS |
| **F309#SSM2 36** | 215 | EVQLLESGGGLVQPGGSLRLSCAASGFTFSSYDMSWVRQAPG KGLEWVSGIYSGDGSIYYADSVKGRFTISRDNSKNTLYLQMN SLRAEDTAVYYCAKDGPMNEESRFDYWGQGTLVTVSS |

**[Table 9]**

| Clone | SEQ IN No. | VL CDR1 | SEQ IN No. | VL CDR2 | SEQ IN No. | VL CDR3 |
|---|---|---|---|---|---|---|
| F309#SSM0 07 | 71 | SGSSSNIGNN . YVS | 93 | ADSKR PS | 199 | AVWDYSLSG YV |
| F309#SSM0 08 | | | | | 200 | ASWDYSLTG YV |
| F309#SSM0 09 | | | | | 201 | ASWDYSLDG YV |
| F309#SSM0 10 | | | | | 202 | ASWDYSLEG YV |

**[Table 10]**

| Clone | SEQ IN No. | VL |
|---|---|---|
| F309#SSM0 07 | 216 | QSVLTQPPSASGTPGQRVTISCSGSSSNIGNNYVSWYQQLPG TAPKLLIYADSKRPSGVPDRFSGSKSGTSASLAISGLRSEDE ADYYCAVWDYSLSGYVFGGGTKLTVL |
| F309#SSM0 08 | 217 | QSVLTQPPSASGTPGQRVTISCSGSSSNIGNNYVSWYQQLPG TAPKLLIYADSKRPSGVPDRFSGSKSGTSASLAISGLRSEDE ADYYCASWDYSLTGYVFGGGTKLTVL |
| F309#SSM0 09 | 218 | QSVLTQPPSASGTPGQRVTISCSGSSSNIGNNYVSWYQQLPG TAPKLLIYADSKRPSGVPDRFSGSKSGTSASLAISGLRSEDE ADYYCASWDYSLDGYVFGGGTKLTVL |
| F309#SSM0 10 | 219 | QSVLTQPPSASGTPGQRVTISCSGSSSNIGNNYVSWYQQLPG TAPKLLIYADSKRPSGVPDRFSGSKSGTSASLAISGLRSEDE ADYYCASWDYSLEGYVFGGGTKLTVL |

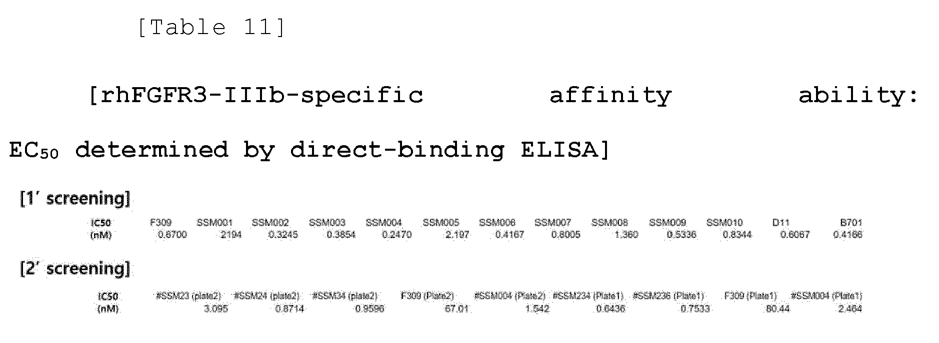

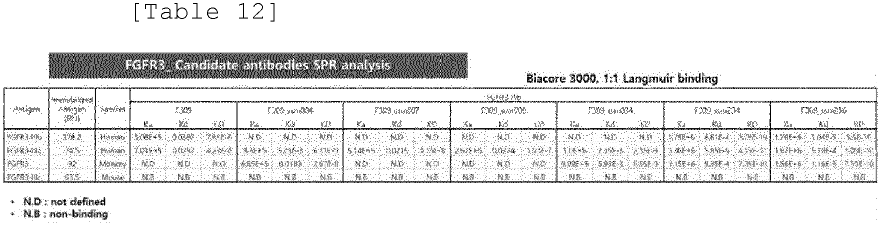

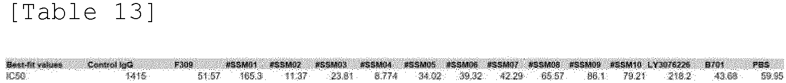

### Example 22: Verification of ADC applicability of F309 antibody

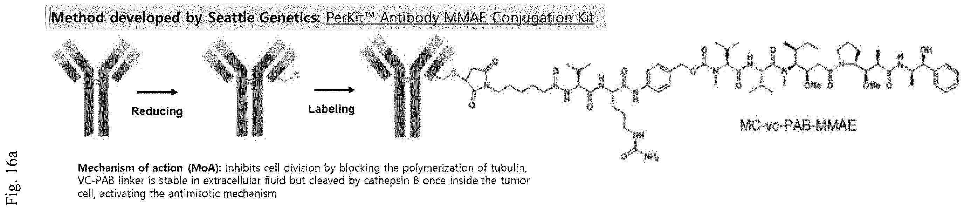

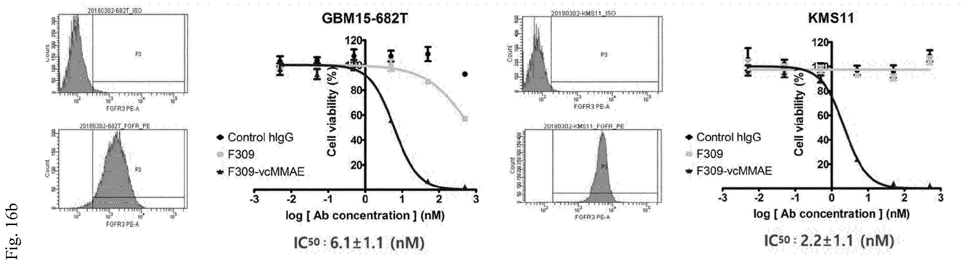

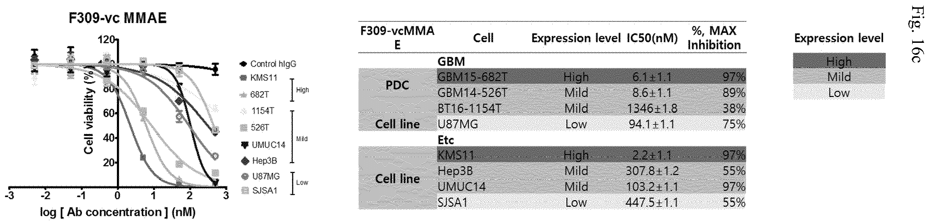

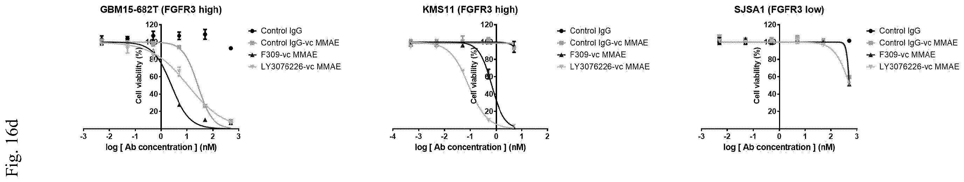

The antibody-drug conjugate has a mechanism in which the antibody specifically binds to an antigen on the cell surface, is introduced into the cell through receptor-mediated cell internalization and is degraded by lysosomes and proteases inside the cell, and cells were killed by the conjugated toxic substance. This is widely used as a next-generation antibody treatment platform for maximizing the therapeutic effect. In the present invention, the applicability of clones having excellent internalization and target resolution for FGFR3 and FGFR3-TACC3 fusion cells to antibody-drug conjugates was determined. The F309 and FS306 clones, as described in the present invention, were found to have FGFR3 target resolution and cell internalization and thus would be applicable as an antibody-drug conjugate platform. In particular, the antibody-drug conjugate for the F309 clone was produced using the PerKit^{™} antibody MMAE conjugation Kit (CellMosaic) and this platform is a first-generation antibody-drug conjugate (a method developed by Seattle Genetics: Sun et al. 2005, Bioconjugate Chem. 16, 1282-1290) produced by optionally conjugating - vc MMAE to the interchain disulfide of the antibody (FIG. 16A). Various patient-derived cells and cancer cell lines were selected based on the FACS expression criteria and relatively grouped into High, Mild, and Low, and the cytotoxicity test of F309 was conducted. The evaluation method was measured through the CellTiter-Glo^{®} luminescent cell viability assay as described in the present invention. The result showed that F309-vc MMAE exhibited excellent cytotoxicity in proportion to the amount of FGFR3 cell surface expression (FIGS. 16B to 16C). To secure additional competitiveness, the effects were compared using LY3076226 parent antibody, which is being clinically developed as FGFR3-ADC, with the same kit. F309-vc MMAE exhibited better cytotoxicity than LY3076266-vc MMAE in the FGFR3-TACC3 fusion GBM15-682T. The growth inhibitory effect of ADC on cells depending on the expression level of each FGFR3 was analyzed by IC₅₀ and MAX inhibition (FIG. 16D) and the result is shown in the table.

### Example 23: Epitope domain mapping and antigen affinity analysis of affinity matured antibodies

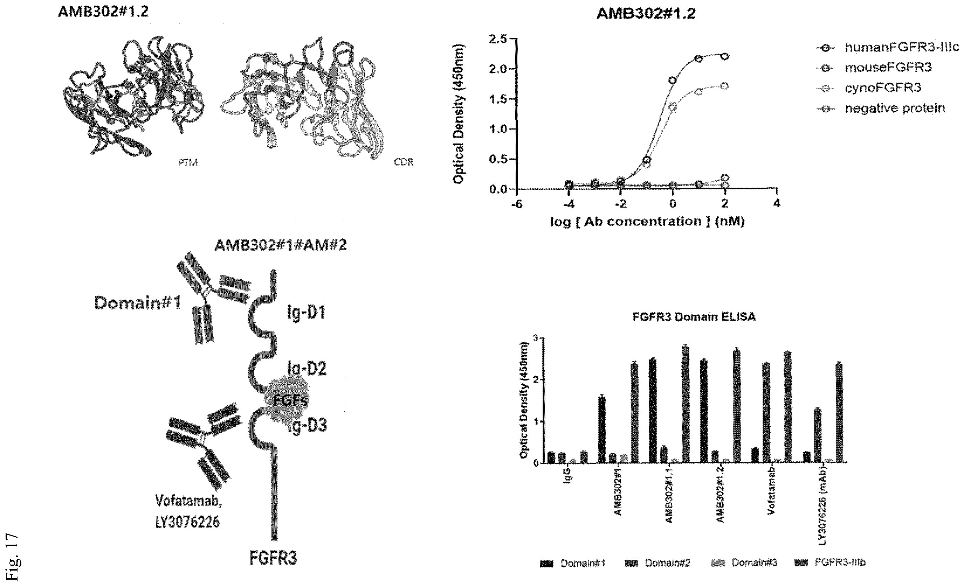

The epitope domain mapping performed above was also performed on the affinity matured antibody F309#SSM236 (hereinafter referred to as AMB302#1.2) . The parent antibody was found to bind to domain D1. Even when analyzed using the same domains (Domain, I, Domain, II, and Domain III), the affinity matured antibody was found to bind specifically to domain D1. The antibody was also found to have specific binding ability to human/cyno FGFR3 (FIG. 17). KD values according to each antigen are shown in Table 14 below.

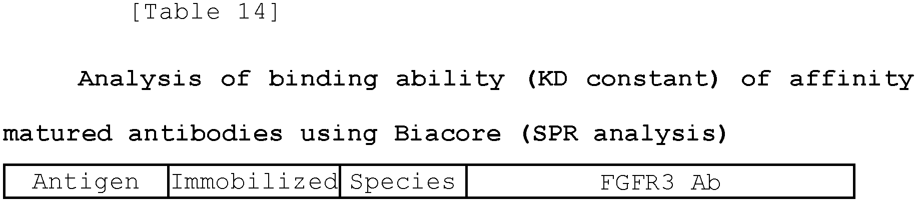

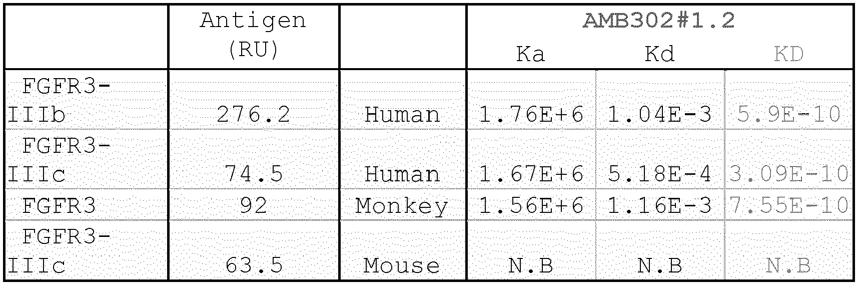

### Example 24: Evaluation of cell internalization and target resolution of affinity matured antibodies

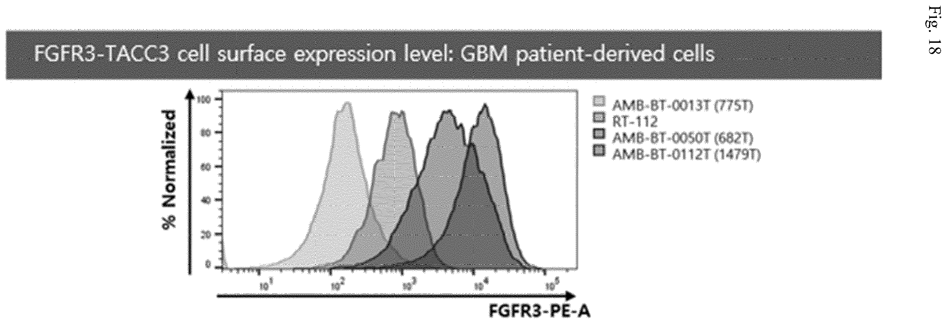

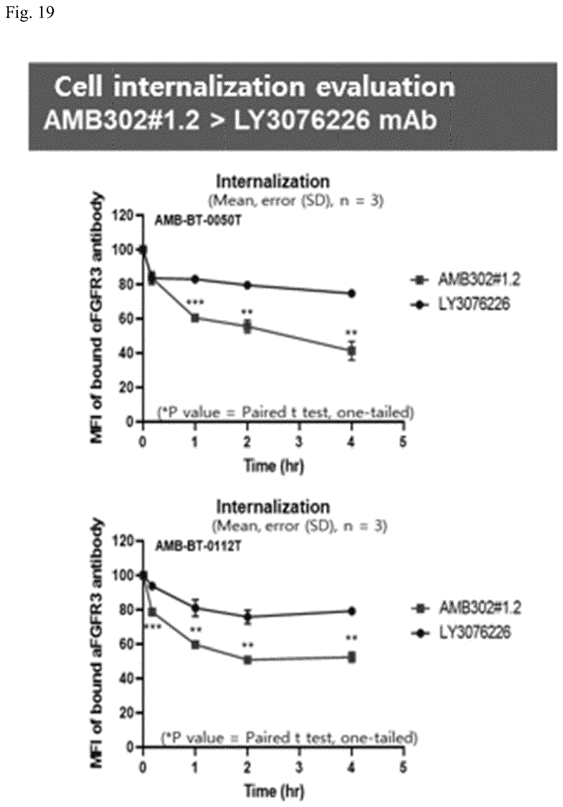

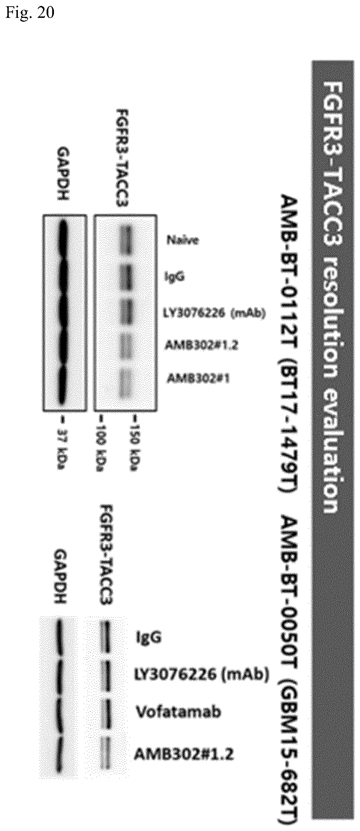

Whether or not F309 variants with significantly matured affinity efficiently degrade the FGFR3 target on the cell surface was determined. In the same manner as above, first, the detection of FGFR3 cell surface expression level and evaluation of cell internalization were performed through FACS, and target resolution was tested through Western blotting. Affinity matured variants had significant cell internalization efficiency and target resolution (FIGS. 18 to 20).

### Example 25: Rat/Monkey PK analysis of affinity matured antibodies

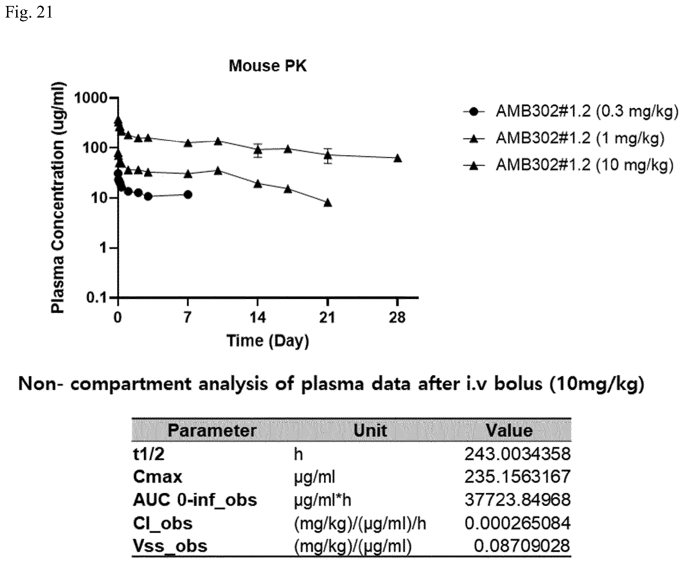

To validate the *in vivo* PK profile of affinity matured antibodies (AMB302#1.2, SSM236), analysis was performed on rat/monkey subjects. 10 mg/kg, 1 mg/kg, or 0.1 mg/kg of monoclonal antibody was administered to rat BALB/c mice (8 weeks, ~20 g, n=3, female). Immediately after administration, at intervals of 10 min, 1 h, 3 h, 5h, 8h, 24h, and 2 days, 3 days, 7 days, 10 days, 14 days, 17 days, 21 days, and 28 days, orbital blood sampling was alternately performed on three viable subjects hourly. To remove blood cells from blood, plasma was obtained through centrifugation, and antibody titer in plasma was quantified based on quantification ELISA. The result showed that the AMB302#1.2 antibody had a stable half-life (t 1/2) of more than 10 days and CL *in vivo* in mice. In addition, no biologically specific clinical side effects and weight changes were observed at all administration concentrations (FIG. 21).

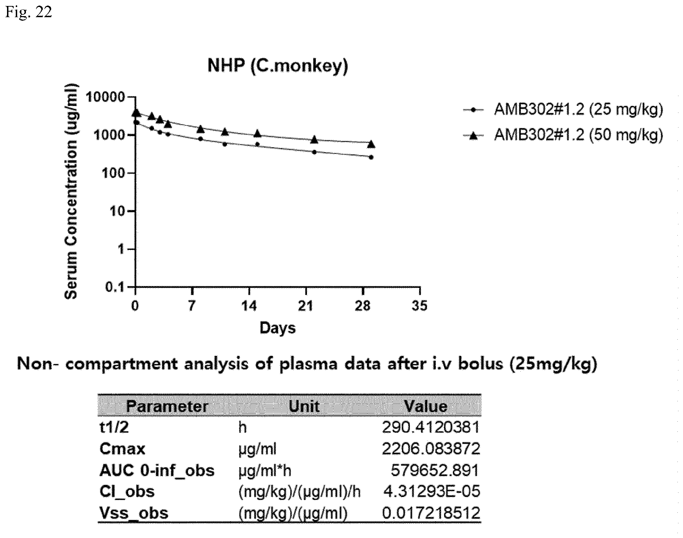

A single dose of 25 mg/kg and then 50 mg/kg was administered to one monkey (2-3 years, ~ 3 kg, female, naive) and blood samples were collected at each time (10 minutes, 1 and 6 hours, 1 day, 2 days, 3 days, 7 days, 10 days, 14 days, 21 days, and 28 days). The obtained blood was centrifuged to remove blood cells and obtain plasma. The antibody in the plasma was assayed by quantification ELISA. The result showed that the monkey had a stable half-life (more than 10 days) and CL tendency, similar to the results in rats. In addition, there was no biologically specific clinical side effect and weight change at all administration concentrations (FIG. 22).

### Example 26: ADC preparation of affinity matured antibodies

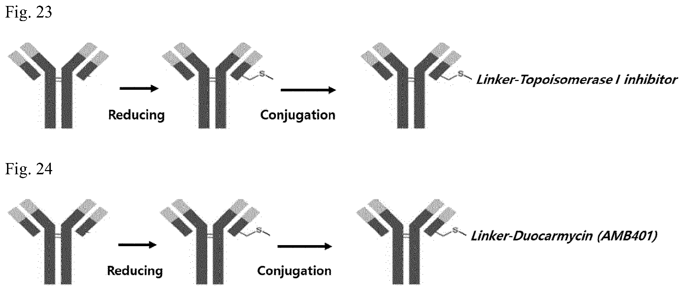

In the present invention, ADCs were prepared using the variants obtained through affinity maturation engineering (FIG. 23). The purified antibody samples were completely reduced at 37°C for 2 hours by treatment with tris(2-carboxyethyl) phosphine hydrochloride (TCEP) at an equivalent ratio of 8.0 or more. After reduction, buffer exchange was performed with PBS (pH 7.4) and a solution of the sample and a linker-payload (topoisomerase I inhibitor, equivalent ratio: 10.0) dissolved in DMA was conjugated with the reduced antibody at 37°C for 2 hours. After conjugation, the buffer was exchanged through a centrifugal column (Amicon Ultra-15 Centrifugal Filter Units).

In the same manner as above, the antibody was partially reduced to prepare an ADC conjugated with a linker-payload (Duocarmycin derivatives, linker-AMB401) (FIG. 24).

### Example 27: Affinity matured antibody ADC assay (TOPOI)

The antigen affinity of the synthesized ADC sample (TOPOI-conjugated ADC) was determined by ELISA and the determination was performed in the same manner as the affinity ELISA performed in the present invention. The result showed that the antibody had the same antigen-binding ability as the parent antibody. In addition, the high purity physical properties of the prepared ADC were identified through SEC-HPLC analysis.

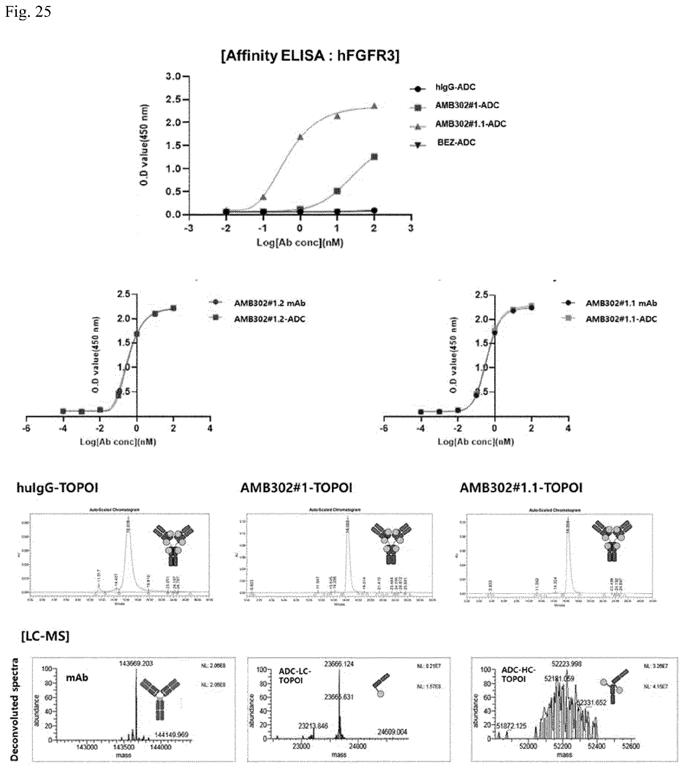

DAR analysis was performed through LC-MS and mass spectrometry of the linker-payload attached to the heavy chain and light chain was performed on the prepared ADCs. The measured value was converted to about DAR of 6 or higher (FIG. 25).

### Example 28: Affinity mature antibody ADC analysis (Duocarmycin, Linker-AMB401)

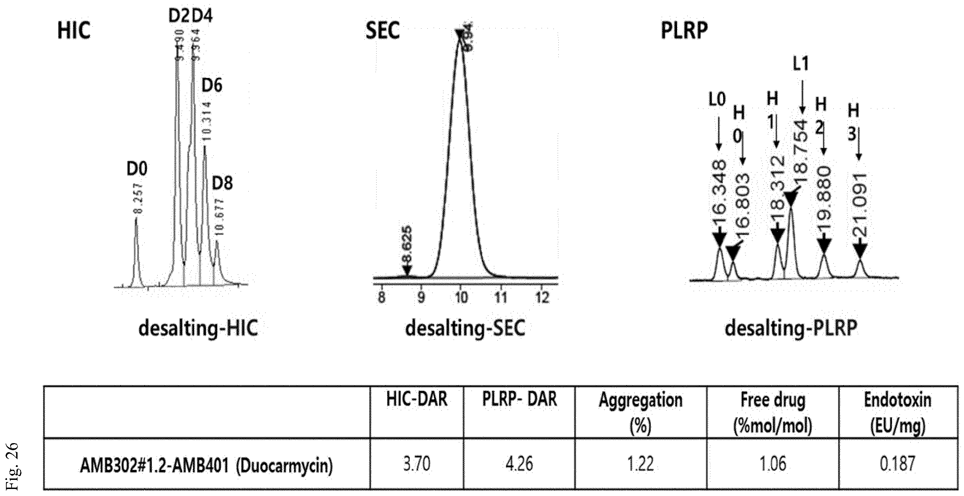

The purity of the synthesized ADC sample (duocarmycin, linker-AMB401; ADC) was analyzed through SEC-HPLC, and DAR thereof was analyzed through HIC-HPLC and PLRP analysis. The sample had a purity of 98% or more and uniform physical properties corresponding to a DAR of about 4 (FIG. 26: 20 mM histidine-acetate pH 6.0).

### Example 29: Evaluation of ADC efficacy of affinity matured variant in patient-derived organoid ex-vivo model (ADC-TOPOI)

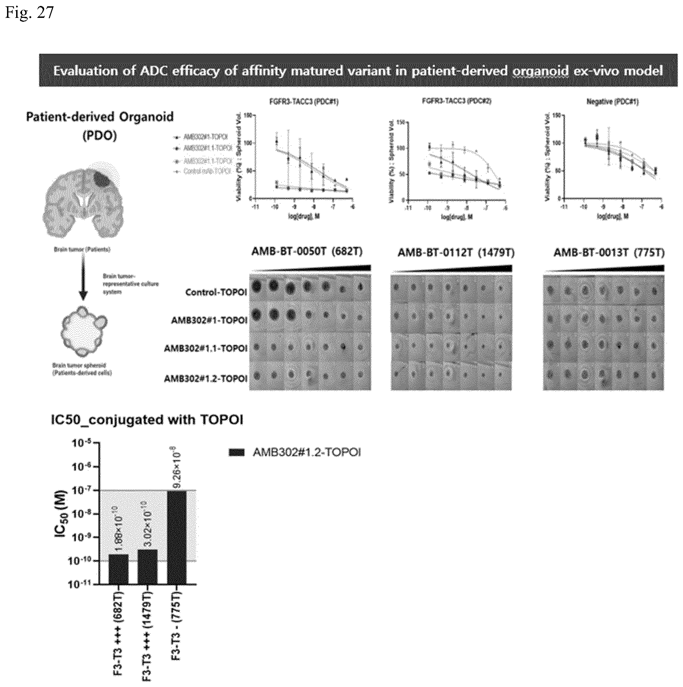

The efficacy of FGFR3 (TACC3 fusion) overexpressing patient-derived organoids was evaluated using the prepared ADC. Cancer cells were isolated from cancer tissues derived from brain tumor patients to form a single spheroid organoid model. Two patient-derived cells with FGFR3-TACC3 fusion (AMB-BT-0050T, AMB-BT-0112T) and other patient-derived cells (AMB-BT-0013T) were incubated overnight to form single spheroids, and then the single spheroids were treated with ADC and incubated for a week. The result was confirmed. The negative control had no difference in sensitivity between ADC and control ADC, and two patient-derived cells with FGFR3-TACC3 fusion specifically inhibited spheroid growth in proportion to the amount of FGFR3 expressed and induced apoptosis compared to control ADC. In particular, the affinity matured variant antibody-ADC significantly improved cytotoxicity and induced apoptosis in FGFR3 (TACC3 fusion) overexpressing cells compared to parental antibody-ADC. FGFR3 (TACC3 fusion) overexpressing cells had an IC₅₀ of 0.3 nM or less, and negative cells had an IC₅₀ of 90 nM or more (FIG. 27).

### Example 30: Evaluation of ADC efficacy of affinity matured variants in patient-derived organoid ex vivo model (ADC-Duocarmycin, linker-AMB401)

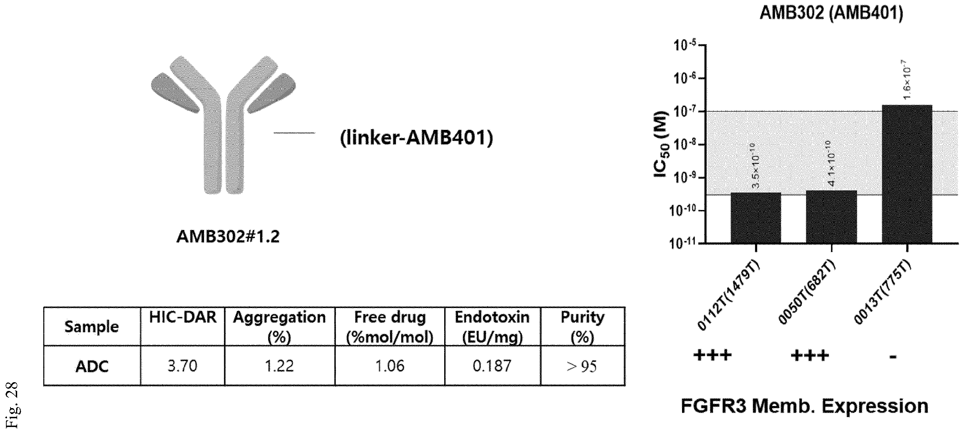

The ex *vivo* efficacy of AMB302#1.2-Duocarmycin ADC was evaluated in the same manner as in Example 27. FGFR3 (TACC3)-overexpressing brain tumor patient-derived cells had a low IC₅₀ of about 0.3 nM, whereas negative cells had a high IC₅₀ of 100 nM or more. This indicates that the ADC induced cell death significantly proportional to FGFR3 expression (FIG. 28).

### Example 31: ADC efficacy evaluation in FGFR3 (TACC3 fusion) brain tumor stereotactic animal model (ADC-TOPOI)

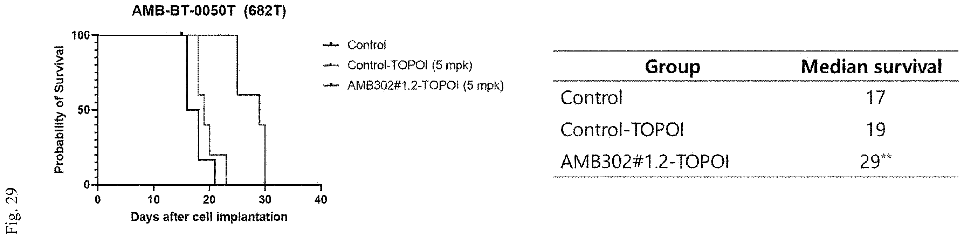

The increase in survival rate in a brain tumor stereotactic animal model (brain tumor, GBM) through ADC treatment was evaluated (FIG. 29). AMB-BT-0050T (682T) patient-derived cells, which are FGFR3 overexpressing cells, were cultured and 2×10⁵ of the cells were mixed with 5 pl of medium. The prepared cell line was injected at a depth of 3.2 mm at a location 1.7 mm left and 0.5 mm right from the bregma of the immunodeficient mouse to construct a brain tumor mouse model. The evaluation was conducted on 7 mice per each of a total of four groups: Control, Control-TOPOI, and PMB302#1.2-TOPOI (single administration, multiple administration). Candidate drugs were administered once by intravenous injection on the 7^{th} day after model construction. On day 15, medium sampling was performed on two mice per group for biomarker analysis. The body weight of the mouse was measured twice a week. When there was a weight loss of 20% or more, euthanasia was performed and the brain of the mouse was removed. PMB302#1.2-TOPOI increased survival time of mice by more than 70% even by a single administration.

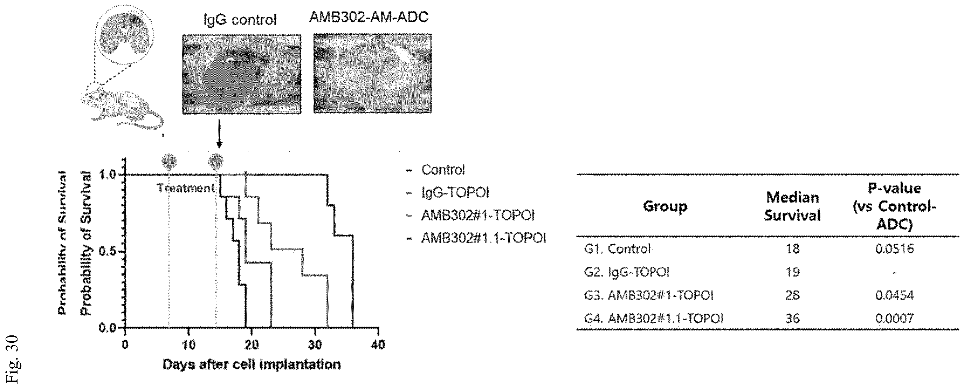

Even when AMB302#1.1-TOPO1 was administered twice (an interval of 1 week, 10 mg/kg) to the same model, the survival time of mice was remarkably increased by more than 100%. In addition, when comparing the tumor size of the mouse brain through intermediate sampling, no tumor was found in the ADC-treated subject compared to the control group. In addition, it was found that the affinity matured ADC exhibited greatly improved tumor suppression and increased survival rate compared to the parental antibody-based ADC (FIG. 30).

### Example 32: Evaluation of ADC efficacy in FGFR3 (TACC3 fusion) subcutaneous transplantation model (ADC-TOPOI)

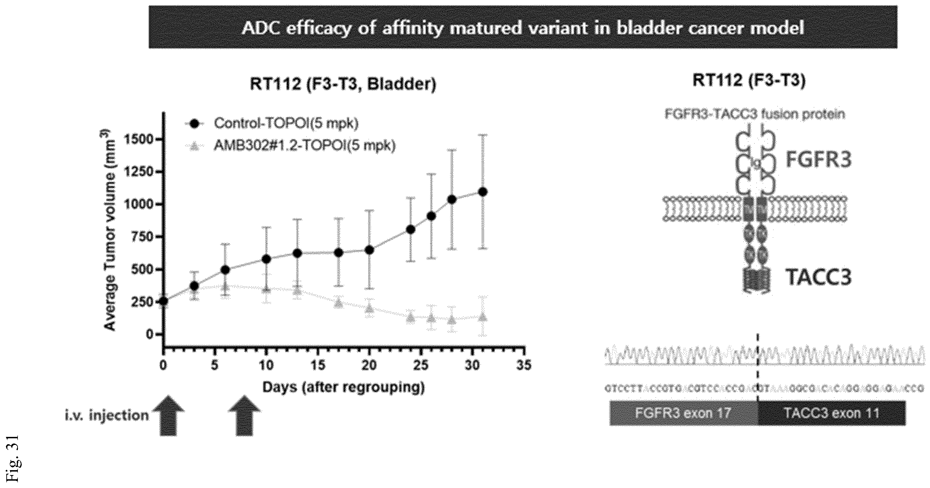

RT112 cells (1.0E+06 cells) were injected into the flank of a BALB/c nude mouse by subcutaneous injection (s.c., subcutaneous) to form a tumor. When the average tumor size reached 100 to 150 mm³, the mice were grouped again such that each group included 5 mice. ADC and vehicle were intravenously injected into each group twice (at interval of one week, 5 mp/kg). The tumor volume was calculated using the formula V=0.5a × b² (a: length of the long axis of the tumor, b: length of the short axis of the tumor) using calipers. The weight of the mouse was also measured on the same day as the tumor measurement. When ADC-TOPOI was administered, it completely inhibited cancer growth, which indicates that ADC-TOPOI had excellent anticancer effects (FIG. 31).

### Example 33: ADC efficacy evaluation in FGFR3 (TACC3 fusion) brain tumor stereotactic animal model (ADC-Duocarmycin, linker-AMB401)

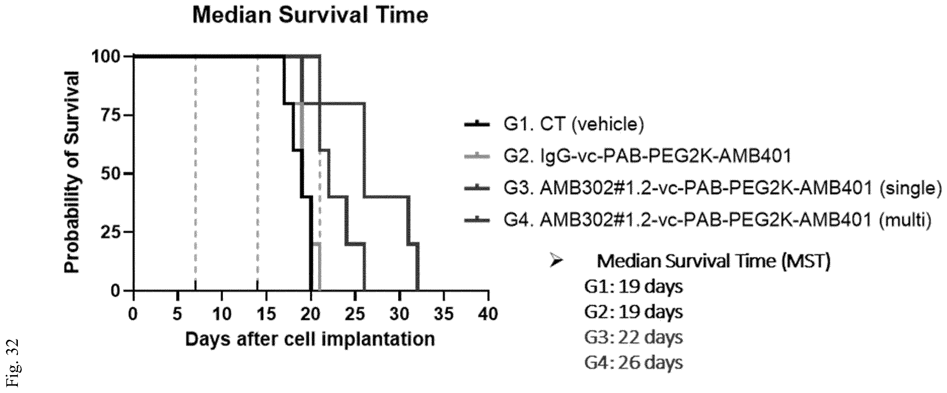

The efficacy of single administration (10 mg/kg) or multiple administration (10 mg/kg, Q4D) of AME302#1.1-AME401 was evaluated. The survival rate increased significantly in the ADC administration group, and in particular, the survival rate increased by 30% or more in the multi-administration group (FIG. 32).

### [Industrial applicability]

The anti-FGFR3 antibody or antigen-binding fragment thereof according to the present invention exhibits superior binding ability to FGFR3 to conventional anti-FGFR3 antibodies, thus being useful to prevent or treat a tumor or cancer of interest.

In particular, an antibody having better efficacy against FGFR3-TACC3 overexpressing cancer cells than known anti-FGFR3 antibodies was selected by screening through FGFR3-TACC3 overexpressing brain tumor patient-derived cells.

Although specific configurations of the present invention have been described in detail, those skilled in the art will appreciate that this detailed description is provided as preferred embodiments and should not be construed as limiting the scope of the present invention. Therefore, the substantial scope of the present invention is defined by the accompanying filed claims and equivalents thereto.

### [Sequence Listing Free Text]

An electronic file is attached.

## Claims

1. An antibody or antigen-binding fragment thereof specifically binding to FGFR3 (fibroblast growth factor receptor 3), comprising:
a heavy chain CDR1 comprising at least one amino acid sequence selected from the group consisting of SEQ ID NOS: 1 to 18;
a heavy chain CDR2 comprising at least one amino acid sequence selected from the group consisting of SEQ ID NOS: 19 to 41;
a heavy chain CDR3 comprising at least one amino acid sequence selected from the group consisting of SEQ ID NOS: 42 to 66 and SEQ ID NOS: 186 to 198;
a light chain CDR1 comprising at least one amino acid sequence selected from the group consisting of SEQ ID NOS: 67 to 88;
a light chain CDR2 comprising at least one amino acid sequence selected from the group consisting of SEQ ID NOS: 89 to 109; and
a light chain CDR3 comprising at least one amino acid sequence selected from the group consisting of SEQ ID NOS: 110 to 132 and SEQ ID NOS: 199 to 202.

2. The antibody or antigen-binding fragment thereof according to claim 1, comprising a heavy-chain variable region comprising at least one amino acid sequence selected from the group consisting of SEQ ID NOS: 133 to 157, and SEQ ID NOS: 203 to 215.

3. The antibody or antigen-binding fragment thereof according to claim 1, comprising a light-chain variable region comprising at least one amino acid sequence selected from the group consisting of SEQ ID NOS: 158 to 182, and SEQ ID NOS: 216 to 219.

4. The antibody or antigen-binding fragment thereof according to claim 1, wherein the antibody comprises a heavy chain CDR1 of SEQ ID NO: 5, a heavy chain CDR2 of SEQ ID NO: 23, and a heavy chain CDR3 of SEQ ID NOS: 203 to 215; and a light-chain variable region comprising a light chain CDR1 of SEQ ID NO: 71, a light chain CDR2 of SEQ ID NO: 93, and a light chain CDR3 of SEQ ID NOS: 216 to 219.

5. The antibody or antigen-binding fragment thereof according to claim 1, comprising a single-chain Fvs (scFv), a single-chain antibody, Fab, F(ab'), or disulfide-linked Fvs (sdFv).

6. The antibody or antigen-binding fragment thereof according to claim 5, wherein the scFv comprises a heavy-chain variable region comprising at least one amino acid sequence selected from the group consisting of SEQ ID NOS: 133 to 157; and a light-chain variable region comprising at least one amino acid sequence selected from the group consisting of SEQ ID NOS: 158 to 182 linked to each other via a linker.

7. The antibody or antigen-binding fragment thereof according to claim 6, wherein the linker has a structure of (GₙS)ₘ, wherein n and m are each 1 to 10.

8. A nucleic acid encoding the antibody or antigen-binding fragment thereof according to any one of claims 1 to 7.

9. A recombinant expression vector comprising the nucleic acid according to claim 8.

10. A host cell transfected with the recombinant expression vector according to claim 9.

11. The host cell according to claim 10, wherein the host cell is COS-7, BHK, CHO, CHOK1, DXB-11, DG-44, CHO/- DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, or HT1080.

12. A method of producing an antibody or antigen-binding fragment thereof specifically binding to FGFR3, the method comprising:
culturing the host cell according to claim 10 to produce an antibody; and
isolating and purifying the produced antibody.

13. A bispecific or multispecific antibody comprising the antibody or antigen-binding fragment thereof according to any one of claims 1 to 7.

14. The bispecific or multispecific antibody according to claim 13, wherein the bispecific or multispecific antibody comprises, as a partner, at least one selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptors, c-Met, EGFR, HER2, KDR, PDGFRa, NRP1, and MARCO.

15. An immune cell-engaging bispecific or multispecific antibody comprising a scFv consisting the scFv of the antibody according to any one of claims 1 to 7 and a second binding domain comprising at least one scFv of an antibody that binds to an immune cell-activating antigen.

16. The immune cell-engaging bispecific or multispecific antibody according to claim 15, wherein the scFv comprises a heavy-chain variable region comprising at least one amino acid sequence selected from the group consisting of SEQ ID NOS: 133 to 157, and SEQ ID NOS: 203 to 215; and a light-chain variable region comprising at least one amino acid sequence selected from the group consisting of SEQ ID NOS: 158 to 182 and SEQ ID NOS: 216 to 219 linked to each other via a linker.

17. The antibody or antigen-binding fragment thereof according to claim 16, wherein the linker has a structure of (GₙS)ₘ, wherein n and m are each 1 to 10.

18. An antibody-drug conjugate (ADC) in which the antibody or antigen-binding fragment thereof according to any one of claims 1 to 7 is conjugated to a drug.

19. The antibody-drug conjugate (ADC) according to claim 18, wherein the drug comprises:
(1) at least one anticancer agent selected from the group consisting of maytansinoid, auristatin (comprising MMAE and MMAF), aminopterin, actinomycin, bleomycin, thalidomide, camptothecin, N8-acetylspermidine, 1-(2 chloroethyl)-1,2-dimethyl sulfonyl hydrazide, esperamicin, etoposide, 6-mercaptopurine, dolastatin, trichothecene, calicheamicin, taxol, taxane, paclitaxel, docetaxel, methotrexate, vincristine, vinblastine, doxorubicin, melphalan, mitomycin A, mitomycin C, chlorambucil, duocarmycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, Cytoxan, etoposide, 5-fluorouracil, bis-chloroethylnitrosourea (CNU), irinotecan, camptothecin, bleomycin, idarubicin, daunorubicin, dactinomycin, plicamycin, mitoxantrone, asparaginase, vinorelbine, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, EICAR (5-ethynyl-1-beta-D-ribofuranosylimidazole-4-carboxamide), hydroxyurea, deferoxamine, floxuridine, doxifluridine, raltitrexed, cytarabine(ara C), cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, interferon-a, interferon-γ, tumor necrosis factors, gemcitabine, Velcade, Revlimid, Thalomid, lovastatin, 1-methyl-4-phenylpyridinium ion, staurosporine, actinomycin D, dactinomycin, bleomycin A2, bleomycin B2, peplomycin, epirubicin, pirarubicin, zorubicin, mitoxantrone, verapamil, and thapsigargin, nucleases and toxins derived from bacteria or plants and animals;
(2) an anti-inflammatory agent comprising a steroidal agent, a non-steroidal anti-inflammatory drug (NSAID), or an immune-specific anti-inflammatory drug (ImSAIDs); or
(3) a therapeutic agent for immune diseases comprising azathioprine, chlorambucil, cyclophosphamide, cyclosporine, mycophenolate, azathioprine, or methotrexate.

20. The antibody-drug conjugate (ADC) according to claim 18, wherein the antibody or antigen-binding fragment thereof is bound to the drug via a linker.

21. The antibody-drug conjugate (ADC) according to claim 20, wherein the linker is a cleavable linker or a non-cleavable linker.

22. The antibody-drug conjugate (ADC) according to claim 21, wherein the cleavable linker is an acid-labile linker, a disulfide linker, a peptide linker, or a beta-glucuronide linker, or the non-cleavable linker comprises a thioether group or a maleimidocaproyl group.

23. The antibody-drug conjugate (ADC) according to claim 20, wherein the linker binds to a cysteine residue exposed during reduction of a disulfide bond of an antibody or to a cysteine residue present in a tag bound to an antibody.

24. A chimeric antigen receptor (CAR) comprising:
an extracellular domain comprising an antigen-binding site;
a transmembrane domain; and
an intracellular signaling domain,
wherein the antigen-binding site of the extracellular domain is a scFv of the antibody according to any one of claims 1 to 7.

25. The chimeric antigen receptor (CAR) according to claim 24, wherein the scFv comprises a heavy-chain variable region comprising at least one amino acid sequence selected from the group consisting of SEQ ID NOS: 133 to 157; and a light-chain variable region comprising at least one amino acid sequence selected from the group consisting of SEQ ID NOS: 158 to 182 linked to each other via a linker.

26. The chimeric antigen receptor (CAR) according to claim 25, wherein the linker has a structure of (GₙS)ₘ, wherein n and m are each 1 to 10.

27. An immune cell into which the chimeric antigen receptor according to claim 24 is introduced.

28. The immune cell according to claim 27, wherein the immune cell comprises one or more selected from the group consisting of T cells, NK cells, cytokine induced killer cells (CIK), activated cytotoxic T lymphocytes (CTL), macrophages, tumor-infiltrating lymphocytes (TIL) and dendritic cells.

29. The immune cell according to claim 27, wherein the immune cell further comprises a chimeric antigen receptor (CAR) comprising, as the antigen-binding site of the extracellular domain, a scFv for an antibody other than the anti-FGFR3 antibody.

30. The immune cell according to claim 29, wherein the antibody other than the anti-FGFR3 antibody is an antibody targeting at least one selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptors, c-Met, EGFR, HER2, KDR, PDGFRa, NRP1, and MARCO.

31. A composition for combination therapy comprising the immune cell according to claim 27 and a drug other than an anti-FGFR3 antibody.

32. The composition according to claim 31, wherein the drug other than the anti-FGFR3 antibody is an antibody targeting at least one selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptors, c-Met, EGFR, HER2, KDR, PDGFRa, NRP1, and MARCO.

33. A composition for combination therapy comprising:
the antibody or antigen-binding fragment thereof according to any one of claims 1 to 7; and
at least one selected from the group consisting of:
(i) an immune cell;
(ii) an immune cell comprising a chimeric antigen receptor (CAR) comprising a scFv fragment of an antibody other than the anti-FGFR3 antibody as an extracellular domain; and
(iii) an immune checkpoint inhibitor.

34. The composition according to claim 33, wherein the immune cell comprises one or more selected from the group consisting of T cells, NK cells, cytokine induced killer cells (CIK), activated cytotoxic T lymphocytes (CTL), macrophages, tumor-infiltrating lymphocytes (TIL) and dendritic cells.

35. The composition according to claim 33, wherein the antibody other than the anti-FGFR3 antibody is an antibody targeting at least one selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptors, c-Met, EGFR, HER2, KDR, PDGFRa, NRP1, and MARCO.

36. The composition according to claim 33, wherein the immune checkpoint inhibitor is a drug targeting at least one selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226(DNAM1), CD96, CD200, CD200R, transferrin receptors, c-Met, EGFR, HER2, KDR, PDGFRa, NRP1, and MARCO.

37. A composition for combination therapy comprising:
the bispecific or multispecific antibody according to claim 13; and
at least one selected from the group consisting of:
(i) an immune cell;
(ii) an immune cell comprising a chimeric antigen receptor (CAR) comprising a scFv fragment of an antibody other than the anti-FGFR3 antibody as an extracellular domain; and
(iii) an immune checkpoint inhibitor.

38. The composition according to claim 37, wherein the antibody other than the anti-FGFR3 antibody is an antibody targeting at least one selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptors, c-Met, EGFR, HER2, KDR, PDGFRa, NRP1, and MARCO.

39. The composition according to claim 37, wherein the immune checkpoint inhibitor is a drug targeting at least one selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226(DNAM1), CD96, CD200, CD200R, transferrin receptors, c-Met, EGFR, HER2, KDR, PDGFRa, NRP1, and MARCO.

40. A composition for treating cancer or an inflammatory or immune disease comprising:
the antibody or antigen-binding fragment thereof according to any one of claims 1 to 7;
the bispecific or multispecific antibody comprising the antibody or antigen-binding fragment thereof;
the antibody-drug conjugate comprising the antibody or antigen-binding fragment thereof;
the chimeric antigen receptor comprising the antibody or antigen-binding fragment thereof; or
the immune cell comprising the chimeric antigen receptor.

41. A composition for treating cancer or an inflammatory or immune disease comprising the antibody or antigen-binding fragment thereof according to any one of claims 1 to 7, or the antibody-drug conjugate comprising the antibody or antigen-binding fragment thereof.

42. A composition for treating cancer or an inflammatory or immune disease determined that a fusion gene of FGFR3 and TACC3 is amplified, the fusion gene has mutation, the fusion gene is overexpressed, or the fusion gene is constitutively activated, the composition comprising the antibody or antigen-binding fragment thereof according to any one of claims 1 to 7, the bispecific or multispecific antibody comprising the antibody or antigen-binding fragment thereof, the antibody-drug conjugate comprising the antibody or antigen-binding fragment thereof, the chimeric antigen receptor comprising the antibody or antigen-binding fragment thereof, or the immune cell comprising the chimeric antigen receptor.

43. A method of treating cancer or an inflammatory or immune disease comprising:
determining, in a patient-derived sample, whether or not a fusion gene of FGFR3 and TACC3 is amplified, the fusion gene has mutation, the fusion gene is overexpressed, or the fusion gene is constitutively activated; and
administering, to the patient, the antibody or antigen-binding fragment thereof according to any one of claims 1 to 7; the bispecific or multispecific antibody comprising the antibody or antigen-binding fragment thereof; the antibody-drug conjugate comprising the antibody or antigen-binding fragment thereof; the chimeric antigen receptor comprising the antibody or antigen-binding fragment thereof; or the immune cell comprising the chimeric antigen receptor, upon determining that the fusion gene of FGFR3 and TACC3 is amplified, the fusion gene has mutation, the fusion gene is overexpressed, or the fusion gene is constitutively activated.

44. A vesicle comprising the antibody or antigen-binding fragment thereof according to any one of claims 1 to 7 exposed to the outside or present inside.

45. The vesicle according to claim 44, wherein the vesicle is an exosome.
